(19) Europäisches Patentamt / European Patent Office / Office européen des brevets

(11) **EP 4 640 843 A2**

(12) **EUROPEAN PATENT APPLICATION**

(43) Date of publication:
**29.10.2025 Bulletin 2025/44**

(21) Application number: **25187669.4**

(22) Date of filing: **24.01.2020**

(51) International Patent Classification (IPC):
**C12Q 1/6832** (2018.01)

(52) Cooperative Patent Classification (CPC):
(C-Sets available)
**C12Q 1/6886; C12Q 1/6832;** C12Q 2600/154
(Cont.)

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**

(30) Priority: **25.01.2019 US 201962797176 P**
**25.01.2019 US 201962797174 P**
**25.01.2019 US 201962797170 P**

(62) Document number(s) of the earlier application(s) in accordance with Art. 76 EPC:
**23217634.7 / 4 353 834**
**20710332.6 / 3 914 736**

(71) Applicant: **Grail, Inc.**
**Menlo Park, California 94025 (US)**

(72) Inventors:
• **VENN, Oliver Claude**
  **Menlo Park, CA 94025 (US)**
• **FIELDS, Alexander P.**
  **Menlo Park, CA 94025 (US)**
• **GROSS, Samuel S.**
  **Menlo Park, CA 94025 (US)**
• **LIU, Qinwen**
  **Menlo Park, CA 94025 (US)**
• **SCHELLENBERGER, Jan**
  **Menlo Park, CA 94025 (US)**

• **BREDNO, Joerg**
  **Menlo Park, CA 94025 (UM)**
• **BEAUSANG, John F.**
  **Menlo Park, CA 94025 (US)**
• **SHOJAEE, Seyedmehdi**
  **Menlo Park, CA 94025 (US)**
• **SAKARYA, Onur**
  **Menlo Park, CA 94025 (US)**
• **MAHER, M. Cyrus**
  **Menlo Park, CA 94025 (US)**
• **JAMSHIDI, Arash**
  **Menlo Park, 94025 (US)**

(74) Representative: **Murgitroyd & Company**
**165-169 Scotland Street**
**Glasgow G5 8PL (GB)**

Remarks:
•The complete document including Reference Table(s) and the Sequence Listing(s) can be downloaded from the EPO website
•This application was filed on 04-07-2025 as a divisional application to the application mentioned under INID code 62.
•Claims filed after the date of filing of the application / after the date of receipt of the divisional application (Rule 68(4) EPC).

(54) **DETECTING CANCER, CANCER TISSUE OF ORIGIN, AND/OR A CANCER CELL TYPE**

(57) The present description provides a cancer assay panel for targeted detection of cancer-specific methylation patterns. Further provided herein includes methods of designing, making, and using the cancer assay panel to detect cancer and particular types of cancer.

**FIG. 1A**

EP 4 640 843 A2

(52) Cooperative Patent Classification (CPC): (Cont.)

C-Sets
**C12Q 1/6832, C12Q 2523/125, C12Q 2525/204,**
**C12Q 2535/122, C12Q 2521/539, C12Q 2537/164**

**Description**

**CROSS-REFERENCE**

This application claims the benefit of U.S. Provisional Application No. 62/797,176, filed January 25, 2019, U.S. Provisional Application No. 62/797,174, filed January 25, 2019, and U.S. Provisional Application No. 62/797,170, filed January 25, 2019, which applications are incorporated herein by reference in their entireties.

**SEQUENCE LISTING**

The instant application contains a "lengthy" Sequence Listing which has been submitted via CD-R in lieu of a printed paper copy and is hereby incorporated by reference in its entirety. Said CD-R, recorded on January 23, 2020, are labeled "CRF", "Copy 1", "Copy 2" and "Copy 3", respectively, and each contains only one identical 243,821,056 bytes file (50251-849_601_SL.txt). Said CD-R and identical copies are hereby incorporated by reference in their entireties.

**BACKGROUND**

DNA methylation plays an important role in regulating gene expression. Aberrant DNA methylation has been implicated in many disease processes, including cancer. DNA methylation profiling using methylation sequencing (e.g., whole genome bisulfite sequencing (WGBS)) is increasingly recognized as a valuable diagnostic tool for detection, diagnosis, and/or monitoring of cancer. For example, specific patterns of differentially methylated regions may be useful as molecular markers for various diseases.

However, WGBS is not ideally suitable for a product assay. The reason is that the vast majority of the genome is either not differentially methylated in cancer, or the local CpG density is too low to provide a robust signal. Only a few percent of the genome is likely to be useful in classification.

Furthermore, there have been various challenges in identifying differentially methylated regions in various diseases. First off, determining differentially methylated regions in a disease group only holds weight in comparison with a group of control subjects, such that if the control group is small in number, the determination loses confidence with the small control group. Additionally, among a group of control subjects, methylation status can vary which can be difficult to account for when determining the regions are differentially methylated in a disease group. On another note, methylation of a cytosine at a CpG site is strongly correlated with methylation at a subsequent CpG site. To encapsulate this dependency is a challenge in itself.

Accordingly, a cost-effective method of accurately diagnosing a disease by detecting differentially methylated regions has not yet been available.

**SUMMARY**

Provided herein are compositions comprising a plurality of different bait oligonucleotides, wherein the plurality of different bait oligonucleotides is configured to collectively hybridize to DNA molecules derived from at least 200 target genomic regions, wherein each genomic region of the at least 200 target genomic regions is differentially methylated in at least one cancer type relative to another cancer type or relative to a non-cancer type, and wherein the at least 200 target genomic regions comprise, for at least 80% of all possible pairs of cancer types selected from a set comprising at least 10 cancer types, at least one target genomic region that is differentially methylated between the pair of cancer types.

In some embodiments, the at least 10 cancer types comprise at least 2, 3, 4, 5, 10, 12, 14, 16, 18, or 20 cancer types. In some embodiments, the cancer types are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma , hepatobiliary biliary, head and neck cancer, colorectal cancer, cervical cancer, breast cancer, bladder cancer, and anorectal cancer. In some embodiments, the cancer types are selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer. In some embodiments, the cancer types are selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper GI cancer, multiple myeloma, lymphoid neoplasm, and lung cancer. In some embodiments, the at least 200 target genomic regions are selected from any one of lists 1-16. In some embodiments, the at least 200 target genomic regions comprise at least 20%, 30%, 40%, 50%, 60%, 70%, 80%, 90% or 95% of the target genomic regions in any one of lists 1-16. In some embodiments, the at least 200 target genomic regions comprise at least 500, 1,000, 5,000, 10,000, 15,000, 20,000, 30,000, 40,000, or 50,000 target genomic regions in any one of lists 1-16. In some embodiments, the at least 200 target genomic regions are selected from any one of lists 1-3. In some embodiments, the at least 200 target genomic regions comprise at least 20%, 30%, 40%, 50%, 60%, 70%, 80%, 90% or 95% of the target genomic regions in any one of lists 1-3. In some embodiments, the at least 200 target genomic regions comprise at least 500, 1,000, 5,000, 10,000, 15,000, 20,000, 30,000, 40,000, or 50,000 target genomic regions in any one of lists 1-3. In some embodiments, the at least 200 target genomic regions are selected from any one of lists 13-16. In some embodiments, the at least 200 target genomic regions comprise at least 10%, 20%, 25%, 30%, 40%, 50%, 60%, 70%, 80%, 90% or 95% of the target genomic regions in any one of lists 13-16. In some embodiments, the at least 200 target genomic regions comprise at least 500, 1,000, 5,000, 10,000, 15,000, 20,000, 30,000, 40,000, or 50,000 target genomic regions in any one of lists 13-16. In some embodiments, the at least 200 target genomic regions are selected from list 12. In some embodiments, the at least 200 target genomic regions comprise at least 10%, 20%, 25%, 30%, 40%, 50%, 60%, 70%, 80%, 90% or 95% of the target genomic regions in list 12. In some embodiments, the at least 200 target genomic regions comprise at least 500, 1,000, 5,000, 10,000, 15,000, 20,000, 30,000, 40,000, or 50,000 target genomic regions in list 12. In some embodiments, the at least 200 target genomic regions are selected from any one of lists 8-11. In some embodiments, the at least 200 target genomic regions comprise at least 20%, 30%, 40%, 50%, 60%, 70%, 80%, 90% or 95% of the target genomic regions in any one of lists 8-11. In some embodiments, the at least 200 target genomic regions comprise at least 500, 1,000, 5,000, 10,000, 15,000, 20,000, 30,000, 40,000, or 50,000 target genomic regions in any one of lists 8-11. In some embodiments, the at least 200 target genomic regions comprise at least 40%, 50%, 60%, or 70% of the target genomic regions listed in List 4. In some embodiments, wherein the at least 200 target genomic regions comprise, for at least 90% or for 100% of all possible pairs of cancer types selected from a set comprising at least 10 cancer types, at least one target genomic region that is differentially methylated between the pair of cancer types. In some embodiments, the plurality of bait oligonucleotides hybridize to at least 15 nucleotides or to at least 30 nucleotides of the DNA molecules derived from the at least 200 target genomic regions. In some embodiments, the DNA molecules derived from the at least 200 target genomic regions are converted cfDNA fragments. In some embodiments, the cfDNA fragments are converted by a process comprising treatment with bisulfite. In some embodiments, the cfDNA fragments are converted by an enzymatic conversion reaction. In some embodiments, the cfDNA fragments are converted by a cytosine deaminase. In some embodiments, each bait oligonucleotide is conjugated to an affinity moiety. In some embodiments, the affinity moiety is biotin. In some embodiments, each bait oligonucleotide is between 50 and 300 bases in length, between 60 and 200 bases in length, between 100 and 150 bases in length, between 110 and 130 bases in length, and/or 120 bases in length.

Also provided herein are compositions comprising a plurality of different bait oligonucleotides configured to hybridize to DNA molecules derived from at least 100 target genomic regions selected from any one of Lists 1-16.

In some embodiments, the at least 100 target genomic regions comprises at least 200 target genomic regions. In some embodiments, the at least 100 target genomic regions are selected from any one of lists 1-16. In some embodiments, the at least 100 target genomic regions comprise at least 20%, 30%, 40%, 50%, 60%, 70%, 80%, 90% or 95% of the target genomic regions in any one of lists 1-16. In some embodiments, the at least 100 target genomic regions comprise at least 500, 1,000, 5,000, 10,000, 15,000, 20,000, 30,000, 40,000, or 50,000 target genomic regions in any one of lists 1-16. In some embodiments, the at least 100 target genomic regions are selected from any one of lists 1-3. In some embodiments, the at least 100 target genomic regions comprise at least 20%, 30%, 40%, 50%, 60%, 70%, 80%, 90% or 95% of the target genomic regions in any one of lists 1-3. In some embodiments, the at least 100 target genomic regions comprise at least 500, 1,000, 5,000, 10,000, 15,000, 20,000, 30,000, 40,000, or 50,000 target genomic regions in any one of lists 1-3. In some embodiments, the at least 100 target genomic regions are selected from list 12. In some embodiments, the at least 100 target genomic regions comprise at least 10%, 20%, 25%, 30%, 40%, 50%, 60%, 70%, 80%, 90% or 95% of the target genomic regions in list 12. In some embodiments, the at least 100 target genomic regions comprise at least 500, 1,000, 5,000, 10,000, 15,000, 20,000, 30,000, 40,000, or 50,000 target genomic regions in list 12. In some embodiments, the at least 100 target genomic regions are selected from list 8. In some embodiments, the at least 100 target genomic regions comprise at least 20%, 30%, 40%, 50%, 60%, 70%, 80%, 90% or 95% of the target genomic regions in list 8. In some embodiments, the at least 100 target genomic regions comprise at least 500, 1,000, 5,000, 10,000, 15,000, 20,000, 30,000, 40,000, or 50,000 target genomic regions in list 8. In some embodiments, the at least 100 target genomic regions comprise at least 40%, 50%, 60%, or 70% of the target genomic regions listed in List 4. In some embodiments, the DNA molecules derived from the at least 100 target genomic regions are converted cfDNA fragments. In some embodiments, the cfDNA fragments are converted by a process comprising treatment with bisulfite. In some embodiments, the composition further comprises cfDNA fragments from a test subject. In some embodiments, the cfDNA fragments from the test subject are converted cfDNA molecules. In some embodiments, the cfDNA fragments from the test subject are converted by a process comprising treatment with bisulfite. In some embodiments, each target genomic region comprises at least 5 CpG dinucleotides. In some embodiments, each bait oligonucleotide is between 60 and 200 bases in length, between 100 and 150 bases in length, between 110 and 130 bases in length, and/or 120 bases in length. In some embodiments, the different bait oligonucleotides comprise a plurality of sets of two or more bait oligonucleotides, wherein each bait oligonucleotide within a set of bait oligonucleotides is configured to bind to the converted DNA molecules from the same target genomic region. In some embodiments, the ratio of bait oligonucleotides configured to hybridize to hypermethylated target regions to bait oligonucleotides configured to hybridize to hypomethylated target regions is between 0.5 and 1.0. In some embodiments, each set of bait oligonucleotides comprises one or more pairs of a first bait oligonucleotide and a second bait oligonucleotide, each bait oligonucleotide comprises a 5' end and a 3' end, a sequence of at least X nucleotide bases at the 3' end of the first bait oligonucleotide is identical to a sequence of X nucleotide bases at the 5' end the second bait oligonucleotide, and X is at least 20, at least 25, or at least 30. In some embodiments, X is 30.

Also provided herein are methods for enriching a cfDNA sample, the method comprising: contacting a converted or unconverted cfDNA sample with a bait set described above and enriching the sample for cfDNA corresponding to a first set of genomic regions by hybridization capture. In some embodiments, the cfDNA sample is a converted cfDNA sample

Also provided herein are methods for obtaining sequence information informative of a presence or absence of cancer or a type of cancer, the method comprising sequencing enriched converted cfDNA prepared by a method comprising contacting a converted or unconverted cfDNA sample with a bait set described above; and enriching the sample for cfDNA corresponding to a first set of genomic regions by hybridization capture. In some embodiments, the cfDNA sample is a converted cfDNA sample

Also described herein are methods of determining a presence or absence of cancer in a subject, the method comprising capturing cfDNA fragments from the subject with a composition described above, sequencing the captured cfDNA fragments, and applying a trained classifier to the cfDNA sequences to determine the presence or absence of cancer. In some embodiments, the likelihood of a false positive determination of a presence or absence of cancer is less than 1% and the likelihood of an accurate determination of a presence or absence of cancer is at least 40%. In some embodiments, the cancer is a stage I cancer, the likelihood of a false positive determination of a presence or absence of cancer is less than 1%, and the likelihood of an accurate determination of a presence or absence of cancer is at least 10%. In some embodiments, the cfDNA fragments are converted cfDNA fragments.

Also provided herein are methods of detecting a cancer type comprising capturing cfDNA fragments from a subject with a composition comprising a plurality of different oligonucleotide baits, sequencing the captured cfDNA fragments, and applying a trained classifier to the cfDNA sequences to determine a cancer type; wherein the oligonucleotide baits are configured to hybridize to cfDNA fragments derived from a plurality of target genomic regions, wherein the plurality of target genomic regions is differentially methylated in one or more cancer types relative to a different cancer type or a non-cancer type, wherein the likelihood of a false-positive determination of cancer is less than 1%, and wherein the likelihood of an accurate assignment of a cancer type is at least 75%, at least 80%, at least 85% or at least 89%, or at least 90%. Some embodiments further comprise applying the trained classifier to the cfDNA sequences to determine a presence of cancer before determining the cancer type.

In some embodiments, the cancer type is a stage I cancer type, and the likelihood of an accurate assignment is at least 75%. In some embodiments, the cancer type is a stage II cancer type, and the likelihood of an accurate assignment is at least 85%. In some embodiments, the cancer type is prostate cancer and the likelihood of an accurate assignment of prostate cancer is at least 85% or at least 90%. the cancer type is breast cancer and the likelihood of an accurate assignment of breast cancer is at least 90% or at least 95%. In some embodiments, the cancer type is uterine cancer and the likelihood of an accurate assignment of uterine cancer is at least 90% or at least 95%. In some embodiments, the cancer type is ovarian cancer and the likelihood of an accurate assignment of ovarian cancer is at least 85% or at least 90%. In some embodiments, the cancer type is bladder & urothelial cancer and the likelihood of an accurate assignment of bladder & urothelial is at least 90% or at least 95%. In some embodiments, the cancer type is colorectal cancer and the likelihood of an accurate assignment of colorectal cancer is at least 65% or at least 70%. In some embodiments, the cancer type is liver & bile duct cancer and the likelihood of an accurate assignment of liver & bile duct cancer is at least 90% or at least 95%. In some embodiments, the cancer type is pancreas & gallbladder cancer and the likelihood of an accurate assignment of pancreas & gallbladder cancer is at least 85% or at least 90%. In some embodiments, the cfDNA fragments are converted cfDNA fragments. In some embodiments, the cancer type is selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer. In some embodiments, the cancer type is selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer. In some embodiments, the cancer type is selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper GI cancer, multiple myeloma, lymphoid neoplasm, and lung cancer. In some embodiments, the cancer type is sarcoma and the likelihood of a detecting sarcoma is at least 35% or at least 40%. In some embodiments, the likelihood of detecting stage III or stage IV renal cancer is at least 50% or at least 70%. In some embodiments, the likelihood of detecting stage III or stage IV breast cancer is at least 70% or at least 85%. In some embodiments, the likelihood of detecting stage III or stage IV uterine cancer is at least 50%. In some embodiments, the likelihood of detecting ovarian cancer is at least 60% or at least 80%. In some embodiments, the likelihood of detecting bladder cancer is at least 35% or at least 40%. In some embodiments, the likelihood of detecting anorectal cancer is at least 60% or 70%. In some embodiments, the likelihood of detecting head and neck cancer is at least 75% or at least 80%. In some embodiments, the likelihood of detecting stage 1 head and neck cancer is at least 80%. In some embodiments, the likelihood of detecting colorectal cancer is at least 50% or at least 59%. In some embodiments, the likelihood of detecting liver cancer is at least 75% or 80%. In some embodiments, the likelihood of detecting pancreas and gallbladder cancer is at least 64% or at least 70%. In some embodiments, the likelihood of detecting upper GI cancer is at least at least 60% or at least 68%. In some embodiments, the likelihood of detecting multiple myeloma is at least 65% or at least 75%. In some embodiments, the likelihood of detecting type I multiple myeloma is at least 60%. In some embodiments, the likelihood of detecting lymphoid neoplasm is at least 65% or at least 69%. In some embodiments, the likelihood of detecting lung cancer is at least 50% or at least 58%. In some embodiments, the composition comprising oligonucleotide baits is a composition provided above. In some embodiments, the plurality of genomic regions comprises no more than 95,000 genomic regions, no more than 60,000 genomic regions, no more than 40,000 genomic regions, no more than 35,000 genomic regions, no more than 20,000 genomic regions, no more than 15,000 genomic regions, no more than 8,000 genomic regions, no more than 4,000 genomic regions, no more than 2,000 genomic regions, or no more than 1,400 genomic regions. In some embodiments, the total size of the plurality of genomic regions is less than 4 MB, less than 2 MB, less than 1 MB, less than 0.7 MB, or less than 0.4 MB. In some embodiments, the subject has an elevated risk of one or more cancer types. In some embodiments, the subject manifests symptoms associated with one or more cancer types. In some embodiments, the subject has not been diagnosed with a cancer. In some embodiments, the classifier was trained on converted DNA sequences derived from a least 100 subjects with a first cancer type, at least 100 subjects with a second cancer type, and at least 100 subjects with no cancer. In some embodiments, the first cancer type is ovarian cancer. In some embodiments, the first cancer type is liver cancer. In some embodiments, the first cancer type is selected from thyroid cancer, melanoma, sarcoma, myeloid neoplasm, renal cancer, prostate cancer, breast cancer, uterine cancer, ovarian cancer, bladder cancer, urothecal cancer, cervical cancer, anorectal cancer head & neck cancer, colorectal cancer, liver cancer, pancreatic cancer, gallbladder cancer, esophageal cancer, stomach cancer, multiple myeloma, lymphoid neoplasm, lung cancer, or leukemia. In some embodiments, the classifier was trained on converted DNA sequences derived from at least 1000, at least 2000, or at least 4000 target genomic regions selected from any one of Lists 1-16.

**In** some embodiments, the classifier is trained on converted DNA sequences derived from at least 1000, at least 2000, or at least 4000 target genomic regions selected from any one of Lists 1-16. In some embodiments the trained classifier determines the presence or absence of cancer or a cancer type by (a) generating a set of features for the sample, wherein each feature in the set of features comprises a numerical value; (b) inputting the set of features into the classifier, wherein the classifier comprises a multinomial classifier; (c) based on the set of features, determining, at the classifier, a set of probability scores, wherein the set of probability scores comprises one probability score per cancer type class and per non-cancer type class; and (d) thresholding the set of probability scores based on one or more values determined during training of the classifier to determine a final cancer classification of the sample. In some embodiments, the set of features comprises a set of binarized features. In some embodiments, the numerical value comprises a single binary value. In some embodiments, the multinomial classifier comprises a multinomial logistic regression ensemble trained to predict a source tissue for the cancer. In some embodiments, the classifier determines a final cancer classification based on a top-two probability score differential relative to a minimum value, wherein the minimum value corresponds to a predefined percentage of training cancer samples that had been assigned the correct cancer type as their highest score during training of the classifier. In some embodiments, the classifier assigns a cancer label corresponding to the highest probability score determined by the classifier as the final cancer classification when it is determined that the top-two probability score differential exceeds the minimum value; and assigns an indeterminate cancer label as the final cancer classification when it is determined that the top-two probability score differential does not exceed the minimum value.

Also provided herein are methods of treating a type of cancer in a subject in need thereof, the method comprising detecting the type of cancer by a method described above, and administering an anti-cancer therapeutic agent to the subject. In some embodiments, the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

Also provided herein are cancer assay panels comprising: at least 500 pairs of probes, wherein each pair of the at least 500 pairs comprise two probes configured to overlap each other by an overlapping sequence, wherein the overlapping sequence comprises a 30-nucleotide sequence, and wherein the 30-nucleotide sequence is configured to hybridize to a converted cfDNA molecule corresponding to, or derived from one or more of genomic regions, wherein each of the genomic regions comprises at least five methylation sites, and wherein the at least five methylation sites have an abnormal methylation pattern in cancerous samples.

In some embodiments, each of the at least 500 pairs of probes is conjugated to a non-nucleotide affinity moiety. In some embodiments, the non-nucleotide affinity moiety is a biotin moiety. In some embodiments, the cancerous samples are from subjects having cancer selected from the group consisting of breast cancer, uterine cancer, cervical cancer, ovarian cancer, bladder cancer, urothelial cancer of renal pelvis, renal cancer other than urothelial, prostate cancer, anorectal cancer, colorectal cancer, hepatobiliary cancer arising from hepatocytes, hepatobiliary cancer arising from cells other than hepatocytes, pancreatic cancer, squamous cell cancer of the upper gastrointestinal tract, upper gastrointestinal cancer other than squamous, head and neck cancer, lung adenocarcinoma, small cell lung cancer, squamous cell lung cancer and cancer other than adenocarcinoma or small cell lung cancer, neuroendocrine cancer, melanoma, thyroid cancer, sarcoma, multiple myeloma, lymphoma, and leukemia. In some embodiments, the abnormal methylation pattern has at least a threshold p-value rarity in the cancerous samples. In some embodiments, each of the probes is designed to have less than 20 off-target genomic regions. In some embodiments, the less than 20 off-target genomic regions are identified using a k-mer seeding strategy. In some embodiments, the less than 20 off-target genomic regions are identified using k-mer seeding strategy combined to local alignment at seed locations. In some embodiments, the cancer assay panel comprises at least 10,000, 50,000, 100,000, 200,000, 300,000, 400,000, 500,000, 600,000, 700,000 or 800,000 probes. In some embodiments, the at least 500 pairs of probes together comprise at least 2 million, 3 million, 4 million, 5 million, 6 million, 8 million, 10 million, 12 million, 14 million, or 15 million nucleotides. In some embodiments, each of the probes comprises at least 50, 75, 100, or 120 nucleotides. In some embodiments, each of the probes comprises less than 300, 250, 200, or 150 nucleotides. In some embodiments, each of the probes comprises 100-150 nucleotides. In some embodiments, each of the probes comprises less than 20, 15, 10, 8, or 6 methylation sites. In some embodiments, at least 80, 85, 90, 92, 95, or 98% of the at least five methylation sites are either methylated or unmethylated in the cancerous samples. In some embodiments, at least 3%, 5%, 10%, 15%, or 20% of the probes comprise no G (Guanine). In some embodiments, each of the probes comprise multiple binding sites to the methylation sites of the converted cfDNA molecule, wherein at least 80, 85, 90, 92, 95, or 98% of the multiple binding sites comprise exclusively either CpG or CpA. In some embodiments, each of the probes is configured to have less than 15, 10 or 8 off-target genomic regions. In some embodiments, at least 30% of the genomic regions are in exons or introns. In some embodiments, at least 15% of the genomic regions are in exons. In some embodiments, at least 20% of the genomic regions are in exons. In some embodiments, less than 10% of the genomic regions are in intergenic regions. In some embodiments, the genomic regions are selected from any one of Lists 1-3 or Lists 4-16. In some embodiments, the genomic regions comprise at least 20%, 30%, 40%, 50%, 60%, 70%, 80%, 90% or 95% of the genomic regions in any one of Lists 1-3 or Lists 4-16. In some embodiments, the genomic regions comprise at least 500, 1,000, 5000, 10,000, or 15,000, 20,000, 30,000, 40,000, 50,000, 60,000, or 70,000 genomic regions in any one of Lists 1-3 or Lists 4-16.

Also provided herein are cancer assay panels comprising a plurality of probes, wherein each of the plurality of probes is configured to hybridize to a converted cfDNA molecule corresponding to one or more of the genomic regions in any one of Lists 1-3 or Lists 4-16.

In some embodiments, the plurality of probes together is configured to hybridize to a plurality of converted cfDNA molecules corresponding to at least 20%, 30%, 40%, 50%, 60%, 70%, 80%, or 90%, 95% or 100% of the genomic regions of any one of Lists 1-3 or Lists 4-16.

In some embodiments, the plurality of probes together is configured to hybridize to a plurality of converted cfDNA molecules corresponding to at least 500, 1,000, 5000, 10,000, 15,000, 20,000, 30,000, 40,000, or 50,000 genomic regions of any one of Lists 1-3 or Lists 4-16. In some embodiments, at least 3%, 5%, 10%, 15%, or 20% of the probes comprise no G (Guanine). In some embodiments, each of the probes comprise multiple binding sites to methylation sites of the converted cfDNA molecule, wherein at least 80, 85, 90, 92, 95, or 98% of the multiple binding sites comprise exclusively either CpG or CpA. In some embodiments, each of the probes is conjugated to a non-nucleotide affinity moiety. In some embodiments, the non-nucleotide affinity moiety is a biotin moiety.

Also provided herein are methods of determining a tissue of origin (TOO) of a cancer, comprising: receiving a sample comprising a plurality of cfDNA molecules; treating the plurality of cfDNA molecules to convert unmethylated C (cytosine) to U (uracil), thereby obtaining a plurality of converted cfDNA molecules; applying a cancer assay panel provided above to the plurality of converted cfDNA molecules, thereby enriching a subset of the converted cfDNA molecules; and sequencing the enriched subset of the converted cfDNA molecule, thereby providing a set of sequence reads.

Some embodiments further comprise the step of: determining a health condition by evaluating the set of sequence reads, wherein the health condition is a presence or absence of cancer; a presence or absence of cancer of a tissue of origin (TOO); a presence or absence of a cancer cell type; or a presence or absence of at least 5, 10, 15, or 20 different types of cancer. In some embodiments, the sample comprising a plurality of cfDNA molecules was obtained from a human subject.

Also provided herein are methods for detecting a cancer, comprising the steps of: obtaining a set of sequence reads by sequencing a set of nucleic acid fragments from a subject, wherein the nucleic acid fragments are corresponding to, or derived from a plurality of genomic regions selected from any one of Lists 1-3 or Lists 4-16; for each of the nucleic acid fragments, determining methylation status at a plurality of CpG sites; and detecting a health condition of the subject by evaluating the methylation status for the sequence reads, wherein the health condition is (i) a presence or absence of cancer; (ii) a presence or absence of cancer of a tissue of origin (TOO); (iii) a presence or absence of a cancer cell type; or (iv) a presence or absence of at least 5, 10, 15, or 20 different types of cancer.

In some embodiments, the plurality of genomic regions comprises at least 20%, 30%, 40%, 50%, 60%, 70%, 80%, 90%, 95%, or 100% of the genomic regions of any one of Lists 1-3 or lists 4-16. In some embodiments, the plurality of genomic regions comprises 500, 1,000, 5000, 10,000, 15,000, 20,000, 30,000, 40,000, 50,000, 60,000, 70,000, or 80,000 of the genomic regions of any one of Lists 1-3 or Lists 4-16.

Also provided herein are methods of designing a cancer assay panel for diagnosing cancer of a tissue of origin (TOO) comprising the steps of: identifying a plurality of genomic regions, wherein each of the plurality of genomic regions (i) comprises at least 30 nucleotides, and (ii) comprises at least five methylation sites, selecting a subset of the genomic regions, wherein the selection is made when cfDNA molecules corresponding to, or derived from each of the genomic regions in cancerous samples have an abnormal methylation pattern, wherein the abnormal methylation pattern comprises at least five methylation sites either hypomethylated or hypermethylated, and designing a cancer assay panel comprising a plurality of probes, wherein each of the probes is configured to hybridize to a converted cfDNA molecule corresponding to or derived from one or more of the subset of the genomic regions.

Also provided herein are bait sets for hybridization capture, a bait set comprising a plurality of different oligonucleotide-containing probes, wherein each of the oligonucleotide-containing probes comprises a sequence of at least 30 bases in length that is complementary to either: (1) a sequence of a genomic region; or (2) a sequence that varies from the sequence of (1) only by one or more transitions, wherein each respective transition of the one or more transitions occurs at a cytosine in the genomic region, and wherein each probe of the different oligonucleotide-containing probes is complementary to a sequence corresponding to a CpG site that is differentially methylated in samples from subjects with a first cancer type relative to samples from subjects with a second cancer type or a non-cancer type.

In some embodiments, the first cancer type and the second cancer type are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary hepatocellular carcinoma, hepatobiliary biliary, head and neck cancer, colorectal cancer, cervical cancer, breast cancer, bladder cancer, and anorectal cancer.

The bait set of any of claims 140-141, wherein the bait set comprises at least 500, 1,000, 2,000, 2,500, 5,000, 6,000, 7,500, 10,000, 15,000, 20,000, 25,000, 50,000, 100,000, 200,000, 300,000, 500,000, or 800,000 different oligonucleotide-containing probes. In some embodiments, for each of the different oligonucleotide-containing probes, the sequence of at least 30 bases in length is complementary to either (1) a sequence within a genomic region selected from the genomic regions set forth in any one of Lists 1-16; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in any one of Lists 1-3; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in in any one of Lists 5 or 7; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in any one of Lists 4, 8, or 8-12; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in any one of Lists 13-16; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in any one of Lists 13-16; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in any one of Lists 4 or 6; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in List 4; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in List 8; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in List 9; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in List 10; or (2) a sequence that varies from the sequence of (1) only by one or more transitions, wherein each respective transition of the one or more transitions occurs at a cytosine in the genomic region. **In** some embodiments, the sequence of at least 30 bases in length is complementary to either (1) a sequence within a genomic region selected from the genomic regions set forth in List 11; or (2) a sequence that varies from the sequence of (1) only by one or more transitions, wherein each respective transition of the one or more transitions occurs at a cytosine in the genomic region. In some embodiments, the sequence of at least 30 bases in length is complementary to either (1) a sequence within a genomic region selected from the genomic regions set forth in List 12; or (2) a sequence that varies from the sequence of (1) only by one or more transitions, wherein each respective transition of the one or more transitions occurs at a cytosine in the genomic region. In some embodiments, the plurality of different oligonucleotide-containing probes are each conjugated to an affinity moiety. In some embodiments, the affinity moiety is biotin. In some embodiments, at least 80%, 90%, or 95% of the oligonucleotide-containing probes in the bait set do not include an at least 30, at least 40, or at least 45 base sequence that has 20 or more off-target regions in the genome. In some embodiments, the oligonucleotide-containing probes in the bait set do not include an at least 30, at least 40, or at least 45 base sequence that has 20 or more off-targets regions in the genome. In some embodiments, the sequence of at least 30 bases of each of the probes is at least 40 bases, at least 45 bases, at least 50 bases, at least 60 bases, at least 75, or at least 100 bases in length. In some embodiments, each of the oligonucleotide-containing probes has a nucleic acid sequence of at least 45, 40, 75, 100, or 120 bases in length. In some embodiments, each of the oligonucleotide-containing probes have a nucleic acid sequence of no more than 300, 250, 200, or 150 bases in length. In some embodiments, each of the plurality of different oligonucleotide-containing probes is between 60 and 200 bases in length, between 100 and 150 bases in length, between 110 and 130 bases in length, and/or 120 bases in length. In some embodiments, the different oligonucleotide-containing probes comprise at least 500, at least 1000, at least 2,000, at least 2,500, at least 5,000, at least 6,000, at least 7,500, and least 10,000, at least 15,000, at least 20,000, or at least 25,000 different pairs of probes, wherein each pair of probes comprises a first probe and second probe, wherein the second probe differs from the first probe and overlaps with the first probe by an overlapping sequence that is at least 30, at least 40, at least 50, or at least 60 nucleotides in length. In some embodiments, the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 1-16. In some embodiments, the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 1-3. In some embodiments, the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 4-12. In some embodiments, the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 4, 6, or 8-12. In some embodiments, the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in List 8. In some embodiments, an entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 1-16. In some embodiments, the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 1-3. In some embodiments, the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 4-12. In some embodiments, the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 4, 6, or 8-12. In some embodiments, the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from List 8. In some embodiments, an entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 1-16. In some embodiments, the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 1-3. In some embodiments, the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 4-12. In some embodiments, the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 4, 6, or 8-12. In some embodiments, the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in List 8. In some embodiments, the plurality of oligonucleotide-containing probes comprise at least 500, 1,000, 5,000, or 10,000 different subsets of probes, wherein each subset of probes comprises a plurality of probes that collectively extend across a genomic region selected from the genomic regions of any one of Lists 1-16 in a 2× tiled fashion. In some embodiments, plurality of oligonucleotide-containing probes comprise at least 500, 1,000, 5,000, or 10,000 different subsets of probes, wherein each subset of probes comprises a plurality of probes that collectively extend across a genomic region selected from the genomic regions of any one of Lists 1-4, 6, or 8-12 in a 2× tiled fashion. In some embodiments, the plurality of probes that collectively extend across the genomic region in a 2× tiled fashion comprises at least one pair of probes that overlap by a sequence of at least 30 bases, at least 40 bases, at least 50 bases, or at least 60 bases in length. In some embodiments, the plurality of probes collectively extend across portions of the genome that collectively are a combined size of less than 4 MB, less than 2 MB, less than 1 MB, less than 0.7 MB, or less than 0.4 MB. In some embodiments, the plurality of probes collectively extend across portions of the genome that collectively are a combined size of between 0.2 and 30 MB, between 0.5 MB and 30 MB, between 1 MB and 30 MB, between 3 MB and 25 MB, between 3 MB and 15, MB, between 5 MB and 20 MB, or between 7 MB and 12 MB. In some embodiments, each of the different oligonucleotide-containing probes comprises less than 20, 15, 10, 8, or 6 CpG detection sites. In some embodiments, at least 80%, 85%, 90%, 92%, 95%, or 98% of the plurality of oligonucleotide-containing probes have exclusively either CpG or CpA on all CpG detection sites.

Also provided herein are mixtures comprising: converted cfDNA; and a bait set provided above. In some embodiments, the converted cfDNA comprises bisulfite-converted cfDNA.

The mixture of claim 187, wherein the converted cfDNA comprises cfDNA that has been converted via a cytosine deaminase.

Also provided herein are methods for enriching a converted cfDNA sample, a method comprising: contacting the converted cell-free DNA sample with a bait set provided above; and enriching the sample for a first set of genomic regions by hybridization capture.

Also provided herein are methods for providing sequence information informative of a presence or absence of a cancer or a type of cancer, the method comprising: processing cfDNA from a biological sample with a deaminating agent to generate a cell-free DNA sample comprising deaminated nucleotides; enriching the cfDNA sample for informative cell-free DNA molecules; and sequencing the enriched cfDNA molecules, thereby obtaining a set of sequence reads informative of a presence or absence of a cancer or a type of cancer.

In some embodiments, enriching the cfDNA comprises amplifying, via PCR, portions of the cell-free DNA fragments using primers configured to hybridize to a plurality of genomic regions selected from any one of Lists 1-16. In some embodiments, enriching the cfDNA sample comprises contacting the cell-free DNA with a plurality of probes configured to hybridize to converted fragments obtained from the cfDNA molecules corresponding to or derived from the genomic regions in any one of Lists 1-16. In some embodiments, the cfDNA sample comprises contacting the cell-free DNA with a plurality of probes configured to hybridize to converted fragments obtained from the cfDNA molecules corresponding to or derived from at least 30%, 40%, 50%, 60%, 70%, 80%, 90%, 95% of the genomic regions in any one of Lists 1-16. In some embodiments, the genomic regions are selected from any one of Lists 1-3. In some embodiments, the genomic regions are selected from any one of Lists 4-12. In some embodiments, the genomic regions are selected from any one of Lists 4, 6, or 8-12. In some embodiments, the genomic regions are selected from List 8. In some embodiments, the cfDNA sample is enriched by a method provided above. In some embodiments, the method further comprises determining a cancer classification by evaluating the set of sequence reads, wherein the cancer classification is a presence or absence of cancer; or a presence or absence of a type of cancer. In some embodiments, the step of determining a cancer classification comprises: generating a test feature vector based on the set of sequence reads; and applying the test feature vector to a classifier. In some embodiments, the classifier comprises a model that is trained by a training process with a first cancer set of fragments from one or more training subjects with a first cancer type and a second cancer set of fragments from one or more training subjects with a second cancer type, wherein both the first cancer set of fragments and the second cancer set of fragments comprise a plurality of training fragments. In some embodiments, the cancer classification is a presence or absence of cancer. In some embodiments, has an area under a receiver operating characteristic curve of at least 0.8. In some embodiments, the cancer classification is a type of cancer. In some embodiments, the type of cancer is selected from among at least 12, 14, 16, 18, or 20 cancer types. In some embodiments, the cancer types are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary hepatocellular carcinoma, hepatobiliary biliary, head and neck cancer, colorectal cancer, cervical cancer, breast cancer, bladder cancer, and anorectal cancer. In some embodiments, the cancer types are selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer. In some embodiments, the cancer types are selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer gallbladder cancer, upper GI cancer, multiple myeloma, lymphoid neoplasm, and lung cancer. In some embodiments, at 99% specificity the sensitivity of the method for head and neck cancer is at least 79% or at least 84%; at 99% specificity the sensitivity of the method for liver cancer is at least 82% or at least 85%; at 99% specificity the sensitivity of the method for upper GI tract cancer is at least 62% or at least 68%; wherein at 99% specificity the sensitivity of the method for pancreatic or gallbladder cancer is at least 62% or at least 68%; at 99% specificity the sensitivity of the method for colorectal cancer is at least 60% or at least 65%; at 99% specificity the sensitivity of the method for ovarian cancer is at least 75% or at least 80%; at 99% specificity the sensitivity of the method for lung cancer is at least 60% or at least 65%; at 99% specificity the sensitivity of the method for multiple myeloma is at least 68% or at least 75%; at 99% specificity the sensitivity of the method for lymphoid neoplasm is at least 65% or at least 70%; at 99% specificity the sensitivity of the method for anorectal cancer is at least 60% or at least 65%; and at 99% specificity the sensitivity of the method for bladder cancer is at least 40% or at least 44%. In some embodiments, the cancer classification is a presence or absence of a type of cancer. In some embodiments, the step of determining a cancer classification comprises: generating a test feature vector based on the set of sequence reads; and applying the test feature vector to a classifier. In some embodiments, the classifier comprises a model that is trained by a training process with a first cancer type set of converted DNA sequences from one or more training subjects with a first cancer type and a second cancer type set of converted DNA sequences from one or more training subjects with a second cancer type, wherein both the first cancer type set of converted DNA sequences and the second cancer type set of converted DNA sequences comprise a plurality of training converted DNA sequences. In some embodiments, the type of cancer is selected from the group consisting of head and neck cancer, liver/bile duct cancer, upper GI cancer, pancreatic/gallbladder cancer; colorectal cancer, ovarian cancer, lung cancer, multiple myeloma, lymphoid neoplasms, melanoma, sarcoma, breast cancer, and uterine cancer. In some embodiments, the type of cancer is head and neck cancer, and the method, at 99.0% specificity, has a sensitivity of at least 79% or at least 84%. the type of cancer is liver cancer, and the method, at 99.0% specificity, has a sensitivity of at least 82% or at least 85%. In some embodiments, the type of cancer is an upper GI tract cancer, and the method, at 99.0% specificity, has a sensitivity of at least 62% or at least 68%. In some embodiments, the type of cancer is a pancreatic or gallbladder cancer, and the method, at 99.0% specificity, has a sensitivity of at least 62% or at least 68%. In some embodiments, the type of cancer is colorectal cancer, and the method, at 99.0% specificity, has a sensitivity of at least 60% or at least 65%. In some embodiments, the type of cancer is ovarian cancer, and the method, at 99.0% specificity, has a sensitivity of at least 75% or at least 80%. In some embodiments, the type of cancer is lung cancer, and the method, at 99.0% specificity, has a sensitivity of at least 60% or at least 65%. In some embodiments, the type of cancer is multiple myeloma, and the method, at 99.0% specificity, has a sensitivity of at least 68% or at least 75%. In some embodiments, the type of cancer is a lymphoid neoplasm, and the method, at 99.0% specificity, has a sensitivity of at least 65% or at least 70%. In some embodiments, the type of cancer is anorectal cancer, and the method, at 99.0% specificity, has a sensitivity of at least 60% or at least 65%. In some embodiments, the type of cancer is bladder cancer, and the method, at 99.0% specificity, has a sensitivity of at least 40% or at least 44%. In some embodiments, the total size of the target genomic regions is less than 4 Mb, less than 2 Mb, less than 1 Mb, less than 0.7 Mb or less than 0.4 Mb. In some embodiments, the step of determining a cancer classification comprises:

generating a test feature vector based on the set of sequence reads; and applying the test feature vector to a model obtained by a training process with a cancer set of fragments from one or more training subjects with a cancer and a non-cancer set of fragments from one or more training subjects without cancer, wherein both the cancer set of fragments and the non-cancer set of fragments comprise a plurality of training fragments. In some embodiments, the training process comprises: obtaining sequence information of training fragments from a plurality of training subjects; for each training fragment, determining whether that training fragment is hypomethylated or hypermethylated, wherein each of the hypomethylated and hypermethylated training fragments comprises at least a threshold number of CpG sites with at least a threshold percentage of the CpG sites being unmethylated or methylated, respectively, for each training subject, generating a training feature vector based on the hypomethylated training fragments and hypermethylated training fragments, and training the model with the training feature vectors from the one or more training subjects without cancer and the training feature vectors from the one or more training subjects with cancer. In some embodiments, the training process comprises: obtaining sequence information of training fragments from a plurality of training subjects; for each training fragment, determining whether that training fragment is hypomethylated or hypermethylated, wherein each of the hypomethylated and hypermethylated training fragments comprises at least a threshold number of CpG sites with at least a threshold percentage of the CpG sites being unmethylated or methylated, respectively, for each of a plurality of CpG sites in a reference genome: quantifying a count of hypomethylated training fragments which overlap the CpG site and a count of hypermethylated training fragments which overlap the CpG site; and generating a hypomethylation score and a hypermethylation score based on the count of hypomethylated training fragments and hypermethylated training fragments; for each training fragment, generating an aggregate hypomethylation score based on the hypomethylation score of the CpG sites in the training fragment and an aggregate hypermethylation score based on the hypermethylation score of the CpG sites in the training fragment; for each training subject: ranking the plurality of training fragments based on aggregate hypomethylation score and ranking the plurality of training fragments based on aggregate hypermethylation score; and generating a feature vector based on the ranking of the training fragments; obtaining training feature vectors for one or more training subjects without cancer and training feature vectors for the one or more training subjects with cancer; and training the model with the feature vectors for the one or more training subjects without cancer and the feature vectors for the one or more training subjects with cancer. In some embodiments, the model comprises one of a kernel logistic regression classifier, a random forest classifier, a mixture model, a convolutional neural network, and an autoencoder model. In some embodiments, the method further comprises the steps of: obtaining a cancer probability for the test sample based on the model; and comparing the cancer probability to a threshold probability to determine whether the test sample is from a subject with cancer or without cancer. In some embodiments, the method further comprises the steps of: obtaining a cancer type probability for the test sample based on the model; and comparing the cancer type probability to a threshold probability to determine whether the test sample is from a subject with the cancer type or another cancer type or without cancer. In some embodiments, the method further comprises administering an anti-cancer agent to the subject.

Also provided herein are of treating a cancer patient, the method comprising:

administering an anti-cancer agent to a subject who has been identified as a cancer subject by a method provided above. In some embodiments, the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

Also provided herein are methods of treating a cancer patient, the method comprising administering an anti-cancer agent to a subject who has been identified as a cancer subject by a method provided herein. In some embodiments, the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

Also provided herein are methods for assessing whether a subject has a cancer, the method comprising: obtaining cfDNA from the subject; isolating a portion of the cfDNA from the subject by hybridization capture; obtaining sequence reads derived from the captured cfDNA to determine methylation states cfDNA fragments; applying a classifier to the sequence reads; and determining whether the subject has cancer based on application of the classifier; wherein the classifier has an area under the receiver operator characteristic curve of at least 0.80. In some embodiments, the method further comprises determining a cancer type, wherein the sensitivity of the method for head and neck cancer is at least 79% or at least 84%; wherein the sensitivity of the method for liver cancer is at least 82% or at least 85%; wherein the sensitivity of the method for upper GI tract cancer is at least 62% or at least 68%; wherein the sensitivity of the method for pancreatic or gallbladder cancer is at least 62% or at least 68%%; wherein the sensitivity of the method for colorectal cancer is at least 60% or at least 65%; wherein the sensitivity of the method for ovarian cancer is at least 75% or at least 80%; wherein the sensitivity of the method for lung cancer is at least 60% or at least 65%; wherein the sensitivity of the method for multiple myeloma is at least 68% or at least 75%; wherein the sensitivity of the method for lymphoid neoplasm is at least 65% or at least 70%; wherein the sensitivity of the method for anorectal cancer is at least 60% or at least 65%; and wherein the sensitivity of the method for bladder cancer is at least 40% or at least 44%. In some embodiments, the total size of the target genomic regions is less than 4 Mb, less than 2 Mb, less than 1 Mb, less than 0.7 Mb or less than 0.4 Mb. In some embodiments, the method further comprises converting unmethylated cytosines in the cfDNA to uracil prior to isolating the portion of the cfDNA from the subject by hybridization capture. In some embodiments, the method further comprises unmethylated cytosines in the cfDNA to uracil after isolating the portion of the cfDNA from the subject by hybridization capture. In some embodiments, the classifier is a binary classifier. In some embodiments, the classifier is a mixture model classifier. In some embodiments, isolating a portion of the cfDNA from the subject by hybridization capture comprises contacting the cell-free DNA with a bait set comprising a plurality of different oligonucleotide-containing probes. In some embodiments, the bait set is a bait set provided herein.

Also provided herein are methods comprising the steps of: obtaining a set of sequence reads of modified test fragments, wherein the modified test fragments are or have been obtained by processing a set of nucleic acid fragments from a test subject, wherein each of the nucleic acid fragments corresponds to or is derived from a plurality of genomic regions selected from any one of Lists 1-16; and applying the set of sequence reads or a test feature vector obtained based on the set of sequence reads to a model obtained by a training process with a first set of fragments from a plurality of training subjects with a first cancer type and a second set of fragments from a plurality of training subjects with a second cancer type, wherein both the first set of fragments and the second set of fragments comprise a plurality of training fragments.

In some embodiments, the model comprises one of a kernel logistic regression classifier, a random forest classifier, a mixture model, a convolutional neural network, and an autoencoder model. In some embodiments, the set of sequence reads is obtained by using an assay panel provided above.

## INCORPORATION BY REFERENCE

All publications, patents, and patent applications mentioned in this specification are herein incorporated by reference to the same extent as if each individual publication, patent, or patent application was specifically and individually indicated to be incorporated by reference.

## BRIEF DESCRIPTION OF THE DRAWINGS

The novel features of the disclosure are set forth with particularity in the appended claims. A better understanding of the features and advantages of the present disclosure will be obtained by reference to the following detailed description that sets forth illustrative embodiments, in which the principles of the disclosure are utilized, and the accompanying drawings of which:

FIG. 1A illustrates a 2x tiled probe design, with three probes targeting a small target region, where each base in a target region (boxed in the dotted rectangle) is covered by at least two probes, according to an embodiment.

FIG. 1B illustrates a 2x tiled probe design, with more than three probes targeting a larger target region, where each base in a target region (boxed in the dotted rectangle) is covered by at least two probes, according to an embodiment.

FIG. 1C illustrates probe design targeting hypomethylated and/or hypermethylated fragments in genomic regions, according to an embodiment.

FIG. 2 illustrates a process of generating a cancer assay panel, according to an embodiment.

FIG. 3A is a flowchart describing a process of creating a data structure for a control group, according to an embodiment.

FIG. 3B is a flowchart describing an additional step of validating the data structure for the control group of FIG. 3A, according to an embodiment.

FIG. 4 is a flowchart describing a process for selecting genomic regions for designing probes for a cancer assay panel, according to an embodiment.

FIG. 5 is an illustration of an example p-value score calculation, according to an embodiment.

FIG. 6A is a flowchart describing a process of training a classifier based on hypomethylated and hypermethylated fragments indicative of cancer, according to an embodiment.

FIG. 6B is a flowchart describing a process of identifying fragments indicative of cancer determined by probabilistic models, according to an embodiment.

FIG. 7A is a flowchart describing a process of sequencing a fragment of cell-free (cf) DNA, according to an embodiment.

FIG. 7B is an illustration of the process of FIG. 7A of sequencing a fragment of cell-free (cf) DNA to obtain a methylation state vector, according to an embodiment.

FIG. 8 illustrates extent of bisulfite conversion (upper panel) and mean coverage/sequencing depth (lower panel) across varying stages of cancer.

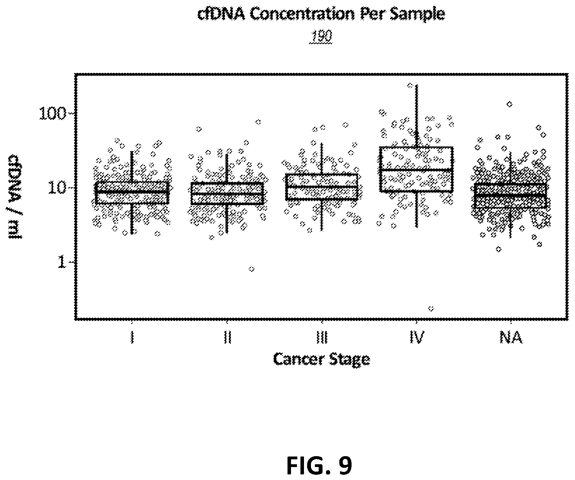

FIG. 9 illustrates concentration of cfDNA per sample across varying stages of cancer.

FIG. 10 is a graph of the amounts of DNA fragments binding to probes depending on the sizes of overlap between the DNA fragments and the probes.

FIG. 11A summarizes frequencies of genomic annotations of targeted genomic regions of List 1 (black) and randomly selected genomic regions (gray). FIG. 11B summarizes frequencies of genomic annotations of targeted genomic regions of List 2 (black) and randomly selected genomic regions (gray). FIG. 11C summarizes frequencies of genomic annotations of targeted genomic regions of List 3 (black) and randomly selected genomic regions (gray).

FIG. 12A illustrates a flowchart of devices for sequencing nucleic acid samples according to one embodiment. FIG. 12B illustrates an analytic system that analyzes methylation status of cfDNA according to one embodiment.

FIG. 13 is a shaded matrix presenting numbers of genomic regions selected for differentiating each target TOO (x-axis) from a contrast TOO (y-axis).

FIG. 14 provides data for verifying selected genomic regions using cfDNA and WBC gDNA. Fractions (y-axis) classifying each TOO (x-axis) correctly are provided.

FIG. 15A depicts a receiver operator curve (ROC) showing the sensitivity and specificity of cancer detection using methylation data for the target genomic regions of List 4. **FIG. 15B** is a confusion matrix depicting the accuracy of cancer type classifications for subjects determined to have cancer using methylation data for the target genomic regions of List 4.

**FIG. 16A** depicts a ROC showing the sensitivity and specificity of cancer detection using methylation data for the target genomic regions of List 5. **FIG. 16B** illustrates actual cancer type vs. predicted cancer type using a classifier generated with the genomic regions of List 5.

**FIG 17A** depicts a ROC showing the sensitivity and specificity of cancer detection using methylation data for the target genomic regions of List 6. **FIG. 17B** illustrates actual cancer type vs. predicted cancer type using a classifier generated with the genomic regions of List 6.

**FIG. 18A** depicts a ROC showing the sensitivity and specificity of cancer detection using methylation data for the target genomic regions of List 7. **FIG. 18B** is a confusion matrix depicting the accuracy of cancer type classifications for subjects determined to have cancer using methylation data for the target of List 7.

**FIG. 19A** depicts a ROC showing the sensitivity and specificity of cancer detection using methylation data for the target genomic regions of List 8. **FIG. 19B** is a confusion matrix depicting the accuracy of cancer type classifications for subjects determined to have cancer using methylation data for the target of List 8.

**FIG. 20A** depicts a ROC showing the sensitivity and specificity of cancer detection using methylation data for the target genomic regions of List 9. **FIG. 20B** is a confusion matrix depicting the accuracy of cancer type classifications for subjects determined to have cancer using methylation data for the target of List 9.

**FIG. 21A** depicts a ROC showing the sensitivity and specificity of cancer detection using methylation data for the target genomic regions of List 10. **FIG. 21B** is a confusion matrix depicting the accuracy of cancer type classifications for subjects determined to have cancer using methylation data for the target of List 10.

**FIG. 22A** depicts a ROC showing the sensitivity and specificity of cancer detection using methylation data for the target genomic regions of List 11. **FIG. 22B** is a confusion matrix depicting the accuracy of cancer type classifications for subjects determined to have cancer using methylation data for the target of List 11.

**FIG. 23A** depicts a ROC showing the sensitivity and specificity of cancer detection using methylation data for the target genomic regions of List 12. **FIG. 23B** is a confusion matrix depicting the accuracy of cancer type classifications for subjects determined to have cancer using methylation data for the target of List 12.

**FIG. 24A** depicts a ROC showing the sensitivity and specificity of cancer detection using methylation data for the target genomic regions of List 13. **FIG. 24B** is a confusion matrix depicting the accuracy of cancer type classifications for subjects determined to have cancer using methylation data for the target of List 13.

**FIG 25A** depicts a ROC showing the sensitivity and specificity of cancer detection using methylation data for the target genomic regions of List 14. **FIG. 25B** is a confusion matrix depicting the accuracy of cancer type classifications for subjects determined to have cancer using methylation data for the target of List 14.

**FIG. 26A** depicts a ROC showing the sensitivity and specificity of cancer detection using methylation data for the target genomic regions of List 15. **FIG. 26B** is a confusion matrix depicting the accuracy of cancer type classifications for subjects determined to have cancer using methylation data for the target of List 15.

**FIG. 27A** depicts a ROC showing the sensitivity and specificity of cancer detection using methylation data for the target genomic regions of List 16. **FIG. 27B** is a confusion matrix depicting the accuracy of cancer type classifications for subjects determined to have cancer using methylation data for the target of List 16.

**FIG. 28A** depicts a ROC showing the sensitivity and specificity of cancer detection using methylation data for a randomly selected subset of 10% of the target genomic regions of List 12. **FIG. 28B** is a confusion matrix depicting the accuracy of cancer type classifications for subjects determined to have cancer using methylation data for a randomly selected subset of 10% of the target genomic regions of List 12.

**FIG. 29A** depicts a ROC showing the sensitivity and specificity of cancer detection using methylation data for a randomly selected subset of 25% of the target genomic regions of List 12. **FIG. 29B** is a confusion matrix depicting the accuracy of cancer type classifications for subjects determined to have cancer using methylation data for a randomly selected subset of 25% of the target genomic regions of List 12.

**FIG. 30A** depicts a ROC showing the sensitivity and specificity of cancer detection using methylation data for a randomly selected subset of 50% of the target genomic regions of List 4. **FIG. 30B** is a confusion matrix depicting the accuracy of cancer type classifications for subjects determined to have cancer using methylation data for a randomly selected subset of 50% of the target genomic regions of List 4.

## DETAILED DESCRIPTION

### Definitions

Unless defined otherwise, all technical and scientific terms used herein have the meaning commonly understood by a person skilled in the art to which this description belongs. As used herein, the following terms have the meanings ascribed to them below.

As used herein any reference to "one embodiment" or "an embodiment" means that a particular element, feature, structure, or characteristic described in connection with the embodiment is included in at least one embodiment. The appearances of the phrase "in one embodiment" in various places in the specification are not necessarily all referring to the same embodiment, thereby providing a framework for various possibilities of described embodiments to function together.

As used herein, the terms "comprises," "comprising," "includes," "including," "has," "having" or any other variation thereof, are intended to cover a non-exclusive inclusion. For example, a process, method, article, or apparatus that comprises a list of elements is not necessarily limited to only those elements but may include other elements not expressly listed or inherent to such process, method, article, or apparatus. Further, unless expressly stated to the contrary, "or" refers to an inclusive or and not to an exclusive or. For example, a condition A or B is satisfied by any one of the following: A is true (or present) and B is false (or not present), A is false (or not present) and B is true (or present), and both A and B are true (or present).

In addition, use of the "a" or "an" are employed to describe elements and components of the embodiments herein. This is done merely for convenience and to give a general sense of the description. This description should be read to include one or at least one and the singular also includes the plural unless it is obvious that it is meant otherwise.

As used herein, ranges and amounts can be expressed as "about" a particular value or range. About also includes the exact amount. Hence "about 5 $\mu$g" means "about 5 $\mu$g" and also "5 $\mu$g." Generally, the term "about" includes an amount that would be expected to be within experimental error. In some embodiments, "about" refers to the number or value recited, "+" or "-" 20%, 10%, or 5% of the number or value. Additionally, ranges recited herein are understood to be shorthand for all of the values within the range, inclusive of the recited endpoints. For example, a range of 1 to 50 is understood to include any number, combination of numbers, or sub-range from the group consisting of 1, 2, 3, 4, 5, 6, 7, 8, 9, 10, 11, 12, 13, 14, 15, 16, 17, 18, 19, 20, 21, 22, 23, 24, 25, 26, 27, 28, 29, 30, 31, 32, 33, 34, 35, 36, 37, 38, 39, 40, 41, 42, 43, 44, 45, 46, 47, 48, 49, and 50.

The term "methylation" as used herein refers to a process by which a methyl group is added to a DNA molecule. For example, a hydrogen atom on the pyrimidine ring of a cytosine base can be converted to a methyl group, forming 5-methylcytosine. The term also refers to a process by which a hydroxymethyl group is added to a DNA molecule, for example by oxidation of a methyl group on the pyrimidine ring of a cytosine base. Methylation and hydroxymethylation tend to occur at dinucleotides of cytosine and guanine referred to herein as "CpG sites."

The term "methylation" can also refer to the methylation status of a CpG site. A CpG site with a 5-methylcytosine moiety is methylated. A CpG site with a hydrogen atom on the pyrimidine ring of the cytosine base is unmethylated.

Should also cover methylation status at a site, i.e., presence or absence of a methyl group. Where the presence of a methyl group is a methylated site / absence of a methyl group is an unmethylated site or non-methylated site.

In such embodiments, the wet laboratory assay used to detect methylation may vary from those described herein as is well known in the art.

The term "methylation site" as used herein refers to a region of a DNA molecule where a methyl group can be added. "CpG" sites are the most common methylation site, but methylation sites are not limited to CpG sites. For example, DNA methylation may occur in cytosines in CHG and CHH, where H is adenine, cytosine or thymine. Cytosine methylation in the form of 5-hydroxymethylcytosine may also assessed (see, e.g., WO 2010/037001 and WO 2011/127136, which are incorporated herein by reference), and features thereof, using the methods and procedures disclosed herein.

The term "CpG site" as used herein refers to a region of a DNA molecule where a cytosine nucleotide is followed by a guanine nucleotide in the linear sequence of bases along its 5' to 3' direction. "CpG" is a shorthand for 5'-C-phosphate-G-3' that is cytosine and guanine separated by only one phosphate group. Cytosines in CpG dinucleotides can be methylated to form 5-methylcytosine.

The term "CpG detection site" as used herein refers to a region in a probe that is configured to hybridize to a CpG site of a target DNA molecule. The CpG site on the target DNA molecule can comprise cytosine and guanine separated by one phosphate group, where cytosine is methylated or unmethylated. The CpG site on the target DNA molecule can comprise uracil and guanine separated by one phosphate group, where the uracil is generated by the conversion of unmethylated cytosine.

The term "UpG" is a shorthand for 5'-U-phosphate-G-3' that is uracil and guanine separated by only one phosphate group. UpG can be generated by a bisulfite treatment of a DNA that converts unmethylated cytosines to uracils. Cytosines can be converted to uracils by other methods known in the art, such as chemical modification, synthesis, or enzymatic conversion.

The term "hypomethylated" or "hypermethylated" as used herein refers to a methylation status of a DNA molecule containing multiple CpG sites (e.g., more than 3, 4, 5, 6, 7, 8, 9, 10, etc.) where a high percentage of the CpG sites (e.g., more than 80%, 85%, 90%, or 95%, or any other percentage within the range of 50%-100%) are unmethylated or methylated, respectively.

The terms "methylation state vector" or "methylation status vector" as used herein refers to a vector comprising multiple elements, where each element indicates the methylation status of a methylation site in a DNA molecule comprising multiple methylation sites, in the order they appear from 5' to 3' in the DNA molecule. For example, $< M_x, M_{x+1}, M_{x+2} >$, $< M_x, M_{x+1}, U_{x+2} >$, ..., $< U_x, U_{x+1}, U_{x+2} >$ can be methylation vectors for DNA molecules comprising three methylation sites, where M represents a methylated methylation site and U represents an unmethylated methylation site.

The term "abnormal methylation pattern" or "anomalous methylation pattern" as used herein refers to the methylation pattern of a DNA molecule or a methylation state vector that is expected to be found in a sample less frequently than a threshold value in a non-cancer or healthy sample. In a particular embodiment provided herein, the expectedness of finding a specific methylation state vector in a healthy control group comprising healthy individuals is represented by a p-value. A low p-value score generally corresponds to a methylation state vector which is relatively unexpected in comparison to other methylation state vectors within samples from healthy individuals. A high p-value score generally corresponds to a methylation state vector which is relatively more expected in comparison to other methylation state vectors found in samples from healthy individuals in the healthy control group. A methylation state vector having a p-value lower than a threshold value (e.g., 0.1, 0.01, 0.001, 0.0001, etc.) can be defined as an abnormal/anomalous methylation pattern. Various methods known in the art can be used to calculate a p-value or expectedness of a methylation pattern or a methylation state vector. Exemplary methods provided herein involve use of a Markov chain probability that assumes methylation statuses of CpG sites to be dependent on methylation statuses of neighboring CpG sites. Alternate methods provided herein calculate the expectedness of observing a specific methylation state vector in healthy individuals by utilizing a mixture model including multiple mixture components, each being an independent-sites model where methylation at each CpG site is assumed to be independent of methylation statuses at other CpG sites.

The term "cancerous sample" as used herein refers to a sample comprising genomic DNAs from an individual diagnosed with cancer. The genomic DNAs can be, but are not limited to, cfDNA fragments or chromosomal DNAs from a subject with cancer. The genomic DNAs can be sequenced and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual diagnosed with cancer, cancerous sample can refer to genomic DNAs or cfDNA fragments having the genomic sequences. The term "cancerous samples" as a plural refers to samples comprising genomic DNAs from multiple individuals, each individual diagnosed with cancer. In various embodiments, cancerous samples from more than 100, 300, 500, 1,000, 2,000, 5,000, 10,000, 20,000, 40,000, 50,000, or more individuals diagnosed with cancer are used.

The term "non-cancerous sample" as used herein refers to a sample comprising genomic DNAs from an individual not diagnosed with cancer. The genomic DNAs can be, but are not limited to, cfDNA fragments or chromosomal DNAs from a subject without cancer. The genomic DNAs can be sequenced and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual without cancer, non-cancerous sample can refer to genomic DNAs or cfDNA fragments having the genomic sequences. The term "non-cancerous samples" as a plural refers to samples comprising genomic DNAs from multiple individuals, each individual is without cancer. In various embodiments, cancerous samples from more than 100, 300, 500, 1,000, 2,000, 5,000, 10,000, 20,000, 40,000, 50,000, or more individuals without cancer are used.

The term "training sample" as used herein refers to a sample used to train a classifier described herein and/or to select one or more genomic regions for cancer detection or detecting a cancer tissue of origin or cancer cell type. The training samples can comprise genomic DNAs or a modification there of, from one or more healthy subjects and from one or more subjects having a disease condition (e.g., cancer, a specific type of cancer, a specific stage of cancer, etc.). The genomic DNAs can be, but are not limited to, cfDNA fragments or chromosomal DNAs. The genomic DNAs can be sequenced and their methylation status can be assessed by methods known in the art, for example, bisulfite sequencing. When genomic sequences are obtained from public database (e.g., The Cancer Genome Atlas (TCGA)) or experimentally obtained by sequencing a genome of an individual, a training sample can refer to genomic DNAs or cfDNA fragments having the genomic sequences.

The term "test sample" as used herein refers to a sample from a subject, whose health condition was, has been or will be tested using a classifier and/or an assay panel described herein. The test sample can comprise genomic DNAs or a modification there of. The genomic DNAs can be, but are not limited to, cfDNA fragments or chromosomal DNAs.

The term "target genomic region" as used herein refers to a region in a genome selected for analysis in test samples. An assay panel is generated with probes designed to hybridize to (and optionally pull down) nucleic acid fragments derived from the target genomic region or a fragment thereof. A nucleic acid fragment derived from the target genomic region refers to a nucleic acid fragment generated by degradation, cleavage, bisulfite conversion, or other processing of the DNA from the target genomic region.

Various target genomic regions are described according to their chromosomal location in the sequence listing filed herewith. Chromosomal DNA is double-stranded, so a target genomic region includes two DNA strands: one with the sequence provided in the listing and a second that is a reverse complement to the sequence in the listing. Probes can be designed to hybridize to one or both sequences. Optionally, probes hybridize to converted sequences resulting from, for example, treatment with sodium bisulfite.

The term "off-target genomic region" as used herein refers to a region in a genome which has not been selected for analysis in test samples but has sufficient homology to a target genomic region to potentially be bound and pulled down by a probe designed to target the target genomic region. In one embodiment, an off-target genomic region is a genomic region that aligns to a probe along at least 45 bp with at least a 90% match rate.

The terms "converted DNA molecules," "converted cfDNA molecules," and "modified fragment obtained from processing of the cfDNA molecules" refer to DNA molecules obtained by processing DNA or cfDNA molecules in a sample for the purpose of differentiating a methylated nucleotide and an unmethylated nucleotide in the DNA or cfDNA molecules. For example, in one embodiment, the sample can be treated with bisulfite ion (e.g., using sodium bisulfite), as is well-known in the art, to convert unmethylated cytosines ("C") to uracils ("U"). In another embodiment, the conversion of unmethylated cytosines to uracils is accomplished using an enzymatic conversion reaction, for example, using a cytidine deaminase (such as APOBEC). After treatment, converted DNA molecules or cfDNA molecules include additional uracils which are not present in the original cfDNA sample. Replication by DNA polymerase of a DNA strand comprising a uracil results in addition of an adenine to the nascent complementary strand instead of the guanine normally added as the complement to a cytosine or methylcytosine.

The terms "cell free nucleic acid," "cell free DNA," or "cfDNA" refers to nucleic acid fragments that circulate in an individual's body (e.g., bloodstream) and originate from one or more healthy cells and/or from one or more cancerous cells. Additionally, cfDNA may come from other sources such as viruses, fetuses, etc.

The term "circulating tumor DNA" or "ctDNA" refers to nucleic acid fragments that originate from tumor cells, which may be released into an individual's bloodstream as result of biological processes such as apoptosis or necrosis of dying cells or actively released by viable tumor cells.

The term "fragment" as used herein can refer to a fragment of a nucleic acid molecule. For example, in one embodiment, a fragment can refer to a cfDNA molecule in a blood or plasma sample, or a cfDNA molecule that has been extracted from a blood or plasma sample. An amplification product of a cfDNA molecule may also be referred to as a "fragment." In another embodiment, the term "fragment" refers to a sequence read, or set of sequence reads, that have been processed for subsequent analysis (e.g., for in machine-learning based classification), as described herein. For example, as is well known in the art, raw sequence reads can be aligned to a reference genome and matching paired end sequence reads assembled into a longer fragment for subsequent analysis.

The term "individual" refers to a human individual. The term "healthy individual" refers to an individual presumed not to have a cancer or disease.

The term "subject" refers to an individual whose DNA is being analyzed. A subject may be a test subject whose DNA is be evaluated using a targeted panel as described herein to evaluate whether the person has cancer or another disease. A subject may also be part of a control group known not to have cancer or another disease. A subject may also be part of a cancer or other disease group known to have cancer or another disease. Control and cancer/disease groups may be used to assist in designing or validating the targeted panel.

The term "sequence reads" as used herein refers to nucleotide sequences reads from a sample. Sequence reads can be obtained through various methods provided herein or as known in the art.

The term "sequencing depth" as used herein refers to the count of the number of times a given target nucleic acid within a sample has been sequenced (e.g., the count of sequence reads at a given target region). Increasing sequencing depth can reduce required amounts of nucleic acids required to assess a disease state (e.g., cancer or cancer tissue of origin).

The term "tissue of origin" or "TOO" as used herein refers to the organ, organ group, body region or cell type that a cancer arises or originates from. The identification of a tissue of origin or cancer cell type typically allows for identification of the most appropriate next steps in the care continuum of cancer to further diagnose, stage and decide on treatment.

The term "transition" generally refers to changes in base composition from one purine to another purine, or from one pyrimidine to another pyrimidine. For instance, the following changes are transitions: C→U, U→C, G→A, A→G, C→T, and T→C.

"An entirety of probes" of a panel or bait set or "an entirety of polynucleotide-containing probes" of a panel or bait set generally refers to all of the probes delivered with a specified panel or bait set. For instance, in some embodiments, a panel or bait set may include both (1) probes having features specified herein (e.g., probes for binding to cell-free DNA fragments corresponding to or derived from genomic regions set forth herein in one or more Lists) and (2) additional probes that do not contain such feature(s). The entirety of probes of a panel generally refers to all probes delivered with the panel or bait set, including such probes that do not contain the specified feature(s).

**Cancer assay panel**

In a first aspect, the present description provides a cancer assay panel comprising a plurality of probes or a plurality of probe pairs. The assay panels described herein can alternatively be referred to as bait sets or as compositions comprising bait oligonucleotides. The probes can be polynucleotide-containing probes that are specifically designed to target one or more genomic regions differentially methylated between cancer and non-cancer samples, between different cancer tissue of origin (TOO) types, between different cancer cell types, between samples of different stages of cancer, as identified by methods provided herein. In some embodiments, the target genomic regions are selected to maximize classification accuracy, subject to a size budget (which is determined by sequencing budget and desired depth of sequencing).

For designing the cancer assay panel, an analytics system may collect samples corresponding to various outcomes under consideration, e.g., samples known to have cancer, samples considered to be healthy, samples from a known tissue of origin, etc. The sources of the cfDNA and/or ctDNA used to select target genomic regions can vary depending on the purpose of the assay. For example, different sources may be desirable for an assay intended to detect cancer generally, a specific type of cancer, a cancer stage, or a tissue of origin. These samples may be processed by whole-genome bisulfite sequencing (WGBS) or obtained from a public database (e.g., TCGA). The analytics system may be any generic computing system with a computer processor and a computer-readable storage medium with instructions for executing the computer processor to perform any or all operations described in this present disclosure.

The analytics system may then select target genomic regions based on methylation patterns of nucleic acid fragments. One approach considers pairwise distinguishability between pairs of outcomes for regions (or more specifically for CpG sites within regions). Another approach considers distinguishability for regions (or more specifically for CpG sites within regions) when considering each outcome against the remaining outcomes. From the selected target genomic regions with high distinguishability power, the analytics system may design probes to target fragments from the selected genomic regions. The analytics system may generate variable sizes of the cancer assay panel, e.g., where a small sized cancer assay panel includes probes targeting the most informative genomic regions, a medium sized cancer assay panel includes probes from the small sized cancer assay panel and additional probes targeting a second tier of informative genomic regions, and a large sized cancer assay panel includes probes from the small-sized and the medium-sized cancer assay panels along with even more probes targeting a third tier of informative genomic regions. With data obtained such cancer assay panels (e.g., the methylation status on nucleic acids derived from the cancer assay panels), the analytics system may train classifiers with various classification techniques to predict a sample's likelihood of having a particular outcome or state, e.g., cancer, specific cancer type, other disorder, other disease, etc.

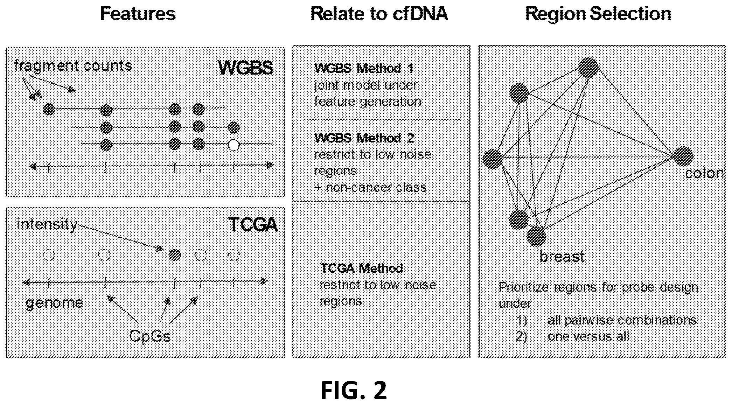

Exemplary methodology for designing a cancer assay panel is generally described in FIG. 2. For instance, to design a cancer assay panel, an analytics system may collect information on the methylation status of CpG sites of nucleic acid fragments from samples corresponding to various outcomes under consideration, e.g., samples known to have cancer, samples considered to be healthy, samples from a known TOO, etc. These samples may be processed (e.g., with whole-genome bisulfite sequencing (WGBS)) to determine the methylation status of CpG sites, or the information may be obtained from TCGA. The analytics system may be any generic computing system with a computer processor and a computer-readable storage medium with instructions for executing the computer processor to perform any or all operations described in this present disclosure.

The analytics system may then select target genomic regions based on methylation patterns of nucleic acid fragments. One approach considers pairwise distinguishability between pairs of outcomes for regions (or more specifically CpG sites). Another approach considers distinguishability for regions (or more specifically CpG sites) when considering each outcome against the remaining outcomes. From the selected target genomic regions with high distinguishability power, the analytics system may design probes to target fragments from the selected genomic regions. The analytics system may generate variable sizes of the cancer assay panel, e.g., where a small sized cancer assay panel includes probes targeting the most informative genomic regions, a medium sized cancer assay panel includes probes from the small sized cancer assay panel and additional probes targeting a second tier of informative genomic regions, and a large sized cancer assay panel includes probes from the small-sized and the medium-sized cancer assay panels along with even more probes targeting a third tier of informative genomic regions. With such cancer assay panels, the analytics system may train classifiers with various classification techniques to predict a sample's likelihood of having a particular outcome or state, e.g., cancer, specific cancer type, other disorder, other disease, etc.

In some embodiments, the cancer assay panel comprises at least 500 pairs of probes, wherein each pair of the at least 500 pairs comprises two probes configured to overlap each other by an overlapping sequence, wherein the overlapping sequence comprises at least 30-nucleotides, and wherein each probe is configured to hybridize to the same strand of an (optionally converted) DNA molecule (e.g., a cfDNA molecule) corresponding to one or more genomic regions. In some embodiments, each of the genomic regions comprises at least five methylation sites, and wherein the at least five methylation sites have an abnormal methylation pattern in cancerous samples or a different methylation pattern between samples of a different TOO. For example, in one embodiment, the at least five methylation sites are differentially methylated either between cancerous and non-cancerous samples or between one or more pairs of samples from cancers with different tissue of origin. In some embodiments, each pair of probes comprises a first probe and a second probe, wherein the second probe differs from the first probe. The second probe can overlap with the first probe by an overlapping sequence that is at least 30, at least 40, at least 50, or at least 60 nucleotides in length.

The target genomic regions can be selected from any one of Lists 1-16 (**TABLE 1)** In some embodiments, the cancer assay panel comprises a plurality of probes, wherein each of the plurality of probes is configured to hybridize to a converted cfDNA molecule corresponding to one or more of the genomic regions in any one of Lists 1-16. In some embodiments, the plurality of different bait oligonucleotides are configured to hybridize to DNA molecules derived from at least 20% of the target genomic regions of any one of Lists 1-16. In some embodiments, the plurality of different bait oligonucleotides are configured to hybridize to DNA molecules derived from at least 30%, 40%, 50%, 60%, 70%, or 80% of the target genomic regions of any one of Lists 1-16.

The target genomic regions can be selected from List 1. The target genomic regions can be selected from List 2. The target genomic regions can be selected from List 3. The target genomic regions can be selected from List 4. The target genomic regions can be selected from List 5. The target genomic regions can be selected from List 6. The target genomic regions can be selected from List 7. The target genomic regions can be selected from List 8. The target genomic regions can be selected from List 9. The target genomic regions can be selected from List 10. The target genomic regions can be selected from List 11. The target genomic regions can be selected from List 12. The target genomic regions can be selected from List 13. The target genomic regions can be selected from List 14. The target genomic regions can be selected from List 15. The target genomic regions can be selected from List 16.

Since the probes are configured to hybridize to a converted DNA or cfDNA molecule corresponding to, or derived from, one or more genomic regions, the probes can have a sequence different from the targeted genomic region. For example, a DNA containing an unmethylated CpG site will be converted to include UpG instead of CpG because unmethylated cytosines are converted to uracils by a conversion reaction (e.g., bisulfite treatment). As a result, a probe is configured to hybridize to a sequence including UpG instead of a naturally-existing unmethylated CpG. Accordingly, a complementary site in the probe to the unmethylated site can comprise CpA instead of CpG, and some probes targeting a hypomethylated site where all methylation sites are unmethylated can have no guanine (G) bases. In some embodiments, at least 3%, 5%, 10%, 15%, or 20% of the probes comprise no CpG sequences.

The cancer assay panel can be used to detect the presence or absence of cancer generally and/or provide a cancer classification such as cancer type, stage of cancer such as I, II, III, or IV, or provide the TOO where the cancer is believed to originate. The panel may include probes targeting genomic regions differentially methylated between general cancerous (pan-cancer) samples and non-cancerous samples, or only in cancerous samples with a specific cancer type (e.g., lung cancer-specific targets). For example, in some embodiments, a cancer assay panel is designed to include differentially methylated genomic regions based on converted (e.g., bisulfite) sequencing data generated from the cfDNA from cancer and non-cancer individuals.

Each of the probes (or probe pairs) may be designed to target one or more target genomic regions. The target genomic regions can be selected based on several criteria designed to increase selective enriching of informative cfDNA fragments while decreasing noise and non-specific bindings.

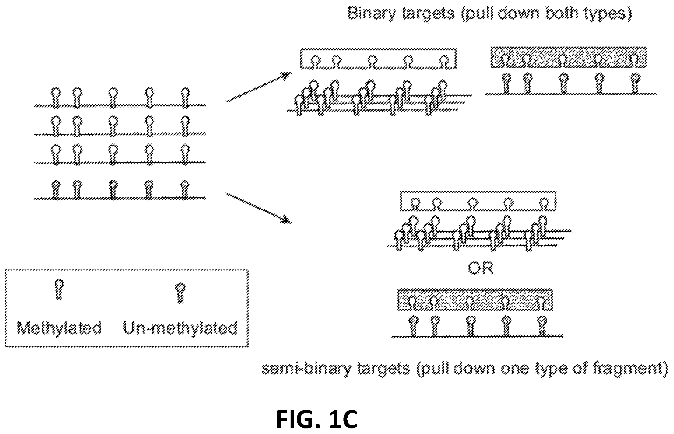

In one example, a panel can include probes that can selectively bind to and enrich cfDNA fragments that are differentially methylated in cancerous samples. In this case, sequencing of the enriched fragments can provide information relevant to detection of cancer. Furthermore, in some embodiments, the probes (or a portion thereof) are designed to target genomic regions that are determined to have an abnormal methylation pattern in cancer samples, or in samples from certain cancer types, tissue types or cell types. In one embodiment, probes are designed to target genomic regions determined to be hypermethylated or hypomethylated in certain cancers or cancer types to provide additional selectivity and specificity of the detection. In some embodiments, a panel comprises probes targeting hypomethylated fragments. In some embodiments, a panel comprises probes targeting hypermethylated fragments. In some embodiments, a panel comprises both a first set of probes targeting hypermethylated fragments and a second set of probes targeting hypomethylated fragments. In some embodiments, a cancer assay panel includes not only probes that are designed to target a region that has a first methylation status (e.g., hypomethylation), but also includes probes that are designed to hybridize to the same target region with the opposite methylation status (e.g., hypermethylation). The targeting of probes to both hypo- and hyper-methylated fragments from the same regions can be referred to as "binary" targeting (see information in the Sequence Listing) (FIG. 1C). In some embodiments, the ratio between the first set of probes targeting hypermethylated fragments and the second set of probes targeting hypomethylated fragments (Hyper:Hypo ratio) ranges between 0.4 and 2, between 0.5 and 1.8, between 0.5 and 1.6, between 0.5 and 1.0, between 1.4 and 1.6, between 1.2 and 1.4, between 1 and 1.2, between 0.8 and 1, between 0.6 and 0.8 or between 0.4 and 0.6. Methods of identifying genomic regions (i.e., genomic regions giving rise to differentially methylated DNA molecules (or anomalously methylated DNA molecules) between cancer and non-cancer samples, between different cancer tissue of origin (TOO) types, between different cancer cell type, or between samples from different stages of cancer are provided in detail herein and methods of identifying anomalously methylated DNA molecules or fragments that are identified as indicative of cancer are also provided in detail herein.

In a second example, genomic regions can be selected when the genomic regions give rise to anomalously methylated DNA molecules in cancer samples or samples with known cancer tissue of origin (TOO) types. For example, as described herein, a Markov model trained on a set of non-cancerous samples can be used to identify genomic regions that give rise to anomalously methylated DNA molecules (i.e., DNA molecules having a methylation pattern below a p-value threshold).

Each of the probes can target a genomic region comprising at least 30 bp, 35 bp, 40 bp, 45 bp, 50 bp, 60 bp, 70 bp, 80 bp, 90 bp, 100 bp or more. In some embodiments, the genomic regions can be selected to have less than 30, 25, 20, 15, 12, 10, 8, or 6 methylation sites.

In some instances, the genomic regions can be selected when at least 80, 85, 90, 92, 95, or 98% of the at least five methylation (e.g., CpG) sites within the region are either methylated or unmethylated in non-cancerous or cancerous samples, or in cancer samples from a tissue of origin (TOO).

Genomic regions may be further filtered to select only those that are likely to be informative based on their methylation patterns, for example, CpG sites that are differentially methylated between cancerous and non-cancerous samples (e.g., abnormally methylated or unmethylated in cancer versus non-cancer), between cancerous samples of a TOO and cancerous samples of a different TOO, CpG sites that are differentially methylated only in cancerous samples of a TOO. For the selection, calculation can be performed with respect to each CpG or a plurality of CpG sites. For example, a first count can be determined that is the number of cancer-containing samples (cancer_count) that include a fragment overlapping that CpG, and a second count is determined that is the number of total samples containing fragments overlapping that CpG site (total). Genomic regions can be selected based on criteria positively correlated to the number of cancer-containing samples (cancer_count) that include a fragment indicative of cancer overlapping that CpG site, and inversely correlated with the number of total samples containing fragments indicative of cancer overlapping that CpG site (total). In one embodiment, the number of non-cancerous samples (nnon-cancer) and the number of cancerous samples (ncancer) having a fragment overlapping a CpG site are counted. Then the probability that a sample is cancer is estimated, for example as (ncancer + 1) / (ncancer + nnon-cancer + 2). This principle could be similarly applied to other outcomes.

CpG sites scored by this metric are ranked and greedily added to a panel until the panel size budget is exhausted. The process of selecting genomic regions indicative of cancer is further detailed herein. In some embodiments, different target regions may be selected depending on whether the assay is intended to be a pan-cancer assay or a single-cancer assay, or depending on what kind of flexibility is desired when picking which CpG sites are contributing to the panel. A panel for detecting a specific cancer type can be designed using a similar process. In this embodiment, for each cancer type, and for each CpG site, the information gain is computed to determine whether to include a probe targeting that CpG site. The information gain may be computed for samples with a given cancer type of a TOO compared to all other samples. For example, consider two random variables, "AF" and "CT". "AF" is a binary variable that indicates whether there is an abnormal fragment overlapping a particular CpG site in a particular sample (yes or no). "CT" is a binary random variable indicating whether the cancer is of a particular type (e.g., lung cancer or cancer other than lung). One can compute the mutual information with respect to "CT" given "AF." That is, how many bits of information about the cancer type (lung vs. non-lung in the example) are gained if one knows whether there is an anomalous fragment overlapping a particular CpG site. This can be used to rank CpG's based on how lung-specific they are. This procedure is repeated for a plurality of cancer types. If a particular region is commonly differentially methylated only in lung cancer (and not other cancer types or non-cancer), CpG's in that region would tend to have high information gains for lung cancer. For each cancer type, CpG sites are ranked by this information gain metric, and then greedily added to a panel until the size budget for that cancer type is exhausted.

Further filtration can be performed to select probes with high specificity for enrichment (i.e., high binding efficiency) of nucleic acids derived from targeted genomic regions. Probes can be filtered to reduce non-specific binding (or off-target binding) to nucleic acids derived from non-targeted genomic regions. For example, probes can be filtered to select only those probes having less than a set threshold of off-target binding events. In one embodiment, probes can be aligned to a reference genome (e.g., a human reference genome) to select probes that align to less than a set threshold of regions across the genome. For example, probes can be selected that align to less than 25, 24, 23, 22, 21, 20, 19, 18, 17, 16, 15, 14, 13, 12, 11, 10, 9 or 8 off-target regions across the reference genome. In other cases, filtration is performed to remove genomic regions when the sequence of the target genomic regions appears more than 5, 10, 15, 20, 21, 22, 23, 24, 25, 26, 27, 28, 29, 30, 31, 32, 33, 34 or 35 times in a genome. Further filtration can be performed to select target genomic regions when a probe sequence, or a set of probe sequences that are 90%, 91%, 92%, 93%, 94%, 95%, 96%, 97%, 98% or 99% homologous to the target genomic regions, appear less than 25, 24, 23, 22, 21, 20, 19, 18, 17, 16, 15, 14, 13, 12, 11, 10, 9 or 8 times in a reference genome, or to remove target genomic regions when the probe sequence, or a set of probe sequences designed to enrich for the targeted genomic region are 90%, 91%, 92%, 93%, 94%, 95%, 96%, 97%, 98% or 99% homologous to the target genomic regions, appear more than 5, 10, 15, 20, 21, 22, 23, 24, 25, 26, 27, 28, 29, 30, 31, 32, 33, 34 or 35 times in a reference genome. This is for excluding repetitive probes that can pull down off-target fragments, which are not desired and can impact assay efficiency.

In some embodiments, a fragment-probe overlap of at least 45 bp was demonstrated to be effective for achieving a non-negligible amount of pulldown (though as one of skill in the art would appreciate this number can very) as provided in Example 1. In some embodiments, more than a 10% mismatch rate between the probe and fragment sequences in the region of overlap is sufficient to greatly disrupt binding, and thus pulldown efficiency. Therefore, sequences that can align to the probe along at least 45 bp with at least a 90% match rate can be candidates for off-target pulldown. Thus, in one embodiment, the number of such regions are scored. The best probes have a score of 1, meaning they match in only one place (the intended target region). Probes with an intermediate score (say, less than 5 or 10) may in some instances be accepted, and in some instances any probes above a particular score are discarded. Other cutoff values can be used for specific samples.

Once the probes hybridize and capture DNA fragments corresponding to, or derived from, a target genomic region, the hybridized probe-DNA fragment intermediates are pulled down (or isolated), and the targeted DNA is amplified and sequenced. The sequence read provides information relevant for detection of cancer. For this end, a panel can be designed to include a plurality of probes that can capture fragments that can together provide information relevant to detection of cancer. In some embodiments, a panel includes at least 500, 1,000, 2,000, 2,500, 5,000, 6,000, 7,500, 10,000, 15,000, 20,000, 25,000, 30,000, 35,000, 40,000, 50,000, 60,000, 70,000, or 80,000 pairs of probes. In other embodiments, a panel includes at least 1,000, 2,000, 5,000, 10,000, 12,000, 15,000, 20,000, 30,000, 40,000, 50,000, 100,000, 200,000, 250,000, 300,000, 400,000, 500,000, 550,000, 600,000, 700,000, or 800,000 probes. The plurality of probes together can comprise at least 0.2 million, 0.4 million, 0.6 million, 0.8 million, 1 million, 2 million, 3 million, 4 million, 5 million, 6 million, 7 million, 8 million, 9 million, 10 million, 12 million, 14 million, 15 million, 20 million, or 25 million nucleotides.

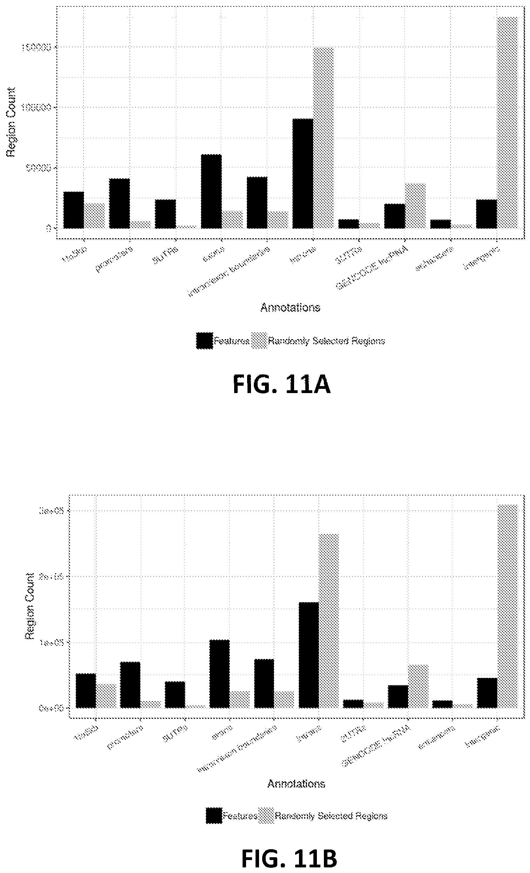

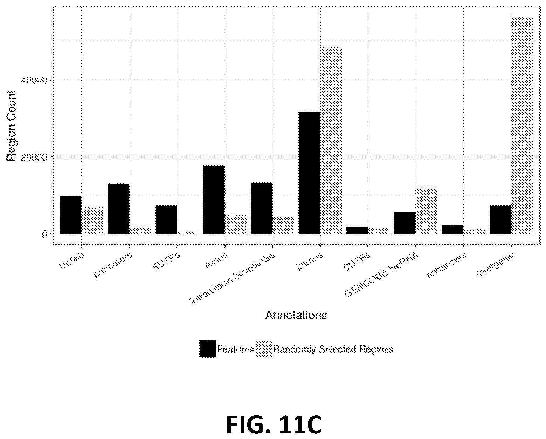

The selected target genomic regions can be located in various positions in a genome, including but not limited to exons, introns, intergenic regions, and other parts. **FIG. 11.** In some embodiments, probes targeting non-human genomic regions, such as those targeting viral genomic regions, can be added.

In some instances, primers may be used to specifically amplify targets/biomarkers of interest (e.g., by PCR), thereby enriching the sample for desired targets/biomarkers (optionally without hybridization capture). For example, forward and reverse primers can be prepared for each genomic region of interest and used to amplify fragments that correspond to or are derived from the desired genomic region. Thus, while the present disclosure pays particular attention to cancer assay panels and bait sets for hybridization capture, the disclosure is broad enough to encompass other methods for enrichment of cell-free DNA. Accordingly, a skilled artisan, with the benefit of this disclosure, will recognize that methods analogous to those described herein in connection with hybridization capture can alternatively be accomplished by replacing hybridization capture with some other enrichment strategy, such as PCR amplification of cell-free DNA fragments that correspond with genomic regions of interest. In some embodiments, bisulfite padlock probe capture is used to enrich regions of interest, such as is described in Zhang et al. (US 2016/0340740). In some embodiments, additional or alternative methods are used for enrichment (e.g., non-targeted enrichment) such as reduced representation bisulfite sequencing, methylation restriction enzyme sequencing, methylation DNA immunoprecipitation sequencing, methyl-CpG-binding domain protein sequencing, methyl DNA capture sequencing, or microdroplet PCR.

*Probes*

The cancer assay panels (alternatively referred to as "bait sets") provided herein can be a panel that includes a set of hybridization probes (also referred to herein as "probes") designed to, during enrichment, target and pull down nucleic acid fragments of interest for the assay. In some embodiments, the probes are designed to hybridize and enrich DNA or cfDNA molecules from cancerous samples that have been treated to convert unmethylated cytosines (C) to uracils (U). In other embodiments, the probes are designed to hybridize and enrich DNA or cfDNA molecules from cancerous samples of a TOO (or a plurality of TOOs) that have been treated to convert unmethylated cytosines (C) to uracils (U). The probes can be designed to anneal (or hybridize) to a target (complementary) strand of DNA or RNA. The target strand can be the "positive" strand (e.g., the strand transcribed into mRNA, and subsequently translated into a protein) or the complementary "negative" strand. In a particular embodiment, a cancer assay panel may include sets of two probes, one probe targeting the positive strand and the other probe targeting the negative strand of a target genomic region.

For each target genomic region, at least four possible probe sequences can be designed. Each target region is double-stranded, and as such, a probe or probe set can target either the "positive" or forward strand or its reverse complement (the "negative" strand). Additionally, in some embodiments, the probes or probe sets are designed to enrich DNA molecules or fragments that have been treated to convert unmethylated cytosines (C) to uracils (U). Because the probes or probe sets are designed to enrich DNA molecules corresponding to, or derived from the targeted regions after conversion, the probe's sequence can be designed to enrich DNA molecules of fragments where unmethylated C's have been converted to U's (by utilizing A's in place of G's at sites that are unmethylated cytosines in DNA molecules or fragments corresponding to, or derived from, the targeted region). In one embodiment, probes are designed to bind to, or hybridize to, DNA molecules or fragments from genomic regions known to contain cancer-specific methylation patterns (e.g., hypermethylated or hypomethylated DNA molecules), thereby enriching for cancer-specific DNA molecules or fragments. Targeting genomic regions, or cancer-specific methylation patterns, can be advantageous allowing one to specifically enrich for DNA molecules or fragments identified as informative for cancer or cancer TOO, and thus, lowering sequencing needs and sequencing costs. In other embodiments, two probe sequences can be designed per a target genomic region (one for each DNA strand). In still other cases, probes are designed to enrich for all DNA molecules or fragments corresponding to, or derived from, a targeted region (i.e., regardless of strand or methylation status). This might be because the cancer methylation status is not highly methylated or unmethylated, or because the probes are designed to target small mutations or other variations rather than methylation changes, with these other variations similarly indicative of the presence or absence of a cancer or the presence or absence of a cancer of one or more TOOs. In that case, all four possible probe sequences can be included per a target genomic region.

The probes can range in length from 10s, 100s, 200s, or 300s of base pairs. The probes can comprise at least 50, 75, 100, or 120 nucleotides. The probes can comprise less than 300, 250, 200, or 150 nucleotides. In an embodiment, the probes comprise 100-150 nucleotides. In one particular embodiment, the probes comprise 120 nucleotides.

In some embodiments, the probes are designed in a "2× tiled" fashion to cover overlapping portions of a target region. Each probe optionally overlaps in coverage at least partially with another probe in the library. In such embodiments, the panel contains multiple pairs of probes, with each probe in a pair overlapping the other by at least 25, 30, 35, 40, 45, 50, 60, 70, 75 or 100 nucleotides. In some embodiments, the overlapping sequence can be designed to be complementary to a target genomic region (or cfDNA derived therefrom) or to be complementary to a sequence with homology to a target region or cfDNA. Thus, in some embodiments, at least two probes are complementary to the same sequence within a target genomic region, and a nucleotide fragment corresponding to or derived from the target genomic region can be bound and pulled down by at least one of the probes. Other levels of tiling are possible, such as 3× tiling, 4× tiling, etc., wherein each nucleotide in a target region can bind to more than two probes.

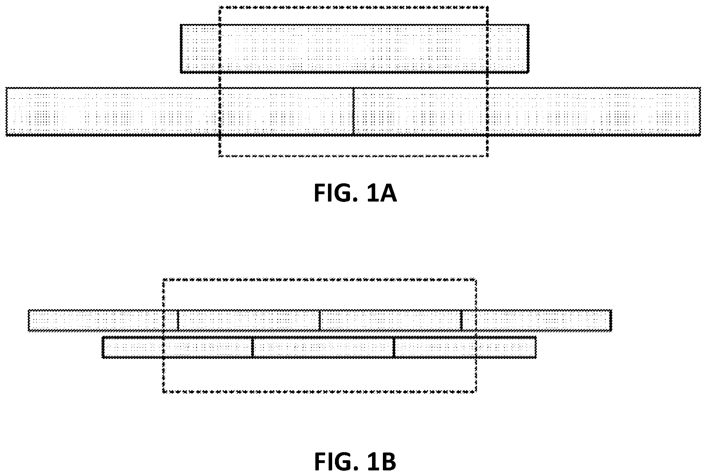

In one embodiment, each base in a target genomic region is overlapped by exactly two probes, as illustrated in **FIG. 1B.** Probes that extend in both directions beyond a target genomic region are useful to pull down cfDNA fragments comprising a portion of the target genomic region and DNA sequences adjacent to the target genomic region. In some instances, even relatively small target regions may be targeted with three probes (see **FIG. 1A**). A probe set comprising three or more probes is optionally used to capture a larger genomic region (see **FIG. 1B).** In some embodiments, subsets of probes will collectively extend across an entire genomic region (e.g., may be complementary to non-converted or converted fragments from the genomic region). A tiled probe set optionally comprises probes that collectively include at least two probes that overlap every nucleotide in the genomic region. This is done to ensure that cfDNAs comprising a small portion of a target genomic region at one end will have a substantial overlap extending into the adjacent non-targeted genomic region with at least one probe, to provide for efficient capture.

For example, a 100 bp cfDNA fragment comprising a 30 nt target genomic region can be guaranteed to have at least 65 bp overlap with at least one of the overlapping probes. Other levels of tiling are possible. For example, to increase target size and add more probes in a panel, probes can be designed to expand a 30 bp target region by at least 70 bp, 65 bp, 60 bp, 55 bp, or 50 bp. To capture any fragment that overlaps the target region at all (even if by only 1 bp), the probes can be designed to extend past the ends of the target region on either side.

The probes are designed to analyze methylation status of target genomic regions (e.g., of the human or another organism) that are suspected to correlate with the presence or absence of cancer generally, presence or absence of certain types of cancers, cancer stage, or presence or absence of other types of diseases.

Furthermore, the probes are designed to effectively bind and pull down cfDNA fragments containing a target genomic region. In some embodiments, the probes are designed to cover overlapping portions of a target region, so that each probe is "tiled" in coverage such that each probe overlaps in coverage at least partially with another probe in the library. In such embodiments, the panel contains multiple pairs of probes, where each pair comprises at least two probes overlapping each other by an overlapping sequence of at least 25, 30, 35, 40, 45, 50, 60, 70, 75 or 100 nucleotides. In some embodiments, the overlapping sequence can be designed to be complementary to a target genomic region (or a converted version thereof), thus a nucleotide fragment derived from or containing the target genomic region can be bound and pulled down by at least one of the probes. Additionally, probes can be designed to cover both strands of a double-stranded cfDNA sequence.

In one embodiment, the smallest target genomic region is 30 or 31 bp. When a new target region is added to the panel (based on the greedy selection as described above), the new target region of 30bp can be centered on a specific CpG site of interest. Then, it is checked whether each edge of this new target is close enough to other targets such that they can be merged. This is based on a "merge distance" parameter which can be 200bp by default but can be tuned. This allows close but distinct target regions to be enriched with overlapping probes. Depending on whether close enough targets exist to the left or right of the new target, the new target can be merged with nothing (increasing the number of panel targets by one), merged with just one target either to the left or the right (not changing the number of panel targets), or merged with existing targets both to the left and right (reducing the number of panel targets by one).

## Methods of selecting target genomic regions

In another aspect, methods of selecting target genomic regions for detecting cancer and/or a TOO. The targeted genomic regions can be used to design and manufacture probes for a cancer assay panel. Methylation status of DNA or cfDNA molecules corresponding to, or derived from, the target genomic regions can be screened using the cancer assay panel. Alternative methods, for example by WGBS or other methods known in the art, can be also implemented to detect methylation status of DNA molecules or fragments corresponding to, or derived from, the target genomic regions.

*Sample processing*

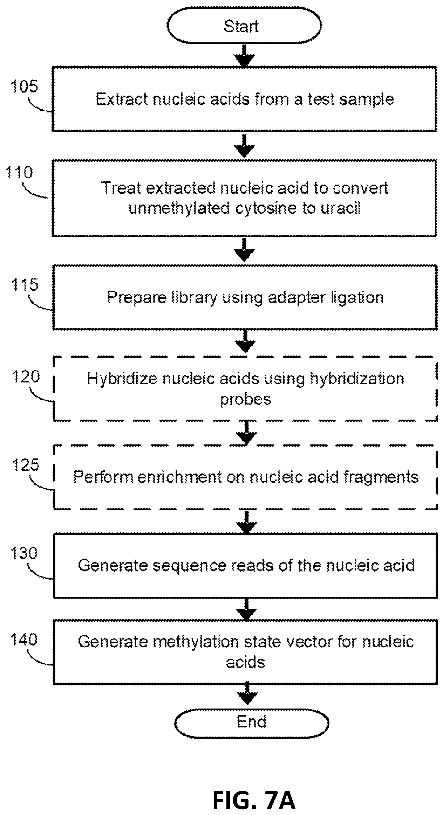

FIG. 7A is a flowchart of a process 100 for processing a nucleic acid sample and generating methylation state vectors for DNA fragments, according to one embodiment. The method includes, but is not limited to, the following steps. For example, any step of the method may comprise a quantitation substep for quality control or other laboratory assay procedures known to one skilled in the art.

In step 105, a nucleic acid sample (DNA or RNA) is extracted from a subject. In the present disclosure, DNA and RNA may be used interchangeably unless otherwise indicated. That is, the embodiments described herein may be applicable to both DNA and RNA types of nucleic acid sequences. However, the examples described herein may focus on DNA for purposes of clarity and explanation. The sample may be any subset of the human genome, including the whole genome. The sample may include blood, plasma, serum, urine, fecal, saliva, other types of bodily fluids, or any combination thereof. In some embodiments, methods for drawing a blood sample (e.g., syringe or finger prick) may be less invasive than procedures for obtaining a tissue biopsy, which may require surgery. The extracted sample may comprise cfDNA and/or ctDNA. For healthy individuals, the human body may naturally clear out cfDNA and other cellular debris. If a subject has a cancer or disease, cfDNA and/or ctDNA in an extracted sample may be present at a detectable level for detecting the cancer or disease.

In step 110, the cfDNA fragments are treated to convert unmethylated cytosines to uracils. In one embodiment, the method uses a bisulfite treatment of the DNA which converts the unmethylated cytosines to uracils without converting the methylated cytosines. For example, a commercial kit such as the EZ DNA Methylation™ - Gold, EZ DNA Methylation™ - Direct or an EZ DNA Methylation™ - Lightning kit (available from Zymo Research Corp (Irvine, CA)) is used for the bisulfite conversion. In another embodiment, the conversion of unmethylated cytosines to uracils is accomplished using an enzymatic reaction. For example, the conversion can use a commercially available kit for conversion of unmethylated cytosines to uracils, such as APOBEC-Seq (NEBiolabs, Ipswich, MA).

In step 115, a sequencing library is prepared. In a first step, a ssDNA adapter is added to the 3'-OH end of a bisulfite-converted ssDNA molecule using a ssDNA ligation reaction. In one embodiment, the ssDNA ligation reaction uses CircLigase II (Epicentre) to ligate the ssDNA adapter to the 3'-OH end of a bisulfite-converted ssDNA molecule, wherein the 5'-end of the adapter is phosphorylated and the bisulfite-converted ssDNA has been dephosphorylated (i.e., the 3' end has a hydroxyl group). In another embodiment, the ssDNA ligation reaction uses Thermostable 5' AppDNA/RNA ligase (available from New England BioLabs (Ipswich, MA)) to ligate the ssDNA adapter to the 3'-OH end of a bisulfite-converted ssDNA molecule. In this example, the first UMI adapter is adenylated at the 5'-end and blocked at the 3'-end. In another embodiment, the ssDNA ligation reaction uses a T4 RNA ligase (available from New England BioLabs) to ligate the ssDNA adapter to the 3'-OH end of a bisulfite-converted ssDNA molecule. In a second step, a second strand DNA is synthesized in an extension reaction. For example, an extension primer, that hybridizes to a primer sequence included in the ssDNA adapter, is used in a primer extension reaction to form a double-stranded bisulfite-converted DNA molecule. Optionally, in one embodiment, the extension reaction uses an enzyme that is able to read through uracil residues in the bisulfite-converted template strand. Optionally, in a third step, a dsDNA adapter is added to the double-stranded bisulfite-converted DNA molecule. Finally, the double-stranded bisulfite-converted DNA is amplified to add sequencing adapters. For example, PCR amplification using a forward primer that includes a P5 sequence and a reverse primer that includes a P7 sequence is used to add P5 and P7 sequences to the bisulfite-converted DNA. Optionally, during library preparation, unique molecular identifiers (UMI) may be added to the nucleic acid molecules (e.g., DNA molecules) through adapter ligation. The UMIs are short nucleic acid sequences (e.g., 4-10 base pairs) that are added to ends of DNA fragments during adapter ligation. In some embodiments, UMIs are degenerate base pairs that serve as a unique tag that can be used to identify sequence reads originating from a specific DNA fragment. During PCR amplification following adapter ligation, the UMIs are replicated along with the attached DNA fragment, which provides a way to identify sequence reads that came from the same original fragment in downstream analysis.

In step 120, targeted DNA sequences may be enriched from the library. This is used, for example, where a targeted panel assay is being performed on the samples. During enrichment, hybridization probes (also referred to herein as "probes") are used to target, and pull down, nucleic acid fragments informative for the presence or absence of cancer (or disease), cancer status, or a cancer classification (e.g., cancer type or tissue of origin). For a given workflow, the probes may be designed to anneal (or hybridize) to a target (complementary) strand of DNA or RNA. The target strand may be the "positive" strand (e.g., the strand transcribed into mRNA, and subsequently translated into a protein) or the complementary "negative" strand. The probes may range in length from 10s, 100s, or 1000s of base pairs. Moreover, the probes may cover overlapping portions of a target region.

After a hybridization step 120, the hybridized nucleic acid fragments are captured and may also be amplified using PCR (enrichment 125). For example, the target sequences can be enriched to obtain enriched sequences that can be subsequently sequenced. In general, any known method in the art can be used to isolate, and enrich for, probe-hybridized target nucleic acids. For example, as is well known in the art, a biotin moiety can be added to the 5'-end of the probes (i.e., biotinylated) to facilitate isolation of target nucleic acids hybridized to probes using a streptavidin-coated surface (e.g., streptavidin-coated beads).

In step 130, sequence reads are generated from the enriched DNA sequences, e.g., enriched sequences. Sequencing data may be acquired from the enriched DNA sequences by known means in the art. For example, the method may include next generation sequencing (NGS) techniques including synthesis technology (Illumina), pyrosequencing (454 Life Sciences), ion semiconductor technology (Ion Torrent sequencing), single-molecule real-time sequencing (Pacific Biosciences), sequencing by ligation (SOLiD sequencing), nanopore sequencing (Oxford Nanopore Technologies), or paired-end sequencing. In some embodiments, massively parallel sequencing is performed using sequencing-by-synthesis with reversible dye terminators.

In step 140, methylation state vectors are generated from the sequence reads. To do so, a sequence read is aligned to a reference genome. The reference genome helps provide the context as to what position in a human genome the fragment cfDNA originates from. In a simplified example, the sequence read is aligned such that the three CpG sites correlate to CpG sites 23, 24, and 25 (arbitrary reference identifiers used for convenience of description). After alignment, there is information both on methylation status of all CpG sites on the cfDNA fragment and which position in the human genome the CpG sites map to. With the methylation status and location, a methylation state vector may be generated for the fragment cfDNA.

*Generation of data structure*

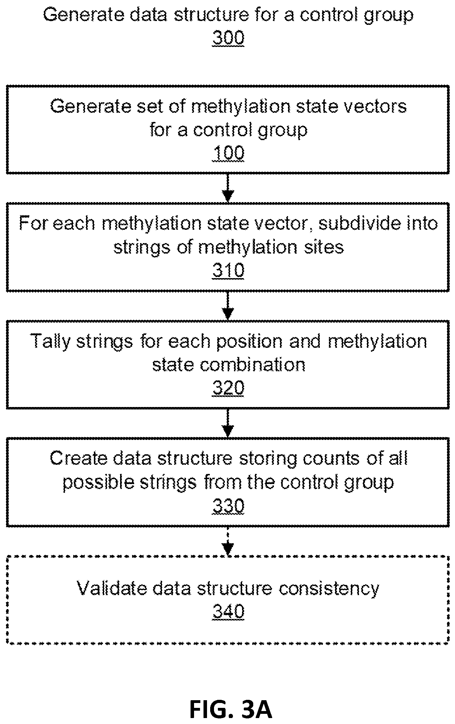

**FIG. 3A** is a flowchart describing a process 300 of generating a data structure for a healthy control group, according to an embodiment. To create a healthy control group data structure, the analytics system obtains information related to methylation status of a plurality of CpG sites on sequence reads derived from a plurality of DNA molecules or fragments from a plurality of healthy subjects. The method provided herein for creating a healthy control group data structure can be performed similarly for subjects with cancer, subjects with cancer of a TOO, subjects with a known cancer type, or subjects with another known disease state. A methylation state vector is generated for each DNA molecule or fragment, for example via the process 100.

The analytics system subdivides 310 the methylation state vector of each DNA fragment into strings of CpG sites. In one embodiment, the analytics system subdivides 310 the methylation state vector such that the resulting strings are all less than a given length. For example, a methylation state vector of length 11 may be subdivided into strings of length less than or equal to 3 would result in 9 strings of length 3, 10 strings of length 2, and 11 strings of length 1. In another example, a methylation state vector of length 7 being subdivided into strings of length less than or equal to 4 would result in 4 strings of length 4, 5 strings of length 3, 6 strings of length 2, and 7 strings of length 1. If the methylation state vector resulting from a DNA fragment is shorter than or the same length as the specified string length, then the methylation state vector may be converted into a single string containing all CpG sites of the vector.

The analytics system tallies 320 the strings by counting, for each possible CpG site and possibility of methylation states in the vector, the number of strings present in the control group having the specified CpG site as the first CpG site in the string and having that possibility of methylation states. For a string length of three at a given CpG site, there are $2^3$ or 8 possible string configurations. For each CpG site, the analytics system tallies 320 how many occurrences of each possible methylation state vector appear in the control group. This may involve tallying the following quantities: $< M_x, M_{x+1}, M_{x+2} >$, $< M_x, M_{x+1}, U_{x+2} >$, ..., $< U_x, U_{x+1}, U_{x+2} >$ for each starting CpG site in the reference genome. The analytics system creates 330 a data structure storing the tallied counts for each starting CpG site and string possibility at each starting CpG.

There are several benefits to setting an upper limit on string length. First, depending on the maximum length for a string, the size of the data structure created by the analytics system can dramatically increase in size. For instance, a maximum string length of 4 means that there are at most $2^4$ numbers to tally at every CpG. Increasing the maximum string length to 5 doubles the possible number of methylation states to tally. Reducing string size helps reduce the computational and data storage burden of the data structure. In some embodiments, the string size is 3. In some embodiments, the string size is 4. A second reason to limit the maximum string length is to avoid overfitting downstream models. Calculating probabilities based on long strings of CpG sites can be problematic if the long CpG strings do not have a strong biological effect on the outcome (e.g., predictions of anomalousness that predictive of the presence of cancer), as it requires a significant amount of data that may not be available, and would thus be too sparse for a model to perform appropriately. For example, calculating a probability of anomalousness/cancer conditioned on the prior 100 CpG sites would require counts of strings in the data structure of length 100, ideally some matching exactly the prior 100 methylation states. If only sparse counts of strings of length 100 are available, there will be insufficient data to determine whether a given string of length of 100 in a test sample is anomalous or not.

*Validation of data structure*

Once the data structure has been created, the analytics system may seek to validate 340 the data structure and/or any downstream models making use of the data structure.

This first type of validation ensures that potential cancerous samples are removed from the healthy control group so as to not affect the control group's purity. This type of validation checks consistency within the control group's data structure. For example, the healthy control group may contain a sample from an individual with an undiagnosed cancer that contains a plurality of anomalously methylated fragments. The analytics system may perform various calculations to determine whether to exclude data from a subject with apparently undiagnosed cancer.

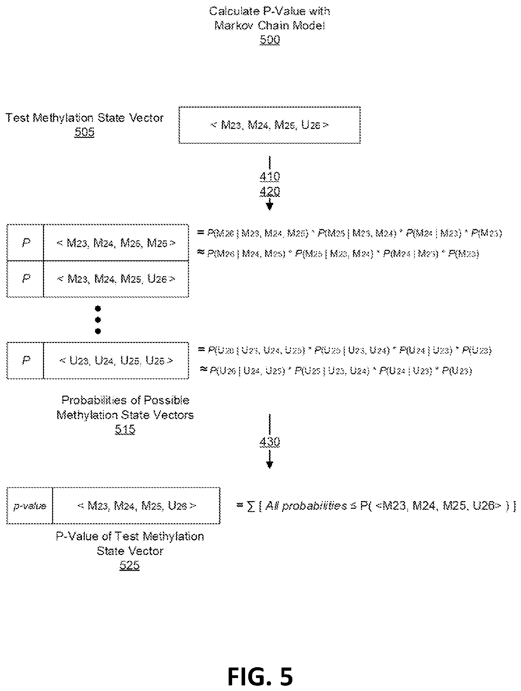

A second type of validation checks the probabilistic model used to calculate p-values with the counts from the data structure itself (i.e., from the healthy control group). A process for p-value calculation is described below in conjunction with **FIG. 5.** Once the analytics system generates a p-value for the methylation state vectors in the validation group, the analytics system builds a cumulative density function (CDF) with the p-values. With the CDF, the analytics system may perform various calculations on the CDF to validate the control group's data structure. One test uses the fact that the CDF should ideally be at or below an identity function, such that $CDF(x) \leq x$. On the converse, being above the identity function reveals some deficiency within the probabilistic model used for the control group's data structure. For example, if 1/100 of fragments have a p-value score of 1/1000 meaning CDF(1/1000) = 1/100 > 1/1000, then the second type of validation fails indicating an issue with the probabilistic model. *See* e.g., U.S. Appl. No. 16/352,602, published as U.S. Publ. No. 2019/0287652, which is hereby incorporated by reference in its entirety.

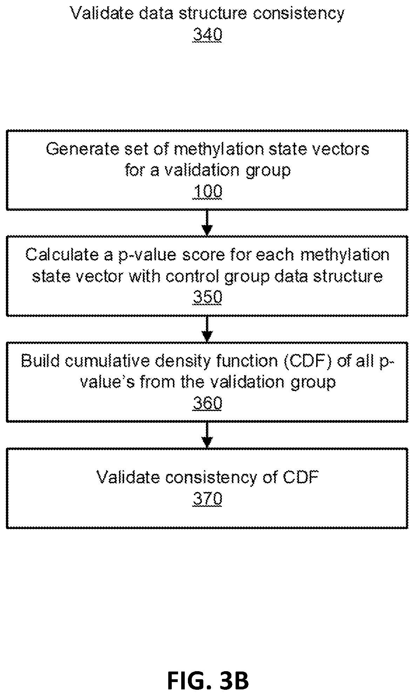

A third type of validation uses a healthy set of validation samples separate from those used to build the data structure. This tests if the data structure is properly built and the model works. An exemplary process for carrying out this type of validation is described below in conjunction with **FIG. 3B.** The third type of validation can quantify how well the healthy control group generalizes the distribution of healthy samples. If the third type of validation fails, then the healthy control group does not generalize well to the healthy distribution.

A fourth type of validation tests with samples from a non-healthy validation group. The analytics system calculates p-values and builds the CDF for the non-healthy validation group. With a non-healthy validation group, the analytics system expects to see the CDF(x) > x for at least some samples or, stated differently, the converse of what was expected in the second type of validation and the third type of validation with the healthy control group and the healthy validation group. If the fourth type of validation fails, then this is indicative that the model is not appropriately identifying the anomalousness that it was designed to identify.

**FIG. 3B** is a flowchart describing the additional step 340 of validating the data structure for the control group of **FIG. 3A,** according to an embodiment. In this embodiment of the step 340 of validating the data structure, the analytics system performs the fourth type of validation test as described above which utilizes a validation group with a supposedly similar composition of subjects, samples, and/or fragments as the control group. For example, if the analytics system selected healthy subjects without cancer for the control group, then the analytics system also uses healthy subjects without cancer in the validation group.

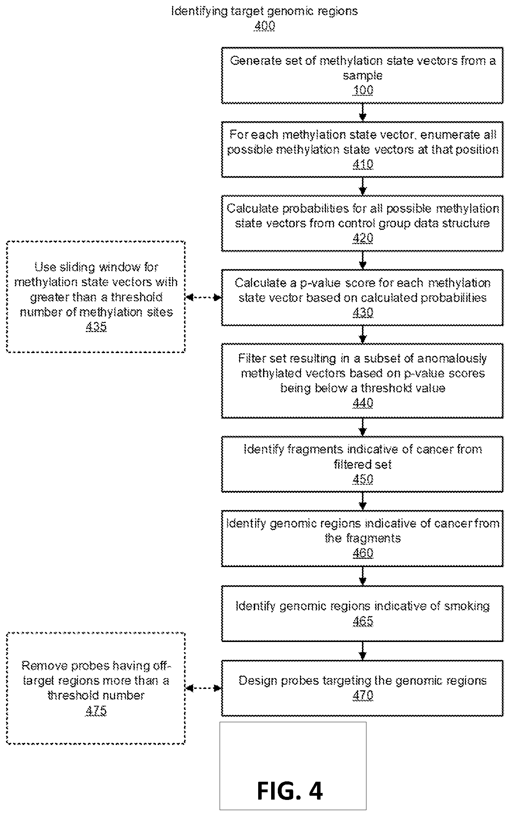

The analytics system takes the validation group and generates 100 a set of methylation state vectors as described in **FIG. 3A.** The analytics system performs a p-value calculation for each methylation state vector from the validation group. The p-value calculation process will be further described in conjunction with **FIGS. 4-5.** For each possible methylation state vector, the analytics system calculates a probability from the control group's data structure. Once the probabilities are calculated for the possibilities of methylation state vectors, the analytics system calculates 350 a p-value score for that methylation state vector based on the calculated probabilities. The p-value score represents an expectedness of finding that specific methylation state vector and other possible methylation state vectors having even lower probabilities in the control group. A low p-value score, thereby, generally corresponds to a methylation state vector which is relatively unexpected in comparison to other methylation state vectors within the control group, whereas a high p-value score generally corresponds to a methylation state vector which is relatively more expected in comparison to other methylation state vectors found in the control group. Once the analytics system generates a p-value score for the methylation state vectors in the validation group, the analytics system builds 360 a cumulative density function (CDF) with the p-value scores from the validation group. The analytics system validates 370 consistency of the CDF as described above in the fourth type of validation tests.

*Anomalously methylated fragments*

Anomalously methylated fragments having abnormal methylation patterns in cancer patient samples, subject with cancer of a TOO, subjects with a known cancer type, or subjects with another known disease state, are selected as target genomic regions, according to an embodiment as outlined in **FIG. 4.** Exemplary processes of selected anomalously methylated fragments 440 are visually illustrated in **FIG. 5** and is further described below the description of **FIG. 4.** In process 400, the analytics system generates 100 methylation state vectors from cfDNA fragments of the sample. The analytics system handles each methylation state vector as follows.

For a given methylation state vector, the analytics system enumerates 410 all possibilities of methylation state vectors having the same starting CpG site and same length (i.e., set of CpG sites) in the methylation state vector. As each methylation state may be methylated or unmethylated there are only two possible states at each CpG site, and thus the count of distinct possibilities of methylation state vectors depends on a power of 2, such that a methylation state vector of length n would be associated with $2^n$ possibilities of methylation state vectors.

The analytics system calculates 420 the probability of observing each possibility of methylation state vector for the identified starting CpG site / methylation state vector length by accessing the healthy control group data structure. In one embodiment, calculating the probability of observing a given possibility uses a Markov chain probability to model the joint probability calculation which will be described in greater detail with respect to **FIG. 5** below. In other embodiments, calculation methods other than Markov chain probabilities are used to determine the probability of observing each possibility of methylation state vector.

The analytics system calculates 430 a p-value score for the methylation state vector using the calculated probabilities for each possibility. In one embodiment, this includes identifying the calculated probability corresponding to the possibility that matches the methylation state vector in question. Specifically, this is the possibility having the same set of CpG sites, or similarly the same starting CpG site and length as the methylation state vector. The analytics system sums the calculated probabilities of any possibilities having probabilities less than or equal to the identified probability to generate the p-value score.

This p-value represents the probability of observing the methylation state vector of the fragment or other methylation state vectors even less probable in the healthy control group. A low p-value score, thereby, generally corresponds to a methylation state vector which is rare in a healthy subject, and which causes the fragment to be labeled abnormally methylated, relative to the healthy control group. A high p-value score generally relates to a methylation state vector is expected to be present, in a relative sense, in a healthy subject. If the healthy control group is a non-cancerous group, for example, a low p-value indicates that the fragment is abnormally methylated relative to the non-cancer group, and therefore possibly indicative of the presence of cancer in the test subject.

As above, the analytics system calculates p-value scores for each of a plurality of methylation state vectors, each representing a cfDNA fragment in the test sample. To identify which of the fragments are abnormally methylated, the analytics system may filter 440 the set of methylation state vectors based on their p-value scores. In one embodiment, filtering is performed by comparing the p-values scores against a threshold and keeping only those fragments below the threshold. This threshold p-value score could be on the order of 0.1, 0.01, 0.001, 0.0001, or similar.

*P-value score calculation*

**FIG. 5** is an illustration 500 of an example p-value score calculation, according to an embodiment. To calculate a p-value score given a test methylation state vector 505, the analytics system takes that test methylation state vector 505 and enumerates 410 possibilities of methylation state vectors. In this illustrative example, the test methylation state vector 505 is < M23, M24, M25, U26 >. As the length of the test methylation state vector 505 is 4, there are $2^4$ possibilities of methylation state vectors encompassing CpG sites 23 - 26. In a generic example, the number of possibilities of methylation state vectors is $2^n$, where n is the length of the test methylation state vector or alternatively the length of the sliding window (described further below).

The analytics system calculates 420 probabilities 515 for the enumerated possibilities of methylation state vectors. As methylation is conditionally dependent on methylation status of nearby CpG sites, one way to calculate the probability of observing a given methylation state vector possibility is to use Markov chain model. Generally, a methylation state vector such as $<S_1, S_2, ..., S_n>$, where S denotes the methylation state whether methylated (denoted as M), unmethylated (denoted as U), or indeterminate (denoted as I), has a joint probability that can be expanded using the chain rule of probabilities as:

$$P(< S_1, S_2, ..., S_n >) = P(S_n \mid S_1, ..., S_{n-1} ) * P(S_{n-1} \mid S_1, ..., S_{n-2} ) * ... * P(S_2 \mid S_1) * P(S_1) \tag{1}$$

Markov chain model can be used to make the calculation of the conditional probabilities of each possibility more efficient. In one embodiment, the analytics system selects a Markov chain order k which corresponds to how many prior CpG sites in the vector (or window) to consider in the conditional probability calculation, such that the conditional probability is modeled as $P(S_n \mid S_1, ..., S_{n-1} ) \sim P(S_n \mid S_{n-k-2}, ..., S_{n-1} )$.

To calculate each Markov modeled probability for a possibility of methylation state vector, the analytics system accesses the control group's data structure, specifically the counts of various strings of CpG sites and states. To calculate $P(M_n \mid S_{n-k-2}, ..., S_{n-1} )$, the analytics system takes a ratio of the stored count of the number of strings from the data structure

matching $< S_{n-k-2}, ..., S_{n-1}, M_n >$ divided by the sum of the stored count of the number of strings from the data structure matching $< S_{n-k-2}, ..., S_{n-1}, M_n >$ and $< S_{n-k-2}, ..., S_{n-1}, U_n >$. Thus, $P(M_n \mid S_{n-k-2}, ..., S_{n-1})$, is calculated ratio having the form:

$$\frac{\# \text{ of } < S_{n-k-2}, ..., S_{n-1}, M_n >}{\# \text{ of } < S_{n-k-2}, ..., S_{n-1}, M_n > + \# \text{ of } < S_{n-k-2}, ..., S_{n-1}, U_n >}$$

The calculation may additionally implement a smoothing of the counts by applying a prior distribution. In one embodiment, the prior distribution is a uniform prior as in Laplace smoothing. As an example of this, a constant is added to the numerator and another constant (e.g., twice the constant in the numerator) is added to the denominator of the above equation. In other embodiments, an algorithmic technique such as Knesser-Ney smoothing is used.

In the illustration, the above denoted formulas are applied to the test methylation state vector 505 covering sites 23 - 26. Once the calculated probabilities 515 are completed, the analytics system calculates 430 a p-value score 525 that sums the probabilities that are less than or equal to the probability of possibility of methylation state vector matching the test methylation state vector 505.

In one embodiment, the computational burden of calculating probabilities and/or p-value scores may be further reduced by caching at least some calculations. For example, the analytic system may cache in transitory or persistent memory calculations of probabilities for possibilities of methylation state vectors (or windows thereof). If other fragments have the same CpG sites, caching the possibility probabilities allows for efficient calculation of p-value scores without needing to re-calculate the underlying possibility probabilities. Equivalently, the analytics system may calculate p-value scores for each of the possibilities of methylation state vectors associated with a set of CpG sites from vector (or window thereof). The analytics system may cache the p-value scores for use in determining the p-value scores of other fragments including the same CpG sites. Generally, the p-value scores of possibilities of methylation state vectors having the same CpG sites may be used to determine the p-value score of a different one of the possibilities from the same set of CpG sites.

*Sliding window*

In one embodiment, the analytics system uses 435 a sliding window to determine possibilities of methylation state vectors and calculate p-values. Rather than enumerating possibilities and calculating p-values for entire methylation state vectors, the analytics system enumerates possibilities and calculates p-values for only a window of sequential CpG sites, where the window is shorter in length (of CpG sites) than at least some fragments (otherwise, the window would serve no purpose). The window length may be static, user determined, dynamic, or otherwise selected.

In calculating p-values for a methylation state vector larger than the window, the window identifies the sequential set of CpG sites from the vector within the window starting from the first CpG site in the vector. The analytic system calculates a p-value score for the window including the first CpG site. The analytics system then "slides" the window to the second CpG site in the vector, and calculates another p-value score for the second window. Thus, for a window size *l* and methylation vector length *m,* each methylation state vector will generate *m-l+1* p-value scores. After completing the p-value calculations for each portion of the vector, the lowest p-value score from all sliding windows is taken as the overall p-value score for the methylation state vector. In another embodiment, the analytics system aggregates the p-value scores for the methylation state vectors to generate an overall p-value score.

Using the sliding window helps to reduce the number of enumerated possibilities of methylation state vectors and their corresponding probability calculations that would otherwise need to be performed. Example probability calculations are shown in **FIG. 5,** but generally the number of possibilities of methylation state vectors increases exponentially by a factor of 2 with the size of the methylation state vector. To give a realistic example, it is possible for fragments to have upwards of 54 CpG sites. Instead of computing probabilities for 2^54 (~$1.8 \times 10$^16) possibilities to generate a single p-value, the analytics system can instead use a window of size 5 (for example) which results in 50 p-value calculations for each of the 50 windows of the methylation state vector for that fragment. Each of the 50 calculations enumerates 2^5 (32) possibilities of methylation state vectors, which total results in $50 \times 2$^5 ($1.6 \times 10$^3) probability calculations. This results in a vast reduction of calculations to be performed, with no meaningful hit to the accurate identification of anomalous fragments. This additional step can also be applied when validating 340 the control group with the validation group's methylation state vectors.

*Identifying fragments indicative of cancer*

The analytics system identifies 450 DNA fragments indicative of cancer from the filtered set of anomalously methylated fragments.

*Hypomethylated and hypermethylated fragments*

According to a first method, the analytics system may identify DNA fragments that are deemed hypomethylated or hypermethylated as fragments indicative of cancer from the filtered set of anomalously methylated fragments. Hypomethylated and hypermethylated fragments can be defined as fragments of a certain length of CpG sites (e.g., more than 3, 4, 5, 6, 7, 8, 9, 10, etc.) with a high percentage of methylated CpG sites (e.g., more than 80%, 85%, 90%, or 95%, or any other percentage within the range of 50%-100%) or a high percentage of unmethylated CpG sites (e.g., more than 80%, 85%, 90%, or 95%, or any other percentage within the range of 50%-100%).

*Probabilistic models*

According to a method described herein, the analytics system identifies fragments indicative of cancer utilizing probabilistic models of methylation patterns fitted to each cancer type and non-cancer type. The analytics system calculates log-likelihood ratios for a sample using DNA fragments in the genomic regions considering the various cancer types with the fitted probabilistic models for each cancer type and non-cancer type. The analytics system may determine a DNA fragment to be indicative of cancer based on whether at least one of the log-likelihood ratios considered against the various cancer types is above a threshold value.

In one embodiment of partitioning the genome, the analytics system partitions the genome into regions by multiple stages. In a first stage, the analytics system separates the genome into blocks of CpG sites. Each block is defined when there is a separation between two adjacent CpG sites that exceeds some threshold, e.g., greater than 200 bp, 300 bp, 400 bp, 500 bp, 600 bp, 700 bp, 800 bp, 900 bp, or 1,000 bp. From each block, the analytics system subdivides at a second stage each block into regions of a certain length, e.g., 500 bp, 600 bp, 700 bp, 800 bp, 900 bp, 1,000 bp, 1,100 bp, 1,200 bp, 1,300 bp, 1,400 bp, or 1,500 bp. The analytics system may further overlap adjacent regions by a percentage of the length, e.g., 10%, 20%, 30%, 40%, 50%, or 60%.

The analytics system analyzes sequence reads derived from DNA fragments for each region. The analytics system may process samples from tissue and/or high-signal cfDNA. High-signal cfDNA samples may be determined by a binary classification model, by cancer stage, or by another metric.

For each cancer type and non-cancer, the analytics system fits a separate probabilistic model for fragments. In one example, each probabilistic model is mixture model comprising a combination of a plurality of mixture components with each mixture component being an independent-sites model where methylation at each CpG site is assumed to be independent of methylation statuses at other CpG sites.

In alternate embodiments, calculation is performed with respect to each CpG site. Specifically, a first count is determined that is the number of cancerous samples (cancer_count) that include an anomalously methylated DNA fragment overlapping that CpG, and a second count is determined that is the total number of samples containing fragments overlapping that CpG (total) in the set. Genomic regions can be selected based on the numbers, for example, based on criteria positively correlated to the number of cancerous samples (cancer_count) that include a DNA fragment overlapping that CpG, and inversely correlated to the total number of samples containing fragments overlapping that CpG (total) in the set.

Cancer of various types having different TOO can be selected from the group consisting of: breast cancer, uterine cancer, cervical cancer, ovarian cancer, bladder cancer, urothelial cancer of renal pelvis, renal cancer other than urothelial, prostate cancer, anorectal cancer, anal cancer, colorectal cancer, hepatobiliary cancer arising from hepatocytes, hepatobiliary cancer arising from cells other than hepatocytes, liver/bile-duct cancer, esophageal cancer, pancreatic cancer, stomach cancer, squamous cell cancer of the upper gastrointestinal tract, upper gastrointestinal cancer other than squamous, head and neck cancer, lung cancer, lung adenocarcinoma, small cell lung cancer, squamous cell lung cancer and cancer other than adenocarcinoma or small cell lung cancer, neuroendocrine cancer, melanoma, thyroid cancer, sarcoma, plasma cell neoplasm, multiple myeloma, myeloid neoplasm, lymphoma, and leukemia.

In some embodiments, various cancer types can be classified and labeled using classification methods available in the art, such as the International Classification of Diseases for Oncology (ICD-O-3) (codes.iarc.fr) or the Surveillance, Epidemiology, and End Results Program (SEER) (seer.cancer.gov). In other embodiments, cancer types are classified in three orthogonal codes, (i) topographical codes, (ii) morphological codes, or (iii) behavioral codes. Under behavioral codes, benign tumor is 0, uncertain behavior is 1, carcinoma in situ is 2, malignant, primary site is 3 and malignant, metastatic site is 6.

In some embodiments, a cancer TOO can be selected from a group defined by the guideline that will be used to stage a detected cancer. For example, the reference, Amin, M.B., Edge, S., Greene, F., Byrd, D.R., Brookland, R.K., Washington, M.K., Gershenwald, J.E., Compton, C.C., Hess, K.R., Sullivan, D.C., Jessup, J.M., Brierley, J.D., Gaspar, L.E., Schilsky, R.L., Balch, C.M., Winchester, D.P., Asare, E.A., Madera, M., Gress, D.M., Meyer, L.R. (Eds.), AJCC Cancer Staging Manual, 8th edition, Springer, 2017, identifies groups of different cancers that are staged together following standard guidelines. Staging is typically a next step in cancer management following its detection and diagnosis.

The analytics system can further calculate log-likelihood ratios ("R") for a fragment indicating a likelihood of the fragment being indicative of cancer considering the various cancer types with the fitted probabilistic models for each cancer type and non-cancer type, or for a cancer TOO. The two probabilities may be taken from probabilistic models fitted for each of the cancer types and the non-cancer type, the probabilistic models defined to calculate a likelihood of observing a methylation pattern on a fragment given each of the cancer types and the non-cancer type. For example, the probabilistic models may be defined fitted for each of the cancer types and the non-cancer type.

*Selection of genomic regions indicative of cancer*

In some embodiments, the analytics system can identify 460 genomic regions indicative of cancer. To identify these informative regions, the analytics system calculates an information gain for each genomic region or more specifically each CpG site that describes an ability to distinguish between various outcomes.

A method for identifying genomic regions capable of distinguishing between cancer type and non-cancer type utilizes a trained classification model that can be applied on the set of anomalously methylated DNA molecules or fragments corresponding to or derived from a cancerous or non-cancerous group. The trained classification model can be trained to identify any condition of interest that can be identified from the methylation state vectors.

In one embodiment, the trained classification model is a binary classifier trained based on methylation states for cfDNA fragments or genomic sequences obtained from a subject cohort with cancer or a cancer TOO, and a healthy subject cohort without cancer, and is then used to classify a test subject probability of having cancer, a cancer TOO, or not having cancer, based on anomalously methylation state vectors. In other embodiments, different classifiers may be trained using subject cohorts known to have particular cancer (e.g., breast, lung, prostrate, etc.); known to have cancer of particular TOO where the cancer is believed to originate; or known to have different stages of particular cancer (e.g., breast, lung, prostrate, etc.). In these embodiments, different classifiers may be trained using sequence reads obtained from samples enriched for tumor cells from subject cohorts known to have particular cancer (e.g., breast, lung, prostrate, etc.). Each genomic region's ability to distinguish between cancer type and non-cancer type in the classification model is used to rank the genomic regions from most informative to least informative in classification performance. The analytics system may identify genomic regions from the ranking according to information gain in classification between non-cancer type and cancer type.

*Computing information gain from hypomethylated and hypermethylated fragments indicative of cancer*

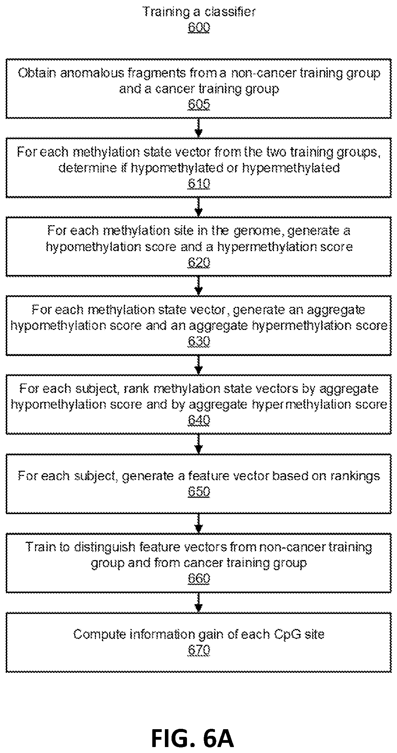

With fragments indicative of cancer, the analytics system may train a classifier according to a process 600 illustrated in **FIG. 6A,** according to an embodiment. The process 600 accesses two training groups of samples - a non-cancer group and a cancer group - and obtains 605 a non-cancer set of methylation state vectors and a cancer set of methylation state vectors comprising anomalously methylated fragments, e.g., via step 440 from the process 400.

The analytics system determines 610, for each methylation state vector, whether the methylation state vector is indicative of cancer. Here, fragments indicative of cancer may be defined as hypermethylated or hypomethylated fragments determined if at least some number of CpG sites have a particular state (methylated or unmethylated, respectively) and/or have a threshold percentage of sites that are the particular state (again, methylated or unmethylated, respectively). In one example, cfDNA fragments are identified as hypomethylated or hypermethylated, respectively, if the fragment overlaps at least 5 CpG sites, and at least 80%, 90%, or 100% of its CpG sites are methylated or at least 80%, 90%, or 100% are unmethylated.

In an alternate embodiment, the analytics system considers portions of the methylation state vector and determines whether the portion is hypomethylated or hypermethylated, and may distinguish that portion to be hypomethylated or hypermethylated. This alternative resolves missing methylation state vectors which are large in size but contain at least one region of dense hypomethylation or hypermethylation. This process of defining hypomethylation and hypermethylation can be applied in step 450 of **FIG. 4.** In another embodiment, the fragments indicative of cancer may be defined according to likelihoods outputted from trained probabilistic models.

In one embodiment, the analytics system generates 620 a hypomethylation score ($P_{hypo}$) and a hypermethylation score ($P_{hyper}$) per CpG site in the genome. To generate either score at a given CpG site, the classifier takes four counts at that CpG site - (1) count of (methylations state) vectors of the cancer set labeled hypomethylated that overlap the CpG site; (2) count of vectors of the cancer set labeled hypermethylated that overlap the CpG site; (3) count of vectors of the non-cancer set labeled hypomethylated that overlap the CpG site; and (4) count of vectors of the non-cancer set labeled hypermethylated that overlap the CpG site. Additionally, the process may normalize these counts for each group to account for variance in group size between the non-cancer group and the cancer group. In alternative embodiments wherein fragments indicative of cancer are more generally used, the scores may be more broadly defined as counts of fragments indicative of cancer at each genomic region and/or CpG site.

In one embodiment, to generate 620 the hypomethylation score at a given CpG site, the process takes a ratio of (1) over (1) summed with (3). Similarly, the hypermethylation score is calculated by taking a ratio of (2) over (2) and (4). Additionally, these ratios may be calculated with an additional smoothing technique as discussed above. The hypomethylation score and the hypermethylation score relate to an estimate of cancer probability given the presence of hypomethylation or hypermethylation of fragments from the cancer set.

The analytics system generates 630 an aggregate hypomethylation score and an aggregate hypermethylation score for each anomalous methylation state vector. The aggregate hyper and hypo methylation scores are determined based on the hyper and hypo methylation scores of the CpG sites in the methylation state vector. In one embodiment, the aggregate hyper and hypo methylation scores are assigned as the largest hyper and hypo methylation scores of the sites in each state vector, respectively. However, in alternate embodiments, the aggregate scores could be based on means, medians, or other calculations that use the hyper/hypo methylation scores of the sites in each vector.

The analytics system ranks 640 all of that subject's methylation state vectors by their aggregate hypomethylation score and by their aggregate hypermethylation score, resulting in two rankings per subject. The process selects aggregate hypomethylation scores from the hypomethylation ranking and aggregate hypermethylation scores from the hypermethylation ranking. With the selected scores, the classifier generates 650 a single feature vector for each subject. In one embodiment, the scores selected from either ranking are selected with a fixed order that is the same for each generated feature vector for each subject in each of the training groups. As an example, in one embodiment the classifier selects the first, the second, the fourth, and the eighth aggregate hyper methylation score, and similarly for each aggregate hypo methylation score, from each ranking and writes those scores in the feature vector for that subject.

The analytics system trains 660 a binary classifier to distinguish feature vectors between the cancer and non-cancer training groups. Generally, any one of a number of classification techniques may be used. In one embodiment the classifier is a non-linear classifier. In a specific embodiment, the classifier is a non-linear classifier utilizing a L2-regularized kernel logistic regression with a Gaussian radial basis function (RBF) kernel.

Specifically, in one embodiment, the number of non-cancer samples or different cancer type(s) ($n_{other}$) and the number of cancer samples or cancer type(s) ($n_{cancer}$) having an anomalously methylated fragment overlapping a CpG site are counted. Then the probability that a sample is cancer is estimated by a score ("S") that positively correlates to $n_{cancer}$ and inversely correlated to $n_{other}$. The score can be calculated using the equation: $(n_{cancer} + 1) / (n_{cancer} + n_{other} + 2)$ or $(n_{cancer}) / (n_{cancer} + n_{other})$. The analytics system computes 670 an information gain for each cancer type and for each genomic region or CpG site to determine whether the genomic region or CpG site is indicative of cancer. The information gain is computed for training samples with a given cancer type compared to all other samples. For example, two random variables 'anomalous fragment' ('AF') and 'cancer type" ('CT') are used. In on embodiment, AF is a binary variable indicating whether there is an anomalous fragment overlapping a given CpG site in a given samples as determined for the anomaly score / feature vector above. CT is a random variable indicating whether the cancer is of a particular type. The analytics system computes the mutual information with respect to CT given AF. That is, how many bits of information about the cancer type are gained if it is known whether there is an anomalous fragment overlapping a particular CpG site.

For a given cancer type, the analytics system uses this information to rank CpG sites based on how cancer specific they are. This procedure is repeated for all cancer types under consideration. If a particular region is commonly anomalously methylated in training samples of a given cancer but not in training samples of other cancer types or in healthy training samples, then CpG sites overlapped by those anomalous fragments will tend to have high information gains for the given cancer type. The ranked CpG sites for each cancer type are greedily added (selected) to a selected set of CpG sites based on their rank for use in the cancer classifier.

*Computing pairwise information gain from fragments indicative of cancer identified from probabilistic models*

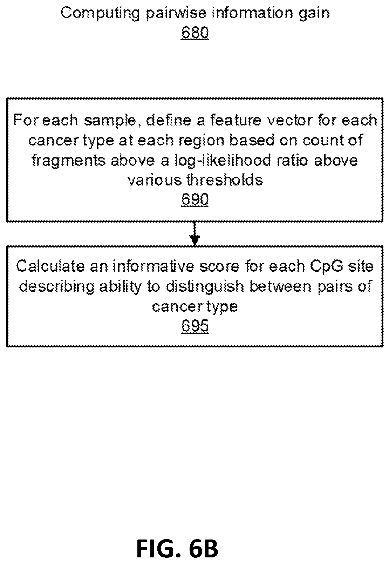

With fragments indicative of cancer identified according to the second method described herein, the analytics may identify genomic regions according to the process 680 in FIG. 6B. The analytics system defines 690 a feature vector for each sample, for each region, for each cancer type by a count of DNA fragments that have a calculated log-likelihood ratio that the fragment is indicative of cancer above a plurality of thresholds, wherein each count is a value in the feature vector. In one embodiment, the analytics system counts the number of fragments present in a sample at a region for each cancer type with log-likelihood ratios above one or a plurality of possible threshold values. The analytics system defines a feature vector for each sample, by a count of DNA fragments for each genomic region for each cancer type that provides a calculated log-likelihood ratio for the fragment above a plurality of thresholds, wherein each count is a value in the feature vector. The analytics system uses the defined feature vectors to calculate an informative score for each genomic region describing that genomic region's ability to distinguish between each pair of cancer types. For each pair of cancer types, the analytics system ranks regions based on the informative scores. The analytics system may select regions based on the ranking according to informative scores.

The analytics system calculates 695 an informative score for each region describing that region's ability to distinguish between each pair of cancer types. For each pair of distinct cancer types, the analytics system may specify one type as a positive type and the other as a negative type. In one embodiment, a region's ability to distinguish between the positive type and the negative type is based on mutual information, calculated using the estimated fraction of cfDNA samples of the positive type and of the negative type for which the feature would be expected to be non-zero in the final assay, i.e., at least one fragment of that tier that would be sequenced in a targeted methylation assay. Those fractions are estimated using the observed rates at which the feature occurs in healthy cfDNA, and in high-signal cfDNA and/or tumor samples of each cancer type. For example, if a feature occurs frequently in healthy cfDNA, then it will also be estimated to occur frequently in cfDNA of any cancer type and would likely result in a low informative score. The analytics system may choose a certain number of regions for each pair of cancer types from the ranking, e.g., 1024.

In additional embodiments, the analytics system further identifies predominantly hypermethylated or hypomethylated regions from the ranking of regions. The analytics system may load the set of fragments in the positive type(s) for a region that was identified as informative. The analytics system, from the loaded fragments, evaluates whether the loaded fragments are predominantly hypermethylated or hypomethylated. If the loaded fragments are predominately hypermethylated or hypomethylated, the analytics system may select probes corresponding to the predominant methylation pattern. If the loaded fragments are not predominantly hypermethylated or hypomethylated, the analytics system may use a mixture of probes for targeting both hypermethylation and hypomethylation. The analytics system may further identify a minimal set of CpG sites that overlap more than some percentage of the fragments.

In other embodiments, the analytics system, after ranking the regions based on informative scores, labels each region with the lowest informative ranking across all pairs of cancer types. For example, if a region was the 10th-most-informative region for distinguishing breast from lung, and the 5th-most-informative for distinguishing breast from colorectal, then it would be given an overall label of "5". The analytics system may design probes starting with the lowest-labeled regions while adding regions to the panel, e.g., until the panel's size budget has been exhausted.

*Off-target genomic regions*

In some embodiments, probes targeting selected genomic regions are further filtered 475 based on the number of their off-target regions. This is for screening probes that pull down too many cfDNA fragments corresponding to, or derived from, off-target genomic regions. Exclusion of probes having many off-target regions can be valuable by decreasing off-target rates and increasing target coverage for a given amount of sequencing.

An off-target genomic region is a genomic region that has sufficient homology to a target genomic region, such that DNA molecules or fragments derived from off-target genomic regions are hybridized to and pulled down by a probe designed to hybridize to a target genomic region. An off-target genomic region can be a genomic region (or a converted sequence of that same region) that aligns to a probe along at least 35 bp, 40 bp, 45 bp, 50 bp, 60 bp, 70 bp, or 80 bp with at least an 80%, 85%, 90%, 95%, or 97% match rate. In one embodiment, an off-target genomic region is a genomic region (or a converted sequence of that same region) that aligns to a probe along at least 45bp with at least a 90% match rate. Various methods known in the art can be adopted to screen off-target genomic regions.

Exhaustively searching the genome to find all off-target genomic regions can be computationally challenging. In one embodiment, a k-mer seeding strategy (which can allow one or more mismatches) is combined to local alignment at the seed locations. In this case, exhaustive searching of good alignments can be guaranteed based on k-mer length, number of mismatches allowed, and number of k-mer seed hits at a particular location. This requires doing dynamic programing local alignment at a large number of locations, so this approach is highly optimized to use vector CPU instructions (e.g., AVX2, AVX512) and also can be parallelized across many cores within a machine and also across many machines connected by a network. A person of ordinary skill will recognize that modifications and variations of this approach can be implemented for the purpose of identifying off-target genomic regions.

In some embodiments, probes having sequence homology with off-target genomic regions, or DNA molecules corresponding to, or derived from off-target genomic regions comprising more than a threshold number are excluded (or filtered) from the panel. For example, probes having sequence homology with off-target genomic regions, or DNA molecules corresponding to, or derived from off-target genomic regions from more than 30, more than 25, more than 20, more than 18, more than 15, more than 12, more than 10, or more than 5 off-target regions are excluded.

In some embodiments, probes are divided into 2, 3, 4, 5, 6, or more separate groups depending on the numbers of off-target regions. For example, probes having sequence homology with no off-target regions or DNA molecules corresponding to, or derived from off-target regions are assigned to high-quality group, probes having sequence homology with 1-18 off-target regions or DNA molecules corresponding to, or derived from 1-18 off-target regions, are assigned to low-quality group, and probes having sequence homology with more than 19 off-target regions or DNA molecules corresponding to, or derived from 19 off-target regions, are assigned to poor-quality group. Other cut-off values can be used for the grouping.

In some embodiments, probes in the lowest quality group are excluded. In some embodiments, probes in groups other than the highest-quality group are excluded. In some embodiments, separate panels are made for the probes in each group. In some embodiments, all the probes are put on the same panel, but separate analysis is performed based on the assigned groups.

In some embodiments, a panel comprises a larger number of high-quality probes than the number of probes in lower groups. In some embodiments, a panel comprises a smaller number of poor-quality probes than the number of probes in other group. In some embodiments, more than 95%, 90%, 85%, 80%, 75%, or 70% of probes in a panel are high-quality probes. In some embodiments, less than 35%, 30%, 20%, 10%, 5%, 4%, 3%, 2% or 1% of the probes in a panel are low-quality probes. In some embodiments, less than 5%, 4%, 3%, 2% or 1% of the probes in a panel are poor-quality probes. In some embodiments, no poor-quality probes are included in a panel.

In some embodiments, probes having below 50%, below 40%, below 30%, below 20%, below 10% or below 5% are excluded. In some embodiments, probes having above 30%, above 40%, above 50%, above 60%, above 70%, above 80%, or above 90% are selectively included in a panel.

### Methods of using cancer assay panel

In yet another aspect, methods of using a cancer assay panel (alternatively referred to as a "bait set") are provided. The methods can comprise steps of treating DNA molecules or fragments to convert unmethylated cytosines to uracils (e.g., using bisulfite treatment), applying a cancer panel (as described herein) to the converted DNA molecules or fragments, enriching a subset of converted DNA molecules or fragments that bind to the probes in the panel, and sequencing the enriched cfDNA fragments. In some embodiments, the sequence reads can be compared to a reference genome (e.g., a human reference genome), allowing for identification of methylation states at a plurality of CpG sites within the DNA molecules or fragments and thus provide information relevant to detection of cancer.

*Analysis of sequence reads*

In some embodiments, the sequence reads may be aligned to a reference genome using known methods in the art to determine alignment position information. The alignment position information may indicate a beginning position and an end position of a region in the reference genome that corresponds to a beginning nucleotide base and end nucleotide base of a given sequence read. Alignment position information may also include sequence read length, which can be determined from the beginning position and end position. A region in the reference genome may be associated with a gene or a segment of a gene.

In various embodiments, a sequence read is comprised of a read pair denoted as $R_1$ and $R_2$. For example, the first read $R_1$ may be sequenced from a first end of a nucleic acid fragment whereas the second read $R_2$ may be sequenced from the second end of the nucleic acid fragment. Therefore, nucleotide base pairs of the first read $R_1$ and second read $R_2$ may be aligned consistently (e.g., in opposite orientations) with nucleotide bases of the reference genome. Alignment position information derived from the read pair $R_1$ and $R_2$ may include a beginning position in the reference genome that corresponds to an end of a first read (e.g., $R_1$) and an end position in the reference genome that corresponds to an end of a second read (e.g., $R_2$). In other words, the beginning position and end position in the reference genome represent the likely location within the reference genome to which the nucleic acid fragment corresponds. An output file having SAM (sequence alignment map) format or BAM (binary alignment map) format may be generated and output for further analysis.

From the sequence reads, the location and methylation state for each of CpG site may be determined based on alignment to a reference genome. Further, a methylation state vector for each fragment may be generated specifying a location of the fragment in the reference genome (e.g., as specified by the position of the first CpG site in each fragment, or another similar metric), a number of CpG sites in the fragment, and the methylation state of each CpG site in the fragment whether methylated (e.g., denoted as M), unmethylated (e.g., denoted as U), or indeterminate (e.g., denoted as I). The methylation state vectors may be stored in temporary or persistent computer memory for later use and processing. Further, duplicate reads or duplicate methylation state vectors from a single subject may be removed. In an additional embodiment, it may be determined that a certain fragment has one or more CpG sites that have an indeterminate methylation status. Such fragments may be excluded from later processing or selectively included where downstream data model accounts for such indeterminate methylation statuses.

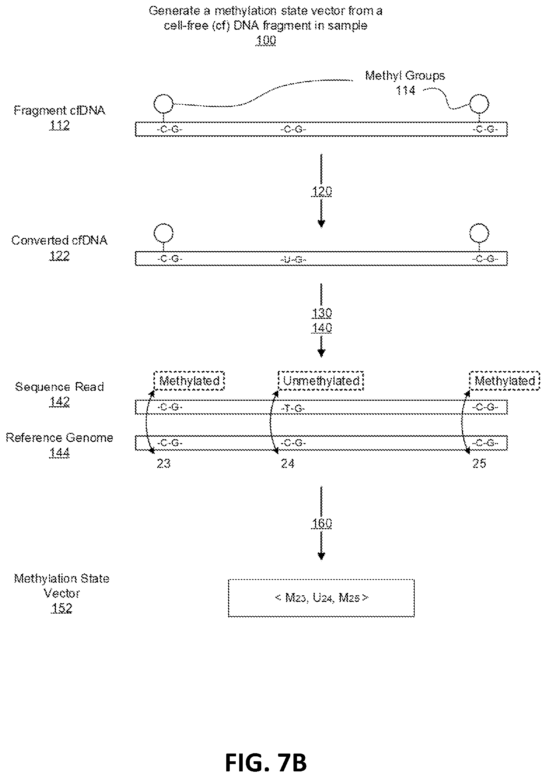

FIG. 7B is an illustration of the process 100 of FIG. 7A of sequencing a cfDNA fragment to obtain a methylation state vector, according to an embodiment. As an example, the analytics system takes a cfDNA fragment 112. In this example, the cfDNA fragment 112 contains three CpG sites. As shown, the first and third CpG sites of the cfDNA fragment 112 are methylated 114. During the treatment step 120, the cfDNA fragment 112 is converted to generate a converted cfDNA fragment 122. During the treatment 120, the second CpG site which was unmethylated has its cytosine converted to uracil. However, the first and third CpG sites are not convert.

After conversion, a sequencing library 130 is prepared and sequenced 140 generating a sequence read 142. The analytics system aligns 150 the sequence read 142 to a reference genome 144. The reference genome 144 provides the context as to what position in a human genome the fragment cfDNA originates from. In this simplified example, the analytics system aligns 150 the sequence read such that the three CpG sites correlate to CpG sites 23, 24, and 25 (arbitrary reference identifiers used for convenience of description). The analytics system thus generates information both on methylation status of all CpG sites on the cfDNA fragment 112 and which to position in the human genome the CpG sites map. As shown, the CpG sites on sequence read 142 which were methylated are read as cytosines. In this example, the cytosine's appear in the sequence read 142 only in the first and third CpG site which allows one to infer that the first and third CpG sites in the original cfDNA fragment were methylated. The second CpG site is read as a thymine (U is converted to T during the sequencing process), and thus, one can infer that the second CpG site was unmethylated in the original cfDNA fragment. With these two pieces of information, the methylation status and location, the analytics system generates 160 a methylation state vector 152 for the fragment cfDNA 112. In this example, the resulting methylation state vector 152 is < $M_{23}$, $U_{24}$, $M_{25}$ >, wherein M corresponds to a methylated CpG site, U corresponds to an unmethylated CpG site, and the subscript numbers correspond to positions of each CpG site in the reference genome.

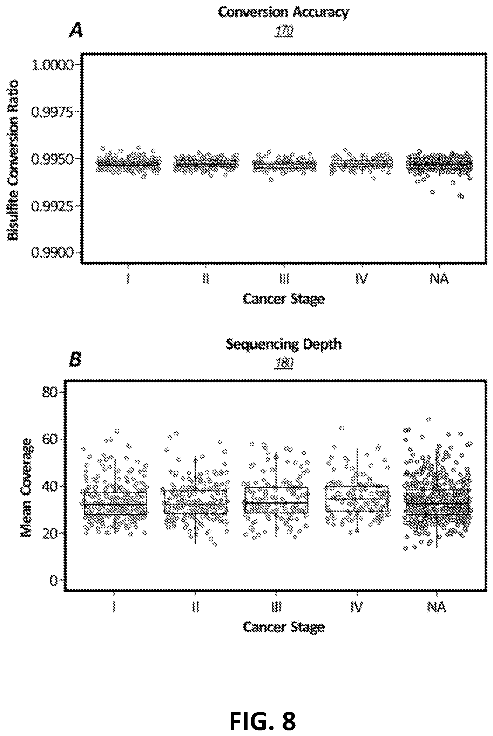

**FIGS. 8A-8B** show three graphs of data validating consistency of sequencing from a control group. The first graph 170 shows conversion accuracy of conversion of unmethylated cytosines to uracil (step 120) on cfDNA fragment obtained from a test sample across subjects in varying stages of cancer - stage 0, stage I, stage II, stage III, stage IV, and non-cancer. As shown, there was uniform consistency in converting unmethylated cytosines on cfDNA fragments into uracils. There was an overall conversion accuracy of 99.47% with a precision at ± 0.024%. The second graph 180 compares coverage (depth of sequencing) over varying stages of cancer. Counting only sequence reads that were confidently mapped to a reference genome, the mean coverage over all groups was ~ 34. The third graph 190 shows the concentration of cfDNA per sample across varying stages of cancer.

*Detection of cancer*

Sequence reads obtained by the methods provided herein are further processed by automated algorithms. For example, the analytics system is used to receive sequencing data from a sequencer and perform various aspects of processing as described herein. The analytics system can be one of a personal computer (PC), a desktop computer, a laptop computer, a notebook, a tablet PC, a mobile device. A computing device can be communicatively coupled to the sequencer through a wireless, wired, or a combination of wireless and wired communication technologies. Generally, the computing device is configured with a processor and memory storing computer instructions that, when executed by the processor, cause the processor to perform steps as described in the remainder of this document. Generally, the amount of genetic data and data derived therefrom is sufficiently large, and the amount of computational power required so great, so as to be impossible to be performed on paper or by the human mind alone.

The clinical interpretation of methylation status of targeted genomic regions is a process that includes classifying the clinical effect of each or a combination of the methylation status and reporting the results in ways that are meaningful to a medical professional. The clinical interpretation can be based on comparison of the sequence reads with database specific to cancer or non-cancer subjects, and/or based on numbers and types of the cfDNA fragments having cancer-specific methylation patterns identified from a sample. **In** some embodiments, targeted genomic regions are ranked or classified based on their likeness to be differentially methylated in cancer samples, and the ranks or classifications are used in the interpretation process. The ranks and classifications can include (1) the type of clinical effect, (2) the strength of evidence of the effect, and (3) the size of the effect. Various methods for clinical analysis and interpretation of genome data can be adopted for analysis of the sequence reads. **In** some other embodiments, the clinical interpretation of the methylation states of such differentially methylated regions can be based on machine learning approaches that interpret a current sample based on a classification or regression method that was trained using the methylation states of such differentially methylated regions from samples from cancer and non-cancer patients with known cancer status, cancer type, cancer stage, TOO, etc.

The clinically meaning information can include the presence or absence of cancer generally, presence or absence of certain types of cancers, cancer stage, or presence or absence of other types of diseases. **In** some embodiments, the information relates to a presence or absence of one or more cancer types, selected from the group consisting of breast cancer, endometrial cancer, cervical cancer, ovarian cancer, bladder cancer, urothelial cancer of renal pelvis, renal cell carcinoma, prostate cancer, anorectal cancer, anal cancer, colorectal cancer, hepatocellular cancer, liver/bile-duct cancer, cholangiocarcinoma and hepatobiliary cancer, pancreatic cancer, upper **GI** adenocarcinoma, esophageal squamous cell cancer, head and neck cancer, lung cancer, squamous cell lung cancer, lung adenocarcinoma, small cell lung cancer, neuroendocrine cancer, melanoma, thyroid cancer, sarcoma, plasma cell neoplasm, multiple myeloma, myeloid neoplasm, lymphoma, and leukemia. **In** some embodiments, the information relates to a presence or absence of one or more cancer types, selected from the group consisting of uterine cancer, upper **GI** squamous cancer, all other upper **GI** cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma (hcc), hepatobiliary biliary, head and neck cancer, colorectal cancer, cervical cancer, breast cancer, bladder cancer, and anorectal cancer. **In** some embodiments, the information relates to a presence or absence of one or more cancer types, selected from the group consisting of anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer. **In** some embodiments, the information relates to a presence or absence of one or more cancer types, selected from the group consisting of thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper **GI** cancer, multiple myeloma, lymphoid neoplasm, and lung cancer. In some embodiments, the samples are not cancerous and are from subjects having white blood cell clonal expansion or no cancer.

*Cancer classifier*

In some examples, the assay panel described herein can be used with a cancer type classifier that predicts a disease state for a sample, such as a cancer or non-cancer prediction, a tissue of origin prediction, and/or an indeterminate prediction. In some examples, the cancer type classifier can generate features based on sequence reads by taking into account methylated or unmethylated fragments of DNA at certain genomic areas of interest. For instance, if the cancer type classifier determines that a methylation pattern at a fragment resembles that of a certain cancer type, then the cancer type classifier can set a feature for that fragment as 1, and otherwise if no such fragment is present, then the feature can be set as 0. In this way, the cancer type classifier can produce a set of binary features (merely by way of example, 30,000 features) for each sample. Further, in some examples, all or a portion of the set of binary features for a sample can be input into the cancer type classifier to provide a set of probability scores, such as one probability score per cancer type class and for a non-cancer type class. Furthermore, in some examples, the cancer type classifier can incorporate or otherwise be used in conjunction with thresholding to determine whether a sample is to be called as cancer or non-cancer, and/or indeterminate thresholding to reflect confidence in a specific TOO call. Such methods are described further below.

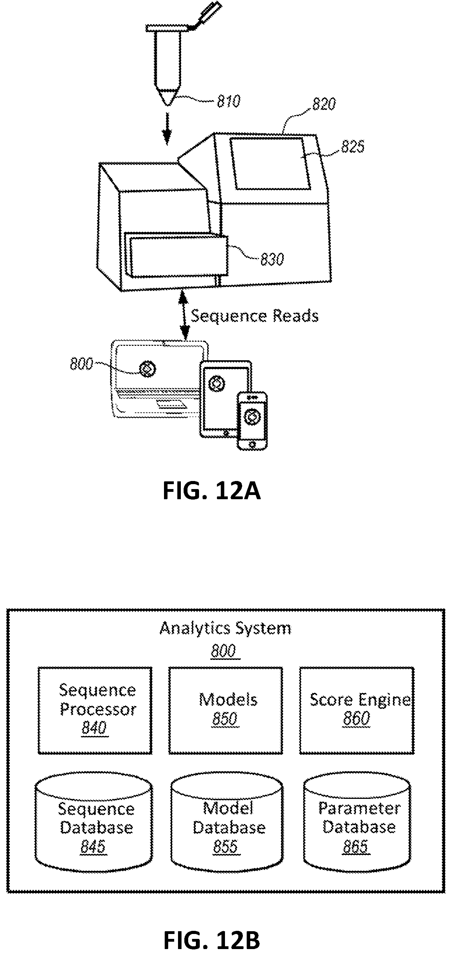

To train the cancer type classifier, the analytics system (e.g., analytics system 800, FIG. 12B) can obtain a set of training samples. In some examples, each training sample includes fragment file(s) (e.g., file containing sequence read data), a label corresponding to a type of cancer (TOO) or non-cancer status of the sample, and/or sex of the individual of the sample. The analytics system can utilize the training set to train the cancer type classifier to predict the disease state of the sample.

In some examples, for training, the analytics system divides the genome (e.g., whole genome) or a subset of the genome (e.g., targeted methylation regions) into regions. Merely by way of example, portions of the genome can be separated into "blocks" of CpGs, whereby a new block begins whenever there is a separation between nearest-neighbor CpGs is at least a minimum separation distance (e.g., at least 500 bp). Further, in some examples, each block can be divided into 1000 bp regions and positioned such that neighboring regions have a certain amount (e.g., 50% or 500 bp) of overlap.

Furthermore, in some examples, the analytics system can split the training set into K subsets or folds to be used in a K-fold cross-validation. In some examples, the folds can be balanced for cancer/non-cancer status, tissue of origin, cancer stage, age (e.g., grouped in 10yr buckets), and/or smoking status. In some examples, the training set is split into 5 folds, whereby 5 separate classifiers are trained, in each case training on 4/5 of the training samples and using the remaining 1/5 for validation.

During training with the training set, the analytics system can, for each cancer type (and for healthy cfDNA), fit a probabilistic model to the fragments deriving from the samples of that type. As used herein a "probabilistic model" is any mathematical model capable of assigning a probability to a sequence read based on methylation status at one or more sites on the read. During training, the analytics system fits sequence reads derived from one or more samples from subjects having a known disease and can be used to determine sequence reads probabilities indicative of a disease state utilizing methylation information or methylation state vectors. In particular, in some cases, the analytics system determines observed rates of methylation for each CpG site within a sequence read. The rate of methylation represents a fraction or percentage of base pairs that are methylated within a CpG site. The trained probabilistic model can be parameterized by products of the rates of methylation. In general, any known probabilistic model for assigning probabilities to sequence reads from a sample can be used. For example, the probabilistic model can be a binomial model, in which every site (e.g., CpG site) on a nucleic acid fragment is assigned a probability of methylation, or an independent sites model, in which each CpG's methylation is specified by a distinct methylation probability with methylation at one site assumed to be independent of methylation at one or more other sites on the nucleic acid fragment.

In some examples, the probabilistic model is a Markov model, in which the probability of methylation at each CpG site is dependent on the methylation state at some number of preceding CpG sites in the sequence read, or nucleic acid molecule from which the sequence read is derived. See, e.g., U.S. Pat. Appl. No. 16/352,602, entitled "Anomalous Fragment Detection and Classification," and filed March 13, 2019, which is incorporated by reference in its entirety herein and can be used for various embodiments.

In some examples, the probabilistic model is a "mixture model" fitted using a mixture of components from underlying models. For example, in some embodiments, the mixture components can be determined using multiple independent sites models, where methylation (e.g., rates of methylation) at each CpG site is assumed to be independent of methylation at other CpG sites. Utilizing an independent sites model, the probability assigned to a sequence read, or the nucleic acid molecule from which it derives, is the product of the methylation probability at each CpG site where the sequence read is methylated and one minus the methylation probability at each CpG site where the sequence read is unmethylated. In accordance with this example, the analytics system determines rates of methylation of each of the mixture components. The mixture model is parameterized by a sum of the mixture components each associated with a product of the rates of methylation. A probabilistic model *Pr* of *n* mixture components can be represented as:

$$Pr(fragment|\{\beta_{ki}, f_k\}) = \sum_{k=1}^{n} f_k \prod_i \beta_{ki}^{m_i} (1 - \beta_{ki})^{1-m_i}$$

For an input fragment, $m_i \in \{0, 1\}$ represents the fragment's observed methylation status at position $i$ of a reference genome, with 0 indicating unmethylation and 1 indicating methylation. A fractional assignment to each mixture component k is $f_k$, where $f_k \geq 0$ and $\sum_{k=1}^{n} f_k = 1$. The probability of methylation at position $i$ in a CpG site of mixture component $k$ is $\beta_{ki}$. Thus, the probability of unmethylation is $1 - \beta_{ki}$. The number of mixture components n can be 1, 2, 3, 4, 5, 6, 7, 8, 9, 10, etc.

In some examples, the analytics system fits the probabilistic model using maximum-likelihood estimation to identify a set of parameters $\{\beta_{ki}, f_k\}$ that maximizes the log-likelihood of all fragments deriving from a disease state, subject to a regularization penalty applied to each methylation probability with regularization strength r. The maximized quantity for N total fragments can be represented as:

$$\sum_{j}^{N} ln \left( Pr \left( fragment_j | \{\beta_{ki}, f_k\} \right) \right) + r \cdot ln \left( \beta_{ki} (1 - \beta_{ki}) \right)$$

In some examples, the analytics system performs fits separately for each cancer type and for healthy cfDNA. As one of skill in the art would appreciate, other means can be used to fit the probabilistic models or to identify parameters that maximize the log-likelihood of all sequence reads derived from the reference samples. For example, in some examples, Bayesian fitting (using e.g., Markov chain Monte Carlo), in which each parameter is not assigned a single value but instead is associated to a distribution, is used. In some examples, gradient-based optimization, in which the gradient of the likelihood (or log-likelihood) with respect to the parameter values is used to step through parameter space towards an optimum, is used. In still some examples, expectation-maximization, in which a set of latent parameters (such as identities of the mixture component from which each fragment is derived) are set to their expected values under the previous model parameters, and then the model's parameters are assigned to maximize the likelihood conditional on the assumed values of those latent variables. The two-step process is then repeated until convergence.

Further, in some examples, the analytics system can generate features for each sample in the training set. For example, for each sample (regardless of label), in each region, for each cancer type, for each fragment, the analytics system can evaluate the log-likelihood ratio R with the fitted probabilistic models according to:

$$R_{cancer\ type\ A} (fragment) \equiv ln \left( \frac{Pr (fragment|cancer\ type\ A)}{Pr (fragment|healthy\ cfDNA)} \right)$$

Next, for each sample, for each region, for each cancer type, for each of a set of "tier" values, the analytics system can count the number of fragments with Rcancer type > tier and assign those counts as non-negative integer-valued features. For example, the tiers include threshold values of 1, 2, 3, 4, 5, 6, 7, 8, and 9, resulting in each region hosting 9 features per cancer type.

In some examples, the analytics system can select certain features for inclusion in a feature vector for each sample. For example, for each pair of distinct cancer types, the analytics system can specify one type as the "positive type" and the other as the "negative type" and rank the features by their ability to distinguish those types. In some cases, the ranking is based on mutual information calculated by the analytics system. For example, the mutual information can be calculated using the estimated fraction of samples of the positive type and negative type (e.g., cancer types A and B) for which the feature is expected to be nonzero in a resulting assay. For instance, if a feature occurs frequently in healthy cfDNA, the analytics system determines the feature is unlikely to occur frequently in cfDNA associated with various types of cancer. Consequently, the feature can be a weak measure in distinguishing between disease states. In calculating mutual information *I*, the variable *X* is a certain feature (e.g., binary) and variable Y represents a disease state, e.g., cancer type A or B:

$$I(X;Y) = \sum_{y \in Y} \sum_{x \in X} p(x,y) \log \log \left( \frac{p(x,y)}{p(x)p(y)} \right)$$

$$I \approx \frac{1}{2} \left( p(1|A) \cdot \log \left( \frac{p(1|A)}{\frac{1}{2}\left(p(1|A) + p(1|B)\right)} \right) + p(1|B) \cdot \log \left( \frac{p(1|B)}{\frac{1}{2}\left(p(1|A)p(1|B)\right)} \right) \right)$$

$$p(1|A) = f_A + f_H - f_H f_A$$

The joint probability mass function of *X* and *Y* is *p*(*x, y*) and the marginal probability mass functions are *p*(*x*) and *p*(*y*). The analytics system can assume that feature absence is uninformative and either disease state is equally likely *a priori,* for example, *p*(*Y* = *A*) = *p*(*Y* = *B*) = 0.5. The probability of observing (e.g., in cfDNA) a given binary feature of cancer type A is represented by *p*(1|*A*), where $f_A$ is the probability of observing the feature in ctDNA samples from tumor (or high-signal cfDNA samples) associated with cancer type A, and $f_H$ is the probability of observing the feature in a healthy or non-cancer cfDNA sample.

**In** some examples, only features corresponding to the positive type are included in the ranking, and only when those features' predicted rate of occurrence is greater in the positive type than in the negative type. For example, if "liver" is the positive type and "breast" is the negative type, then only "liver_x" features are considered, and only if their estimated occurrence in liver cfDNA is greater than their estimated occurrence in breast cfDNA. Further, in some examples, for each region, for each cancer type pair (including non-cancer as a negative type), the analytics system keeps only the best performing tier. Further, in some examples, the analytics system transforms feature values by binarization, whereby any feature value greater than 0 is set to 1, such that all features are either 0 or 1.

In some examples, the analytics system trains a multinomial logistic regression classifier on the training data for a fold, and generates predictions for the held-out data. For example, for each of the K folds, one logistic regression can be trained for each combination of hyperparameters. Such hyperparameters can include L2 penalty and/or topK (e.g., the number of high-ranking regions to keep per tissue type pair (including non-cancer), as ranked by the mutual information procedure outlined above). For each set of hyperparameters, performance is evaluated on the cross-validated predictions of the full training set, and the set of hyperparameters with the best performance is selected for retraining on the full training set. In some examples, the analytics system uses log-loss as a performance metric, whereby the log-loss is calculated by taking the negative logarithm of the prediction for the correct label for each sample, and then summing over samples (i.e. a perfect prediction of 1.0 for the correct label would give a log-loss of 0).

To generate predictions for a new sample, feature values are calculated using the same method described above, but restricted to features (region/positive class combinations) selected under the chosen topK value. Generated features are then used to create a prediction using the logistic regression model trained above.

In some examples, the analytics trains a two-stage classifier. For example, the analytics system trains a binary cancer classifier to distinguish between the labels, cancer and non-cancer, based on the feature vectors of the training samples. In this case, the binary classifier outputs a prediction score indicating the likelihood of the presence or absence of cancer. In another example, the analytics system trains a multiclass cancer classifier to distinguish between many cancer types. In this multiclass cancer classifier, the cancer classifier is trained to determine a cancer prediction that comprises a prediction value for each of the cancer types being classified for. The prediction values can correspond to a likelihood that a given sample has each of the cancer types. For example, the cancer classifier returns a cancer prediction including a prediction value for breast cancer, lung cancer, and non-cancer. For example, the cancer classifier may return a cancer prediction for a test sample including a prediction score for breast cancer, lung cancer, and/or no cancer.

The analytics system can train the cancer classifier according to any one of a number of methods. As an example, the binary cancer classifier may be a L2-regularized logistic regression classifier that is trained using a log-loss function. As another example, the multi-cancer (TOO) classifier may be a multinomial logistic regression. In practice either type of cancer classifier may be trained using other techniques. These techniques are numerous including potential use of kernel methods, machine learning algorithms such as multilayer neural networks, etc. In particular, methods as described in PCT/US2019/022122 and U.S. Patent. App. No. 16/352,602 which are incorporated by reference in their entireties herein can be used for various embodiments. Still further, in some examples, the TOO classifier is trained only on cancer samples that were successfully called as cancer by the binary classifier, thereby ensuring sufficient cancer signal in the cancer sample. On the other hand, in some examples, the binary classifier is trained on the training samples regardless of TOO.

*Exemplary sequencer and analytics system*

FIG. 12A is a flowchart of systems and devices for sequencing nucleic acid samples according to one embodiment. This illustrative flowchart includes devices such as a sequencer 820 and an analytics system 800. The sequencer 820 and the analytics system 800 may work in tandem to perform one or more steps in the processes described herein.

In various embodiments, the sequencer 820 receives an enriched nucleic acid sample 810. As shown in FIG. 12A, the sequencer 820 can include a graphical user interface 825 that enables user interactions with particular tasks (e.g., initiate sequencing or terminate sequencing) as well as one more loading stations 830 for loading a sequencing cartridge including the enriched fragment samples and/or for loading necessary buffers for performing the sequencing assays. Therefore, once a user of the sequencer 820 has provided the necessary reagents and sequencing cartridge to the loading station 830 of the sequencer 820, the user can initiate sequencing by interacting with the graphical user interface 825 of the sequencer 820. Once initiated, the sequencer 820 performs the sequencing and outputs the sequence reads of the enriched fragments from the nucleic acid sample 810.

In some embodiments, the sequencer 820 is communicatively coupled with the analytics system 800. The analytics system 800 includes some number of computing devices used for processing the sequence reads for various applications such as assessing methylation status at one or more CpG sites, variant calling or quality control. The sequencer 820 may provide the sequence reads in a BAM file format to the analytics system 800. The analytics system 800 can be communicatively coupled to the sequencer 820 through a wireless, wired, or a combination of wireless and wired communication technologies. Generally, the analytics system 800 is configured with a processor and non-transitory computer-readable storage medium storing computer instructions that, when executed by the processor, cause the processor to process the sequence reads or to perform one or more steps of any of the methods or processes disclosed herein.

In some embodiments, the sequence reads may be aligned to a reference genome using known methods in the art to determine alignment position information. Alignment position may generally describe a beginning position and an end position of a region in the reference genome that corresponds to a beginning nucleotide based and an end nucleotide base of a given sequence read. Corresponding to methylation sequencing, the alignment position information may be generalized to indicate a first CpG site and a last CpG site included in the sequence read according to the alignment to the reference genome. The alignment position information may further indicate methylation statuses and locations of all CpG sites in a given sequence read. A region in the reference genome may be associated with a gene or a segment of a gene; as such, the analytics system 800 may label a sequence read with one or more genes that align to the sequence read. In one embodiment, fragment length (or size) is determined from the beginning and end positions.

In various embodiments, for example when a paired-end sequencing process is used, a sequence read is comprised of a read pair denoted as R_1 and R_2. For example, the first read R_1 may be sequenced from a first end of a double-stranded DNA (dsDNA) molecule whereas the second read R_2 may be sequenced from the second end of the double-stranded DNA (dsDNA). Therefore, nucleotide base pairs of the first read R_1 and second read R_2 may be aligned consistently (e.g., in opposite orientations) with nucleotide bases of the reference genome. Alignment position information derived from the read pair R_1 and R_2 may include a beginning position in the reference genome that corresponds to an end of a first read (e.g., R_1) and an end position in the reference genome that corresponds to an end of a second read (e.g., R_2). In other words, the beginning position and end position in the reference genome represent the likely location within the reference genome to which the nucleic acid fragment corresponds. In one embodiment, the read pair R_1 and R_2 can be assembled into a fragment, and the fragment used for subsequent analysis and/or classification. An output file having SAM (sequence alignment map) format or BAM (binary) format may be generated and output for further analysis.

Referring now to **FIG. 12B, FIG. 12B** is a block diagram of an analytics system 800 for processing DNA samples according to one embodiment. The analytics system implements one or more computing devices for use in analyzing DNA samples. The analytics system 800 includes a sequence processor 840, sequence database 845, model database 855, models 850, parameter database 865, and score engine 860. In some embodiments, the analytics system 800 performs one or more steps in the processes 300 of **FIG. 3A,** 340 of **FIG. 3B,** 400 of **FIG. 4,** 500 of **FIG. 5,** 600 of **FIG. 6A,** or 680 of **FIG. 6B** and other process described herein.

The sequence processor 840 generates methylation state vectors for fragments from a sample. At each CpG site on a fragment, the sequence processor 840 generates a methylation state vector for each fragment specifying a location of the fragment in the reference genome, a number of CpG sites in the fragment, and the methylation state of each CpG site in the fragment whether methylated, unmethylated, or indeterminate via the process 300 of **FIG. 3A.** The sequence processor 840 may store methylation state vectors for fragments in the sequence database 845. Data in the sequence database 845 may be organized such that the methylation state vectors from a sample are associated to one another.

Further, multiple different models 850 may be stored in the model database 855 or retrieved for use with test samples. In one example, a model is a trained cancer classifier for determining a cancer prediction for a test sample using a feature vector derived from anomalous fragments. The training and use of the cancer classifier is discussed elsewhere herein. The analytics system 800 may train the one or more models 850 and store various trained parameters in the parameter database 865. The analytics system 800 stores the models 850 along with functions in the model database 855.

During inference, the score engine 860 uses the one or more models 850 to return outputs. The score engine 860 accesses the models 850 in the model database 855 along with trained parameters from the parameter database 865. According to each model, the score engine receives an appropriate input for the model and calculates an output based on the received input, the parameters, and a function of each model relating the input and the output. In some use cases, the score engine 860 further calculates metrics correlating to a confidence in the calculated outputs from the model. In other use cases, the score engine 860 calculates other intermediary values for use in the model.

*Cancer and treatment monitoring*

In certain embodiments, the first time point is before a cancer treatment (e.g., before a resection surgery or a therapeutic intervention), and the second time point is after a cancer treatment (e.g., after a resection surgery or therapeutic intervention), and the method utilized to monitor the effectiveness of the treatment. For example, if the second likelihood or probability score decreases compared to the first likelihood or probability score, then the treatment is considered to have been successful. However, if the second likelihood or probability score increases compared to the first likelihood or probability score, then the treatment is considered to have not been successful. In other embodiments, both the first and second time points are before a cancer treatment (e.g., before a resection surgery or a therapeutic intervention). In still other embodiments, both the first and the second time points are after a cancer treatment (e.g., before a resection surgery or a therapeutic intervention) and the method is used to monitor the effectiveness of the treatment or loss of effectiveness of the treatment. In still other embodiments, cfDNA samples may be obtained from a cancer patient at a first and second time point and analyzed. e.g., to monitor cancer progression, to determine if a cancer is in remission (e.g., after treatment), to monitor or detect residual disease or recurrence of disease, or to monitor treatment (e.g., therapeutic) efficacy.

Those of skill in the art will readily appreciate that test samples can be obtained from a cancer patient over any desired set of time points and analyzed in accordance with the methods of the invention to monitor a cancer state in the patient. In some embodiments, the first and second time points are separated by an amount of time that ranges from about 15 minutes up to about 30 years, such as about 30 minutes, such as about 1, 2, 3, 4, 5, 6, 7, 8, 9, 10, 11, 12, 13, 14, 15, 16, 17, 18, 19, 20, 21, 22, 23, or about 24 hours, such as about 1, 2, 3, 4, 5, 10, 15, 20, 25 or about 30 days, or such as about 1, 2, 3, 4, 5, 6, 7, 8, 9, 10, 11, or 12 months, or such as about 1, 1.5, 2, 2.5, 3, 3.5, 4, 4.5, 5, 5.5, 6, 6.5, 7, 7.5, 8, 8.5, 9, 9.5, 10, 10.5, 11, 11.5, 12, 12.5, 13, 13.5, 14, 14.5, 15, 15.5, 16, 16.5, 17, 17.5, 18, 18.5, 19, 19.5, 20, 20.5, 21, 21.5, 22, 22.5, 23, 23.5, 24, 24.5, 25, 25.5, 26, 26.5, 27, 27.5, 28, 28.5, 29, 29.5 or about 30 years. In other embodiments, test samples can be obtained from the patient at least once every 3 months, at least once every 6 months, at least once a year, at least once every 2 years, at least once every 3 years, at least once every 4 years, or at least once every 5 years.

*Treatment*

In still another embodiment, information obtained from any method described herein (e.g., the likelihood or probability score) can be used to make or influence a clinical decision (e.g., diagnosis of cancer, treatment selection, assessment of treatment effectiveness, etc.). For example, in one embodiment, if the likelihood or probability score exceeds a threshold, a physician can prescribe an appropriate treatment (e.g., a resection surgery, radiation therapy, chemotherapy, and/or immunotherapy). In some embodiments, information such as a likelihood or probability score can be provided as a readout to a physician or subject.

A classifier (as described herein) can be used to determine a likelihood or probability score that a sample feature vector is from a subject that has cancer. In one embodiment, an appropriate treatment (e.g., resection surgery or therapeutic) is prescribed when the likelihood or probability exceeds a threshold. For example, in one embodiment, if the likelihood or probability score is greater than or equal to 60, one or more appropriate treatments are prescribed. In another embodiments, if the likelihood or probability score is greater than or equal to 65, greater than or equal to 70, greater than or equal to 75, greater than or equal to 80, greater than or equal to 85, greater than or equal to 90, or greater than or equal to 95, one or more appropriate treatments are prescribed. In other embodiments, a cancer log-odds ratio can indicate the effectiveness of a cancer treatment. For example, an increase in the cancer log-odds ratio over time (e.g., at a second, after treatment) can indicate that the treatment was not effective. Similarly, a decrease in the cancer log-odds ratio over time (e.g., at a second, after treatment) can indicate successful treatment. In another embodiment, if the cancer log-odds ratio is greater than 1, greater than 1.5, greater than 2, greater than 2.5, greater than 3, greater than 3.5, or greater than 4, one or more appropriate treatments are prescribed.

In some embodiments, the treatment is one or more cancer therapeutic agents selected from the group consisting of a chemotherapy agent, a targeted cancer therapy agent, a differentiating therapy agent, a hormone therapy agent, and an immunotherapy agent. For example, the treatment can be one or more chemotherapy agents selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, platinum-based agents and any combination thereof. In some embodiments, the treatment is one or more targeted cancer therapy agents selected from the group consisting of signal transduction inhibitors (e.g. tyrosine kinase and growth factor receptor inhibitors), histone deacetylase (HDAC) inhibitors, retinoic receptor agonists, proteosome inhibitors, angiogenesis inhibitors, and monoclonal antibody conjugates. In some embodiments, the treatment is one or more differentiating therapy agents including retinoids, such as tretinoin, alitretinoin and bexarotene. In some embodiments, the treatment is one or more hormone therapy agents selected from the group consisting of anti-estrogens, aromatase inhibitors, progestins, estrogens, anti-androgens, and GnRH agonists or analogs. In one embodiment, the treatment is one or more immunotherapy agents selected from the group comprising monoclonal antibody therapies such as rituximab (RITUXAN) and alemtuzumab (CAMPATH), non-specific immunotherapies and adjuvants, such as BCG, interleukin-2 (IL-2), and interferon-alfa, immunomodulating drugs, for instance, thalidomide and lenalidomide (REVLIMID). It is within the capabilities of a skilled physician or oncologist to select an appropriate cancer therapeutic agent based on characteristics such as the type of tumor, cancer stage, previous exposure to cancer treatment or therapeutic agent, and other characteristics of the cancer.

## EXAMPLES

The following examples are put forth so as to provide those of ordinary skill in the art with a complete disclosure and description of how to make and use the present description, and are not intended to limit the scope of what the inventors regard as their description nor are they intended to represent that the experiments below are all or the only experiments performed. Efforts have been made to ensure accuracy with respect to numbers used (e.g., amounts, temperature, etc.) but some experimental errors and deviations should be accounted for.

### EXAMPLE 1 - Analysis of probe qualities

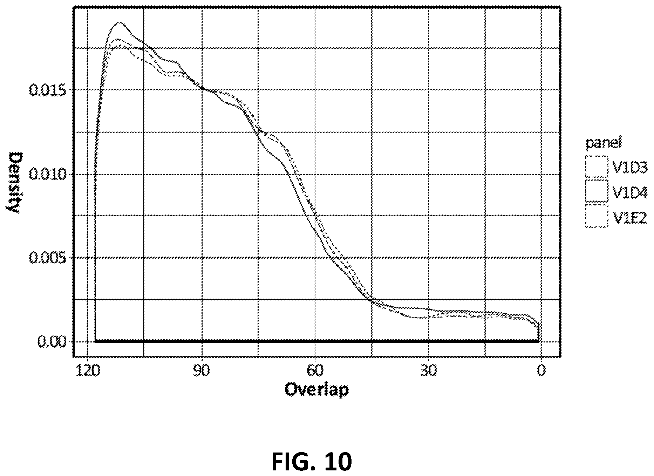

To test how much overlap between a cfDNA fragment and a probe is required to achieve a non-negligible amount of pulldown, various lengths of overlaps were tested using panels designed to include three different types of probes (V1D3, V1D4, V1E2) having various overlaps with 175bp target DNA fragments specific to each probe. Tested overlaps ranged between 0bp and 120bp. Samples comprising 175bp target DNA fragments were applied to the panel and washed, and then DNA fragments bound to the probes were collected. The amounts of the collected DNA fragments were measured and the amounts were plotted as densities over the sizes of overlaps as provided in **FIG. 10.**

There was no significant binding and pull down of target DNA fragments when there were less than 45 bp of overlaps. These results suggest that a fragment-probe overlap of at least 45bp is generally required to achieve a non-negligible amount of pulldown although this number can vary depending on the assay conditions.

Furthermore, it has been suggested that more than a 10% mismatch rate between the probe and fragment sequences in the region of overlap is sufficient to greatly disrupt binding, and thus pulldown efficiency. Therefore, sequences that can align to the probe along at least 45bp with at least a 90% match rate are candidates for off-target pulldown.

Thus, we have performed an exhaustive searching of all genomic regions having 45bp alignments with 90%+ match rate (i.e., off-target regions) for each probe. Specifically, we combined a k-mer seeding strategy (which can allow one or more mismatches) with local alignment at the seed locations. This guaranteed not missing any good alignments based on k-mer length, number of mismatches allowed, and number of k-mer seed hits at a particular location. This involves performing dynamic programing local alignment at a large number of locations, so the implementation was optimized to use vector CPU instructions (e.g., AVX2, AVX512) and parallelized across many cores within a machine and also across many machines connected by a network. This allows an exhaustive search which is valuable in designing a high-performance panel (i.e., low off-target rate and high target coverage for a given amount of sequencing).

Following the exhaustive searching, each probe was scored based on the number of off-target regions. The majority of probes have a score of 1, meaning they match in only one place. Probes with scores between 2-19 were accepted but probes with scores of more than 20 were discarded. Other cutoff values can be used for specific samples. Probes targeting hypermethylated regions tend to have significantly less off-target regions than probes targeting other regions.

**EXAMPLE 2 - Annotation of target genomic regions**

Target genomic regions identified by the process outlined in **FIG. 4** were analyzed to understand features of the target regions. Specifically, selected target genomic regions were aligned to a reference genome to determine alignment positions. The alignment position information was collected for each selected target genomic region, including the chromosome number, beginning nucleotide base, end nucleotide base, and the genomic annotations of the given genomic region. Target genomic regions were positioned in introns, exons, intergenic regions, 5'UTRs, 3'UTRs, or controlling regions such as promoters or enhancers. The number of target genomic regions that fall within each genomic annotation were counted and plotted in the graph provided in **FIG. 11**. **FIG. 11** also compares numbers of the selected target genomic regions (black bars) or numbers of randomly selected genomic regions (gray bars) that fall within each genomic annotation.

The analysis shows that the selected target genomic regions are not random in their genomic distributions and they had higher enrichment for regulatory and functional elements such as promoters and 5'UTRs and less representation of intergenic sequences in comparison with randomly selected targets of the same size. For example, target genomic regions were found to position in promoters, 5'UTR, exons, intron/exon boundaries, introns, 3'UTRs or enhancers, rather than intergenic regions.

**EXAMPLE 3 - Cancer assay panels for detecting cancer and cancer types**

<u>Samples used for genomic region selection:</u> DNA samples for this work came from various sources.

The Circulating Cell-free Genome Atlas Study ("CCGA"; Clinical Trial.gov identifier NCT02889978) is a prospective, multi-center, case-control, observational study with longitudinal follow-up. De-identified biospecimens were collected from approximately 15,000 participants from 142 sites. Samples were selected to ensure a prespecified distribution of cancer types and non-cancers across sites in each cohort, and cancer and non-cancer samples were frequency age-matched by gender.

The Cancer Genome Atlas ("TCGA"; Clinical Trial.gov identifier NCT02889978) is a public resource developed through a collaboration between the National Cancer Institute (NCI) and the National Human Genome Research Institute (NHGRI).

Dissociated tumor cells (DTC) were acquired from Conversant.

Non-cancer cells were provided by Yuval Dor and Ben Glaser (Hebrew University) and originated from human tissue obtained from standard clinical procedures. For example, breast luminal and basal epithelial cells were from breast reduction surgery; colon epithelial cells were from tissue near the site of re-implantation following segmental resection for localized colon pathology; bone marrow cells were from joint replacement surgery; vascular and arterial endothelial cells were from vascular surgery; and head and neck epithelium was from tonsillectomy.

WGBS was performed on more than 1000 genomic DNA samples collected from healthy individuals and individuals diagnosed with cancers of various stages and tissues of origin. The samples included formaldehyde-fixed, paraffin-embedded (FFPE) tissue blocks, disseminated tumor cells (DTC) from cancers of different TOOs, bone marrow mononuclear cells (BMMC), white blood cells (WBC) and peripheral blood mononuclear cells (PBMC). The DTCs were subjected to negative selection to remove WBCs, fibroblasts, and endothelial cells using a negative selection kit prior to gDNA isolation. The negative selection yielded purified tumor cells that allowed differentially methylated regions to be more clearly identified.

The TCGA data was collected by hybridization of bisulfite-converted DNA fragments from 8809 samples to methylation-sensitive oligonucleotide arrays. β-values from this study represent the relative abundance of methylation at 480,000 individual CpG sites. 75,000 of these CpG sites were analyzed after excluding CpGs from noisy genomic regions (360,000) and CpG sites with cross-hybridizing probes (45,000). The TCGA data was analyzed using different algorithms because it describes methylation of individual CpG sites, whereas WGBS data reveals the methylation pattern of strings of adjacent CpG sites on DNA fragments.

<u>Tissue of Origin classes:</u> Each sample was categorized into one of twenty-five (25) different Tissue of Origin (TOO) classes (i.e. Cancer Types): breast cancer, uterine cancer, cervical cancer, ovarian cancer, bladder cancer, urothelial cancer of renal pelvis, renal cancer other than urothelial, prostate cancer, anorectal cancer, colorectal cancer, hepatobiliary cancer arising from hepatocytes, hepatobiliary cancer arising from cells other than hepatocytes, pancreatic cancer, squamous cell cancer of the upper gastrointestinal tract, upper gastrointestinal cancer other than squamous, head and neck cancer, lung adenocarcinoma, small cell lung cancer, squamous cell lung cancer and cancer other than adenocarcinoma or small cell lung cancer, neuroendocrine cancer, melanoma, thyroid cancer, sarcoma, multiple myeloma, lymphoma, and leukemia. These TOO classes encompass 97% of the cancer incidence reported by the Surveillance, Epidemiology, and End Results program (SEER; seer.cancer.gov), after filtering out liquid, brain, small intestine, vagina & vulva and penis & testis. Rare incidence cancers like sarcoma, and neuroendocrine cancers were aggregated to guard against misclassification. International Classification of Diseases for Oncology (ICD-O-3) topographical, morphological, and behavioral codes and World Health Organization (WHO) topography designations were used to categorize individual samples into the TOO classes. For example, the 34 TCGA studies were mapped to 25 TOO classes as shown in **TABLE** 1. The TOO classification was iteratively refined against observed classification performance.

### TABLE 1 - Tissue of Origin (TOO) classification of TCGA types

| TOO class | TCGA type | N |
|---|---|---|
| Breast | BRCA | 779 |
| Renal | KIRC, KIRP, KICH | 657 |
| Brain | LLG, GBM | 654 |
| Upper GI | ESCA, STAD | 580 |
| Melanoma | SKCM, UVM | 550 |
| Head and neck | HNSC | 528 |
| Thyroid | THCA | 507 |
| Prostate | PRAD | 498 |
| Uterine | UCEC, UCS | 484 |
| Lung adenocarcinoma | LUAD | 444 |
| Bladder | BLCA | 409 |
| Colorectal | COAD, READ | 382 |
| Hepatobiliary carcinoma | LIHC | 377 |
| Lung squamous | LUSC | 370 |
| Cervical | CESC | 307 |
| Sarcoma | SARC | 261 |
| Adrenal | ACC, PCPG | 259 |
| Pancreas | PAAD | 184 |
| Leukemia | LAML, LCML | 140 |
| Testicular | TGCT | 134 |
| Thymus | THYM | 124 |
| Mesothelioma | MESO | 87 |
| Lymphoma | DLBC | 48 |
| Hepatobiliary biliary | CHOL | 36 |
| Ovarian | OV | 10 |

**Region selection:** For target selection, fragments having abnormal methylation patterns in cancer samples were selected using one or more methods as described herein. Use of these methods allowed the identification of low noise regions as putative targets. Among the low noise regions, fragments most informative in discriminating cancer types were ranked and selected.

Specifically, in some embodiments, when WGBS data were used, fragment sequences in the database were filtered based on p-value using a non-cancer distribution, and only fragments with p < 0.001 were retained, as described herein. In some cases, the selected cfDNAs were further filtered to retain only those that were at least 90% methylated or 90% unmethylated. Next, for each CpG site in the selected fragments, the numbers of cancer samples or non-cancer samples were counted that include fragments overlapping that CpG site. Specifically, P (cancer | overlapping fragment) for each CpG was calculated and genomic sites with high P values were selected as general cancer targets. By design, the selected fragments had very low noise (i.e., few non-cancer fragments overlapping).

To find cancer type specific targets, similar selection processes were performed. CpG sites were ranked based on their information gain, comparing (i) the numbers of samples of a specific TOO or other samples, including both non-cancer samples and samples of a different TOO, (ii) the numbers of samples of a specific TOO or non-cancer samples, and/or (iii) the numbers of samples of a specific TOO or a different TOO that include fragments overlapping that CpG site. The process was applied to each of the 25 TOOs and the comparison was done for all pairwise combinations for 25 TOOs. For example, P (cancer of a TOO | overlapping fragment) was calculated and then compared with P (cancer of a different TOO | overlapping fragment). An outlier fragment in each TOO having much greater likelihood under cancer of a TOO than under cancer of a different TOO was selected as a target for the TOO. Accordingly, genomic regions selected by the pairwise comparisons included genomic regions differentially methylated to separate a target TOO and a contrast TOO.

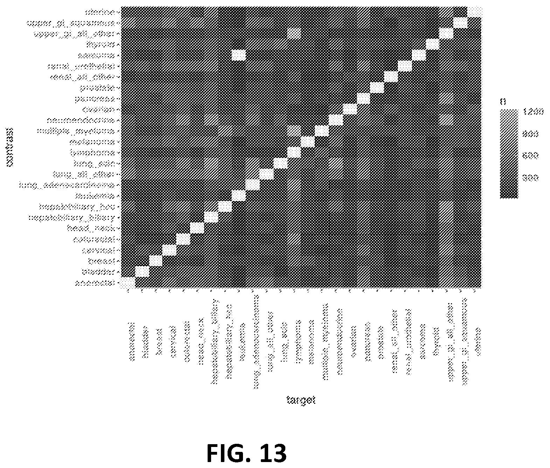

Additional target genomic regions were selected according to methods described in the section above titled "Computing pairwise information gain from fragments indicative of cancer identified from probabilistic models." The numbers of genomic regions for differentiating each target TOO (x-axis) from a contrast TOO (y-axis) are provided in FIG. 13.

When TCGA data were used, CpG beta values indicating intensity of methylation was used to identify target genomic regions. This is because array data are not at the CpG site levels, and thus they are prone to result in false positives. To avoid false positives, CpG sites were converted into 350 bp bins across the genome. Beta values of each bin were calculated as the mean of CpG beta values in that bin. Bins with less than 2 CpG's were excluded from the analysis. Next, bins were selected with beta difference of > 0.95 between (i) samples of a specific TOO and other samples, including both non-cancer samples and samples of a different TOO, (ii) samples of a specific TOO and non-cancer samples, and/or (iii) samples of a specific TOO and a different TOO that include fragments overlapping that CpG site.

Genomic regions selected as described above were then filtered based on the numbers of their off-target genomic regions as specified in 4.4.7. Specifically, numbers of genomic locations that have >=45bp alignments with >=90% identity were calculated as the numbers of off-target genomic regions. Genomic regions having off-target genomic regions more than 20 were discarded.

Various lists of target genomic regions selected as described in this section are identified in TABLE 2. These lists have different but overlapping sets of target genomic regions. They differ in their total numbers of target genomic regions, the total of the lengths of their target genomic regions, and the chromosomal locations of their target genomic regions. Lists 1-3 are small, medium, and large panels. The target genomic regions of lists 4-16 have subsets of the CpG methylation sites found in the target genomic regions of List 3. Lists 4, 6, and 8-16 were filtered to exclude previously known target genomic regions.

**TABLE 2 - SEQ ID NOs corresponding to Lists 1-16.** For each list, the table identifies the total number of target genomic regions in the list, a range of SEQ ID NOs corresponding to all target genomic regions in the list to be found in the sequence listing submitted with this application, and the total of the lengths of all target genomic regions in the list. The sequence listing identifies the chromosomal location of each target genomic region, whether cfDNA fragments to be enriched from the region are hypermethylated or hypomethylated, and the sequence of one DNA strand of the target genomic region. The chromosome numbers and the start and stop positions are provided relative to a known human reference genome, hg19. The sequence of the human reference genome, hg19, is available from Genome Reference Consortium with a reference number, GRCh37/hg19, and also available from Genome Browser provided by Santa Cruz Genomics Institute.

| List | Target Genomic Regions | SEQ ID NOs | | Panel Size (Mb) |
|---|---|---|---|---|
| | | First | Last | |
| 1 | 34844 | 1 | 34844 | 6.43 |
| 2 | 67431 | 34845 | 102275 | 12.14 |
| 3 | 94955 | 102276 | 197230 | 17.72 |
| 4 | 23941 | 197231 | 221171 | 4.63 |
| 5 | 56624 | 221172 | 277795 | 16.42 |
| 6 | 52850 | 277796 | 330645 | 10.45 |
| 7 | 14284 | 330646 | 344929 | 8.48 |
| 8 | 1370 | 344930 | 346299 | 0.39 |
| 9 | 2842 | 346300 | 349141 | 0.79 |
| 10 | 7483 | 349142 | 356624 | 1.94 |
| 11 | 12328 | 356625 | 368952 | 3.08 |
| 12 | 14725 | 368953 | 383677 | 3.65 |
| 13 | 3814 | 383678 | 387491 | 0.62 |

(continued)

| List | Target Genomic Regions | SEQ ID NOs | | Panel Size (Mb) |
|---|---|---|---|---|
| | | First | Last | |
| 14 | 7730 | 387492 | 395221 | 1.26 |
| 15 | 19424 | 395222 | 414645 | 3.23 |
| 16 | 38061 | 414646 | 452706 | 6.58 |

SEQ ID NOs 452,706 - 483,478 provide further information about certain hypermethylated or hypomethylated target genomic regions. These SEQ ID NO records identify target genomic regions that can be differentially methylated in samples from specified pairs of cancer types. The target genomic regions of SEQ ID NOs 452,706 - 483,478 are drawn from list 6. Many of the same target genomic regions are also found in lists 1-5 and 7-16. The entry for each SEQ ID indicates the chromosomal location of the target genomic region relative to hg19, whether cfDNA fragments to be enriched from the region are hypermethylated or hypomethylated, the sequence of one DNA strand of the target genomic region, and the pair or pairs of cancer types that are differentially methylated in that genomic region. As the methylation status of some target genomic regions distinguish more than one pair of cancer types, each entry identifies a first cancer type as indicated in TABLE 3 and one or more second cancer types.

**TABLE 3 - SEQ ID NOs identifying target genomic regions that are differentially methylated between pairs of cancer types**

| First Cancer Type | Target Genomic Regions | SEQ ID NOs | |
|---|---|---|---|
| | | First | Last |
| Anorectal | 1377 | 452707 | 454083 |
| Bladder & Urothelial | 1411 | 454084 | 455494 |
| Breast | 1748 | 455495 | 457242 |
| Cervical | 2011 | 457243 | 459253 |
| Colorectal | 1321 | 459254 | 460574 |
| Head & Neck | 1624 | 460575 | 462198 |
| Liver & Bile duct | 1810 | 462199 | 464008 |
| Lung | 1863 | 464009 | 465871 |
| Lymphoid Neoplasm | 2660 | 465872 | 468531 |
| Melanoma | 1378 | 468532 | 469909 |
| Multiple Myeloma | 986 | 469910 | 470895 |
| Myeloid Neoplasm | 1595 | 470896 | 472490 |
| Ovary | 1041 | 472491 | 473531 |
| Pancreas & Gallbladder | 1682 | 473532 | 475213 |
| Prostate | 1395 | 475214 | 476608 |
| Renal | 1236 | 476609 | 477844 |
| Sarcoma | 1418 | 477845 | 479262 |
| Thyroid | 895 | 479263 | 480157 |
| Upper GI | 1606 | 480158 | 481763 |
| Uterine | 1715 | 481764 | 483478 |

**Verification of selected genomic regions:**

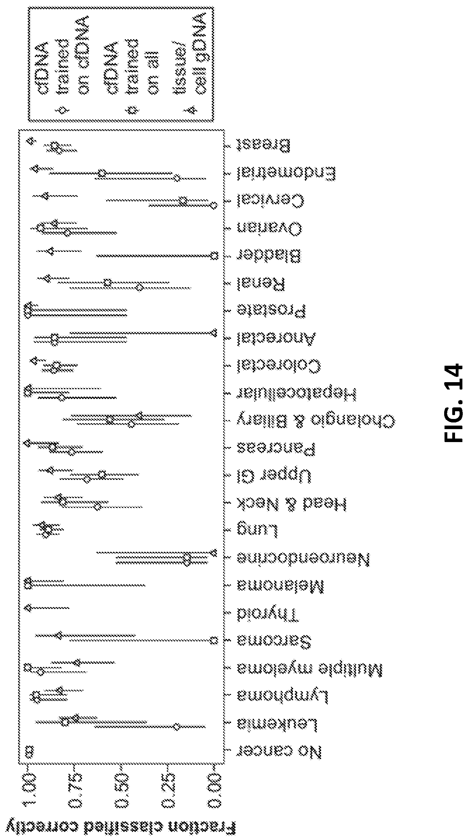

Some of the selected genomic regions were verified (1) without reference (using cfDNA from the CCGA1 30X WGBS database limited to cfDNA from samples with a log-likelihood ratio indicative of cancer greater than 0.9) or (2) with reference (using tissue and WBC samples). **FIG. 14** provides the verification results based on correctly-classified fractions. The results are from (1) verification done with cfDNA over genomic regions trained on cfDNA; (2) verification done with cfDNA over genomic regions trained on all different types of samples used herein; and (3) verification done with tissue and WBC gDNA sample over the selected genomic regions. The verification data is summarized in **TABLE 4,** additionally including data from verification done with all samples. The verification results demonstrate that genomic regions selected by the method described herein can provide information for detection of cancer and various cancer types.

### TABLE 4 - Verification data

| Cancer type | cfDNA trained on cfDNA | cfDNA trained on tissue | Tissue + other non cfDNA | All samples |
|---|---|---|---|---|
| Leukemia | 1/5 [20%] | 4/5 [80%] | 49/66 [74%] | 53/71 [75%] |
| Lymphoma | 22/23 [96%] | 22/23 [96%] | 39/47 [83%] | 61/70 [87%] |
| Multiple myeloma | 13/14 [93%] | 14/14 [100%] | 17/23 [74%] | 31/37 [84%] |
| Sarcoma | | 0/1 [0%] | 5/6 [83%] | 5/7 [71%] |
| Thyroid | | | 11/11 [100%] | 11/11 [100%] |
| Melanoma | | 2/2 [100%] | 13/13 [100%] | 15/15 [100%] |
| Neuroendocrine | 1/7 [14%] | 1/7 [14%] | 0/2 [0%] | 1/9 [11%] |
| Lung | 86/95 [91%] | 84/95 [88%] | 51/55 [93%] | 135/150 [90%] |
| Head & Neck | 10/16 [62%] | 13/16 [81%] | 36/43 [84%] | 49/59 [83%] |
| Upper GI | 17/25 [68%] | 15/25 [60%] | 43/49 [88%] | 58/74 [78%] |
| Pancreas | 23/30 [77%] | 26/30 [87%] | 15/15 [100%] | 41/45 [91%] |
| Cholangio & Biliary | 4/9 [44%] | 5/9 [56%] | 2/5 [40%] | 7/14 [50%] |
| Hepatocellular | 9/11 [82%] | 11/11 [100%] | 5/5 [100%] | 16/16 [100%] |
| Colorectal | 50/58 [86%] | 49/58 [84%] | 70/72 [97%] | 119/130 [92%] |
| Anorectal | 6/7 [86%] | 6/7 [86%] | 0/1 [0%] | 6/8 [75%] |
| Prostate | 3/3 [100%] | 3/3 [100%] | 58/58 [100%] | 61/61 [100%] |
| Renal | 2/5 [40%] | 4/7 [57%] | 50/56 [89%] | 54/63 [86%] |
| Bladder | | 0/2 [0%] | 28/32 [88%] | 28/34 [82%] |
| Ovarian | 11/14 [79%] | 13/14 [93%] | 43/50 [86%] | 56/64 [88%] |
| Cervical | 0/6 [0%] | 1/6 [17%] | 21/23 [91%] | 22/29 [76%] |
| Endometrial | 1/5 [20%] | 3/5 [60%] | 47/49 [96%] | 50/54 [93%] |
| Breast | 69/83 [83%] | 71/83 [86%] | 117/118 [99%] | 188/201 [94%] |
| **Total** | **328/416 [79%]** | **3457/423 [82%]** | **720/799 [90%]** | **1067/1222 [87%]** |

### EXAMPLE 4 - Generation of a mixture model classifier

To maximize performance, the predictive cancer models described in this Example were trained using sequence data obtained from a plurality of samples from known cancer types and non-cancers from both CCGA sub-studies (CCGA1 and CCGA22), a plurality of tissue samples for known cancers obtained from CCGA1, and a plurality of non-cancer samples from the STRIVE study (See Clinical Trail.gov Identifier: NCT03085888 (//clinicaltrials.gov/ct2/-show/NCT03085888)). The STRIVE study is a prospective, multi-center, observational cohort study to validate an assay for the early detection of breast cancer and other invasive cancers, from which additional non-cancer training samples were obtained to train the classifier described herein. The known cancer types included from the CCGA sample set included the following: breast, lung, prostate, colorectal, renal, uterine, pancreas, esophageal, lymphoma, head and neck, ovarian, hepatobiliary, melanoma, cervical, multiple myeloma, leukemia, thyroid, bladder, gastric, and anorectal. As such, a model can be a multi-cancer model (or a multi-cancer classifier) for detecting one or more, two or more, three or more, four or more, five or more, ten or more, or 20 or more different types of cancer.

The classifier performance data shown below was reported out for a locked classifier trained on cancer and non-cancer samples obtained from CCGA2, a CCGA sub-study, and on non-cancer samples from STRIVE. The individuals in the CCGA2 sub-study were different from the individuals in the CCGA1 sub-study whose cfDNA was used to select target genomes. From the CCGA2 study, blood samples were collected from individuals diagnosed with untreated cancer (including 20 tumor types and all stages of cancer) and healthy individuals with no cancer diagnosis (controls). For STRIVE, blood samples were collected from women within 28 days of their screening mammogram. Cell-free DNA (cfDNA) was extracted from each sample and treated with bisulfite to convert unmethylated cytosines to uracils. The bisulfite treated cfDNA was enriched for informative cfDNA molecules using hybridization probes designed to enrich bisulfite-converted nucleic acids derived from each of a plurality of targeted genomic regions in an assay panel comprising all of the genomic regions of Lists 1-16. The enriched bisulfite-converted nucleic acid molecules were sequenced using paired-end sequencing on an Illumina platform (San Diego, CA) to obtain a set of sequence reads for each of the training samples, and the resulting read pairs were aligned to the reference genome, assembled into fragments, and methylated and unmethylated CpG sites identified.

*Mixture model based featurization*

For each cancer type (including non-cancer) a probabilistic mixture model was trained and utilized to assign a probability to each fragment from each cancer and non-cancer sample based on how likely it was that the fragment would be observed in a given sample type.

*Fragment-level Analysis*

Briefly, for each sample type (cancer and non-cancer samples), for each region (where each region was used as-is if less than 1 kb, or else subdivided into 1 kb regions in length with a 50% overlap (e.g., 500 base pairs overlap) between adjacent regions), a probabilistic model was fit to the fragments derived from the training samples for each type of cancer and non-cancer. The probabilistic model trained for each sample type was a mixture model, where each of three mixture components was an independent-sites model in which methylation at each CpG is assumed to be independent of methylation at other CpGs. Fragments were excluded from the model if: they had a p-value (from a non-cancer Markov model) greater than 0.01; were marked as duplicate fragments; the fragments had a bag size of greater than 1 (for targeted methylation samples only); they did not cover at least one CpG site; or if the fragment was greater than 1000 bases in length. Retained training fragments were assigned to a region if they overlapped at least one CpG from that region. If a fragment overlapped CpGs in multiple regions, it was assigned to all of them.

*Local Source Models*

Each probabilistic model was fit using maximum-likelihood estimation to identify a set of parameters that maximized the log-likelihood of all fragments deriving from each sample type, subject to a regularization penalty.
Specifically, in each classification region, a set of probabilistic models were trained, one for each training label (i.e., one for each cancer type and one for non-cancer). Each model took the form of a Bernoulli mixture model with three components. Mathematically,

$$(1) \quad Pr\left(fragment | \{\beta_{ki}, f_k\}\right) = \sum_{k=1}^{n} f_k \prod_i \beta_{ki}^{m_i}(1 - \beta_{ki})^{1-m_i}$$

where n is the number of mixture components, set to 3; $m_i \in \{0, 1\}$ is the fragment's observed methylation at position $i$; $f_k$ is the fractional assignment to component $k$ (with $f_k \geq 0$ and $\Sigma f_k = 1$); and $\beta_{ki}$ is the methylation fraction in component $k$ at CpG $i$. The product over $i$ included only those positions for which a methylation state could be identified from the sequencing. Maximum-likelihood values of the parameters $\{f_k, \beta_{ki}\}$ of each model were estimated by using the rprop algorithm (e.g., the rprop algorithm as described in Riedmiller M, Braun H. RPROP - A Fast Adaptive Learning Algorithm. Proceedings of the International Symposium on Computer and Information Science VII, 1992) to maximize the total log-likelihood of the fragments of one training label, subject to a regularization penalty on $\beta_{ki}$ that took the form of a beta-distributed prior. Mathematically, the maximized quantity was

$$(2) \quad \sum_j ln\left(Pr\left(fragment_j | \{\beta_{ki}, f_k\}\right)\right) + \sum_{k,i} r \ ln\left(\beta_{ki}(1 - \beta_{ki})\right)$$

where r is the regularization strength, which was set to 1.

*Featurization*

Once the probabilistic models were trained, a set of numerical features was computed for each sample. Specifically, features were extracted for each fragment from each training sample, for each cancer type and non-cancer sample, in each region. The extracted features were the tallies of outlier fragments (i.e., anomalously methylated fragments), which were defined as those whose log-likelihood under a first cancer model exceeded the log-likelihood under a second cancer model or non-cancer model by at least a threshold tier value. Outlier fragments were tallied separately for each genomic region, sample model (i.e., cancer type), and tier (for tiers 1, 2, 3, 4, 5, 6, 7, 8, and 9), yielding 9 features per region for each sample type. In this way, each feature was defined by three properties: a genomic region; a "positive" cancer type label (excluding non-cancer); and the tier value selected from the set {1, 2, 3, 4, 5, 6, 7, 8, 9}. The numerical value of each feature was defined as the number of fragments in that region such that

$$(3)\ ln\left(\frac{Pr(fragment|positive\ cancer\ type)}{Pr(fragment|non-cancer)}\right) > tier$$

where the probabilities were defined by equation (1) using the maximum-likelihood-estimated parameter values corresponding to the "positive" cancer type (in the numerator of the logarithm) or to non-cancer (in the denominator).

*Feature ranking*

For each set of pairwise features, the features were ranked using mutual information based on their ability to distinguish the first cancer type (which defined the log-likelihood model from which the feature was derived) from the second cancer type or non-cancer. Specifically, two ranked lists of features were compiled for each unique pair of class labels: one with the first label assigned as the "positive" and the second as the "negative", and the other with the positive/negative assignment swapped (with the exception of the "non-cancer" label, which was only permitted as the negative label). For each of these ranked lists, only features whose positive cancer type label (as in equation (3)) matched the positive label under consideration were included in the ranking. For each such feature, the fraction of training samples with non-zero feature value was calculated separately for the positive and negative labels. Features for which this fraction was greater in the positive label were ranked by their mutual information with respect to that pair of class labels.

The top ranked 256 features from each pairwise comparison were identified and added to the final feature set for each cancer type and non-cancer. To avoid redundancy, if more than one feature was selected from the same positive type and genomic region (i.e., for multiple negative types), only the one assigned the lowest (most informative) rank for its cancer type pair was retained, breaking ties by choosing the higher tier value. The features in the final feature set for each sample (cancer type and non-cancer) were binarized (any feature value greater than 0 was set to 1, so that all features were either 0 or 1).

*Classifier training*

The training samples were then divided into distinct 5-fold cross-validation training sets, and a two-stage classifier was trained for each fold, in each case training on 4/5 of the training samples and using the remaining 1/5 for validation.

In the first stage of training, a binary (two-class) logistic regression model for detecting the presence of cancer was trained to discriminate the cancer samples (regardless of TOO) from non-cancer. When training this binary classifier, a sample weight was assigned to the male non-cancer samples to counteract sex-imbalance in the training set. For each sample, the binary classifier outputs a prediction score indicating the likelihood of a presence or absence of cancer.

In the second stage of training, a parallel multi-class logistic regression model for determining cancer tissue of origin was trained with TOO as the target label. Only the cancer samples that received a score above the 95th percentile of the non-cancer samples in the first stage classifier were included in the training of this multi-class classifier. For each cancer sample used in training the multi-class classifier, the multi-class classifier outputs prediction values for the cancer types being classified, where each prediction value is a likelihood that the given sample has a certain cancer type. For example, the cancer classifier can return a cancer prediction for a test sample including a prediction score for breast cancer, a prediction score for lung cancer, and/or a prediction score for no cancer.

Both binary and multi-class classifiers were trained by stochastic gradient descent with mini-batches, and in each case, training was stopped early when the performance on the validation fold (assessed by cross-entropy loss) began to degrade. For predicting on samples outside of the training set, in each stage, the scores assigned by the five cross-validated classifiers were averaged. Scores assigned to sex-inappropriate cancer types were set to zero, with the remaining values renormalized to sum to one.

Scores assigned to the validation folds within the training set were retained for use in assigning cutoff values (thresholds) to target certain performance metrics. In particular, the probability scores assigned to the training set non-cancer samples were used to define thresholds corresponding to particular specificity levels. For example, for a desired specificity target of 99.4%, the threshold was set at the 99.4th percentile of the cross-validated cancer detection probability scores assigned to the non-cancer samples in the training set. Training samples with a probability score that exceeded a threshold were called as positive for cancer.

Subsequently, for each training sample determined to be positive for cancer, a TOO or cancer type assessment was made from the multiclass classifier. First, the multi-class logistic regression classifier assigned a set of probability scores, one for each prospective cancer type, to each sample. Next, the confidence of these scores was assessed as the difference between the highest and second-highest scores assigned by the multi-class classifier for each sample. Then, the cross-validated training set scores were used to identify the lowest threshold value such that of the cancer samples in the training set with top-two score differential exceeding the threshold, 90% had been assigned the correct TOO label as their highest score. In this way, the scores assigned to the validation folds during training were further used to determine a second threshold for distinguishing between confident and indeterminate TOO calls.

At prediction time, samples receiving a score from the binary (first-stage) classifier below the predefined specificity threshold were assigned a "non-cancer" label. For the remaining samples, those whose top-two TOO-score differential from the second-stage classifier was below the second predefined threshold were assigned the "indeterminate cancer" label. The remaining samples were assigned the cancer label to which the TOO classifier assigned the highest score.

## EXAMPLE 5 - Classifier with the target genomic regions of Lists 4-16

The discriminatory value of the target genomic regions of Lists 4-16 was evaluated by testing the ability of a cancer classifier to detect cancer and any of 20 different cancer types according to the methylation status of these target genomic regions. Performance was evaluated over a set of 1,532 cancer samples and 1,521 non-cancer samples that were not used to train the classifier, as shown in **TABLE 5.** For each sample, differentially methylated cfDNA was enriched using a bait set comprising all of the target genomic regions of Lists 1-16. The classifier was then constrained to provide cancer determinations based only on the methylation status of the target genomic regions of the List being evaluated.

### TABLE 5 - Cancer diagnoses of individuals whose cfDNA was used to train the classifier

| Cancer Type | Total | Stage | | | | |
|---|---|---|---|---|---|---|
| | | I | II | III | IV | Not Reported |
| Non-cancer | 1521 | - | - | - | - | - |
| Lung | 261 | 60 | 23 | 72 | 106 | 0 |
| Breast | 247 | 102 | 110 | 27 | 8 | 0 |
| Prostate | 188 | 39 | 113 | 19 | 17 | 0 |
| Lymphoid neoplasm | 147 | 15 | 27 | 27 | 39 | 39 |
| Colorectal | 121 | 13 | 22 | 41 | 45 | 0 |
| Pancreas and gallbladder | 95 | 15 | 15 | 19 | 46 | 0 |
| Uterine | 84 | 73 | 3 | 5 | 3 | 0 |
| Upper GI | 67 | 9 | 12 | 19 | 27 | 0 |
| Head and neck | 62 | 7 | 13 | 16 | 26 | 0 |
| Renal | 56 | 37 | 4 | 4 | 11 | 0 |
| Ovary | 37 | 4 | 2 | 25 | 6 | 0 |
| Multiple myeloma | 34 | 10 | 13 | 11 | 0 | 0 |
| Not reported | 29 | 8 | 5 | 7 | 6 | 3 |
| Liver bile duct | 29 | 5 | 7 | 7 | 10 | 0 |
| Sarcoma | 17 | 2 | 4 | 5 | 6 | 0 |
| Bladder and urothelial | 16 | 6 | 7 | 3 | 1 | 0 |
| Anorectal | 14 | 4 | 5 | 5 | 0 | 0 |

(continued)

| Cancer Type | Total | Stage | | | | |
|---|---|---|---|---|---|---|
| | | I | II | III | IV | Not Reported |
| Cervical | 11 | 8 | 1 | 2 | 0 | 0 |
| Melanoma | 7 | 3 | 1 | 0 | 3 | 0 |
| Myeloid neoplasm | 4 | 2 | 1 | 0 | 1 | 0 |
| Thyroid | 4 | 0 | 0 | 0 | 0 | 4 |
| Prediction only | 2 | 0 | 0 | 0 | 2 | 0 |

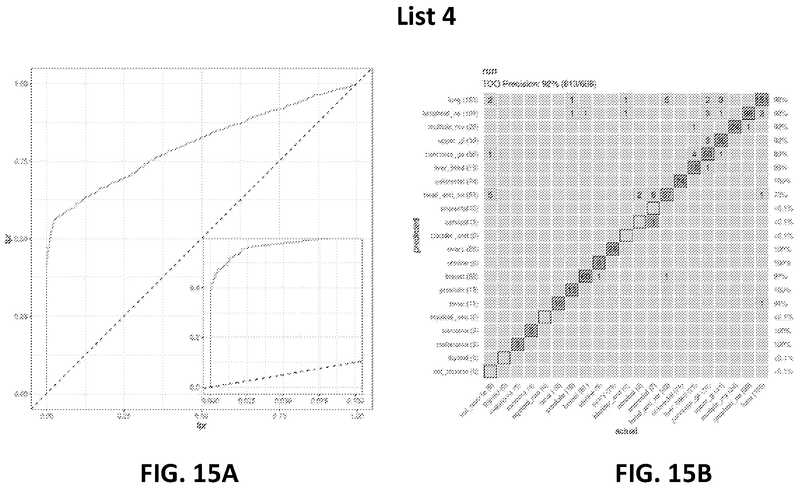

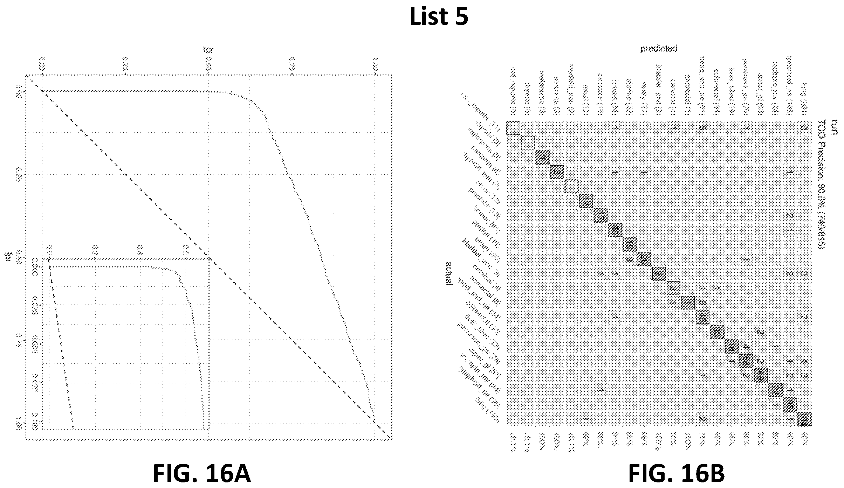

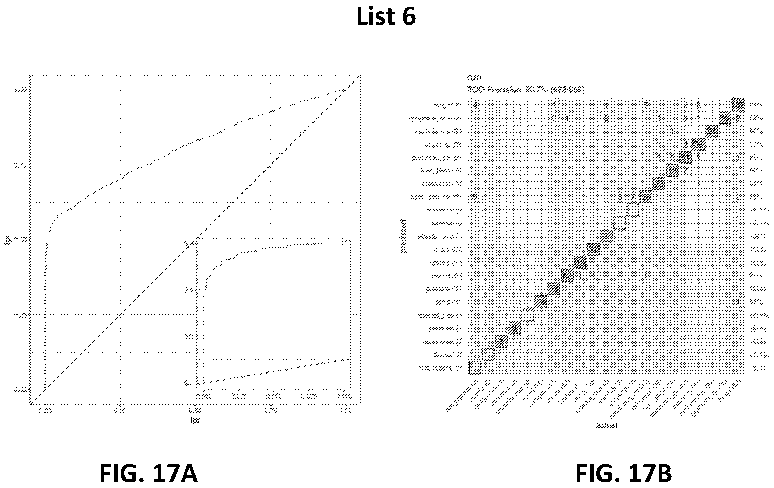

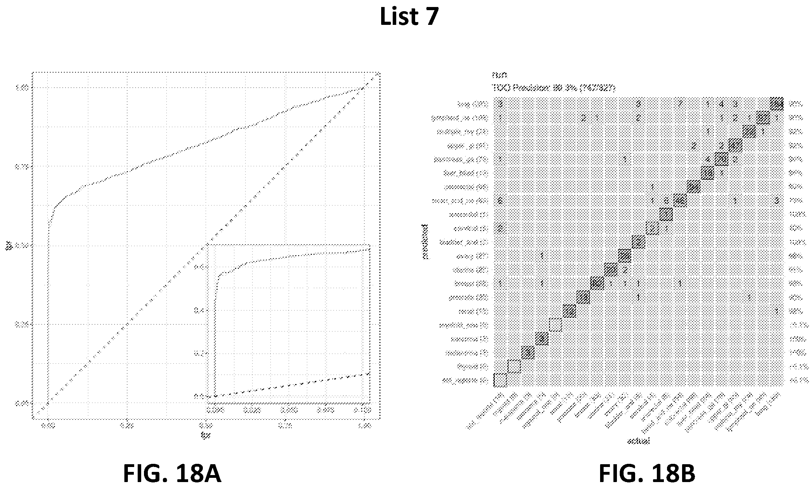

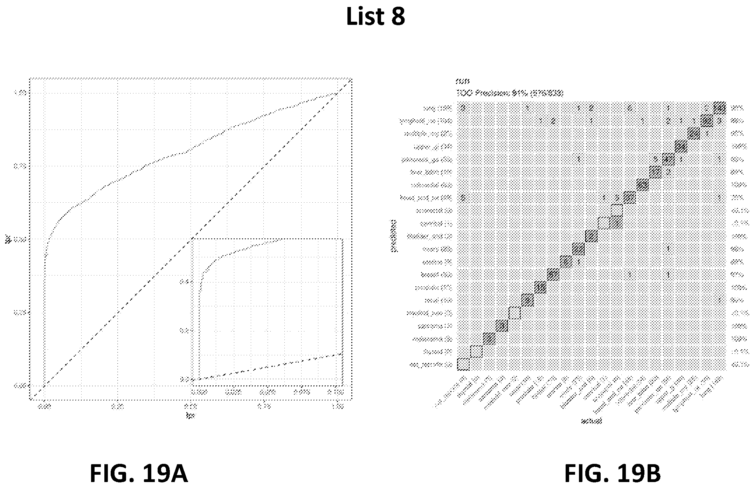

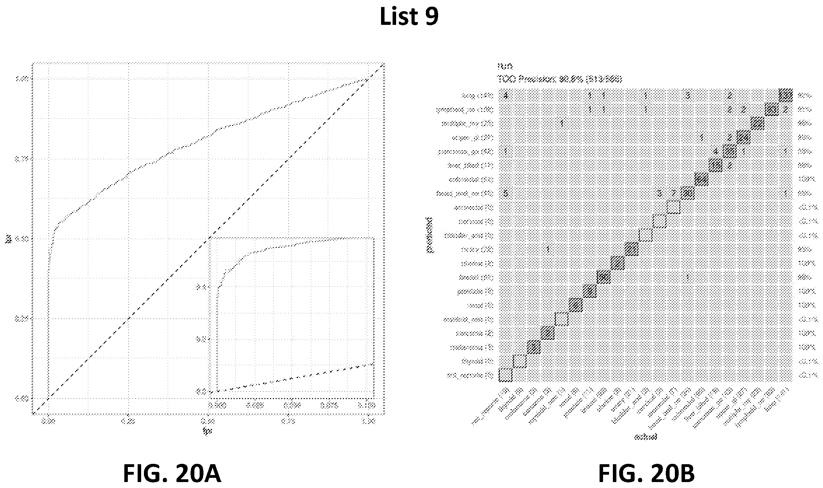

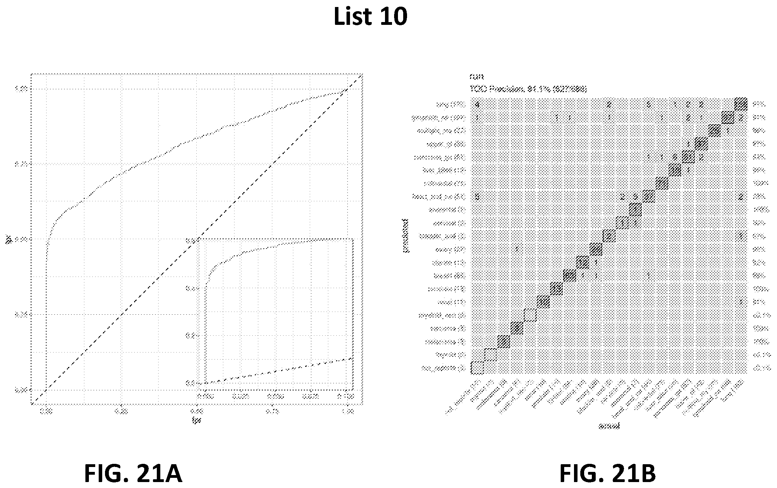

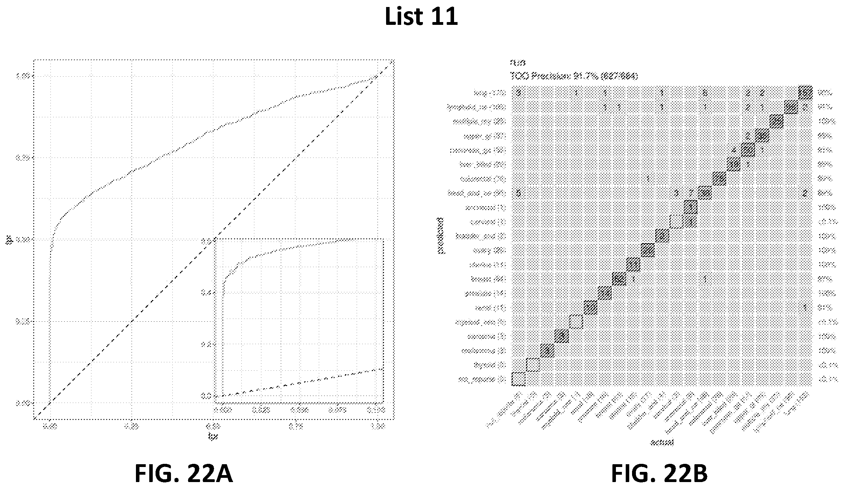

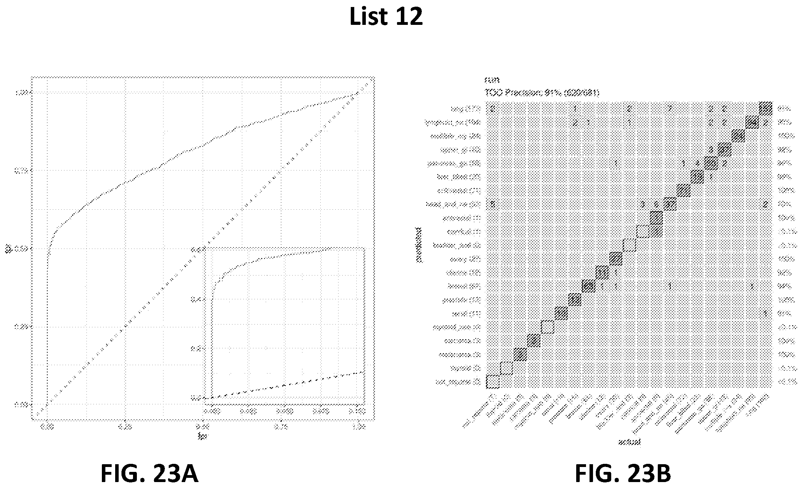

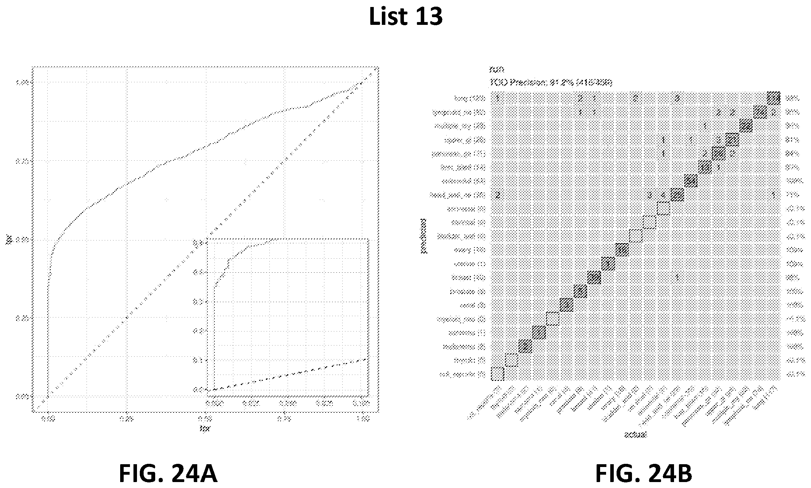

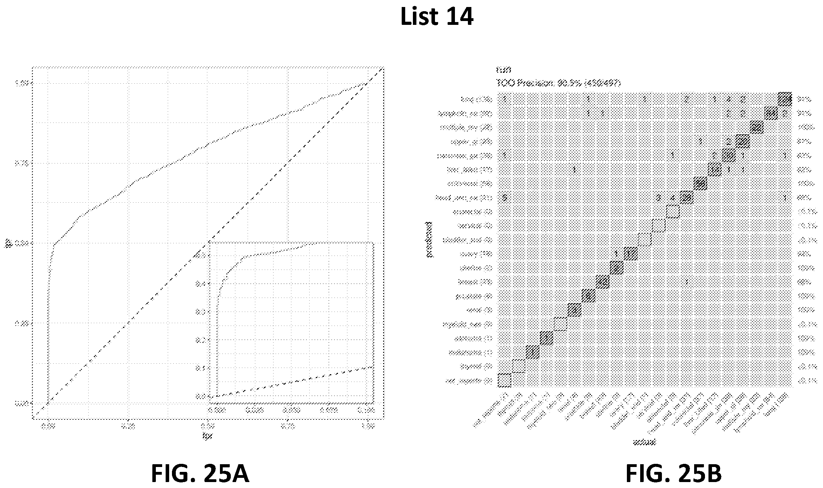

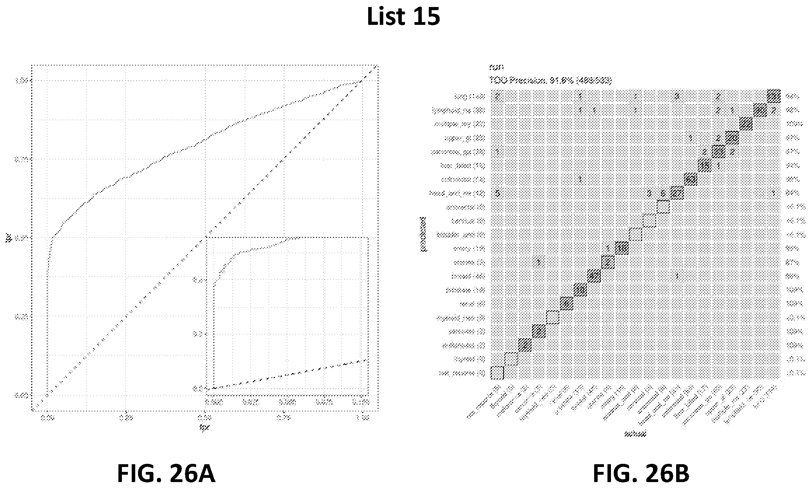

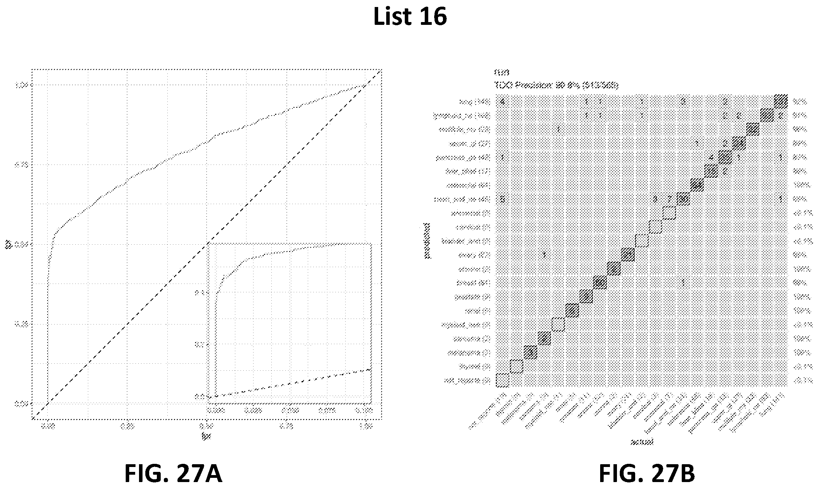

Results from the classifier performance analysis for Lists 4-16 are presented in **FIGURES 15-27.** In each figure, part A is a receiver operator curve (ROC) showing true positive results and false positive results for a determination of cancer or no-cancer. The asymmetric shape of these ROC curves illustrates that the classifier was designed to minimize false positive results. The areas under the curve are tightly clustered between 0.78 and 0.83, as shown in **TABLE 6.** These results indicate that a determination of cancer is not grossly compromised by using smaller panels of less than 1 MB, such as Lists 8, 9, and 13, compared to larger panels of greater than 10 MB, such as Lists 6 and 6.

**TABLE 6.**

| Target regions | AUC |
|---|---|
| List 4 | 0.81 |
| List 5 | 0.83 |
| List 6 | 0.81 |
| List 7 | 0.83 |
| List 8 | 0.80 |
| List 9 | 0.81 |
| List 10 | 0.81 |
| List 11 | 0.81 |
| List 12 | 0.81 |
| List 13 | 0.78 |
| List 14 | 0.79 |
| List 15 | 0.80 |
| List 16 | 0.80 |

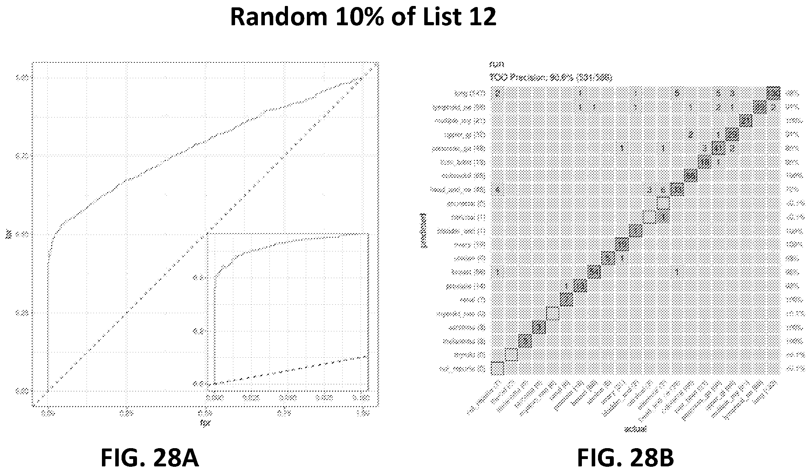

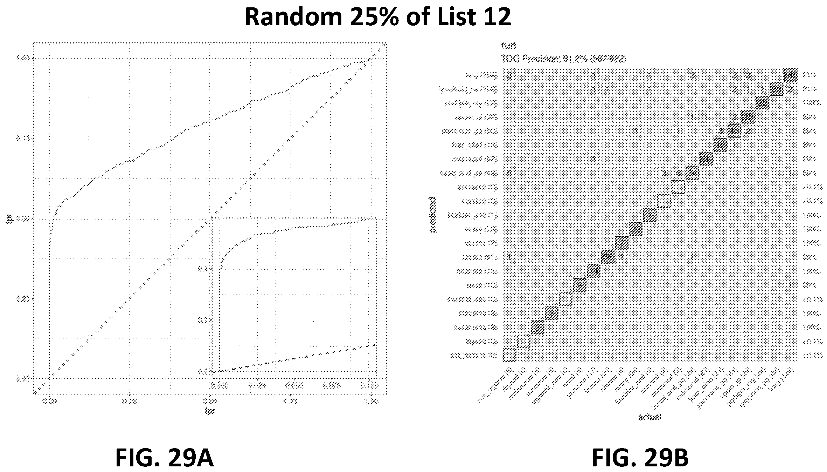

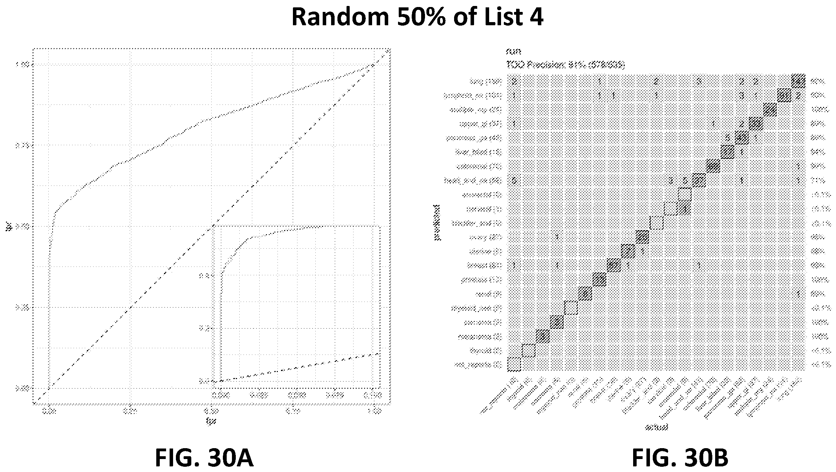

Classifier performance was also evaluated for randomly selected subsets of the target genomic regions of List 4 and List 12, as shown in **FIGURES 28 - 30** and **TABLE 7.** Again, the smallest panel (Random 10% of List 12, 0.36 MB) had similar results to the largest panel (List 4, 4.63 MB), indicating that methylation status results for at least a substantial majority of the target regions in all lists are informative of the presence or absence of cancer.

**TABLE 7**

| Target regions | AUC |
|---|---|
| List 4 | 0.81 |
| Random 50% of List 4 | 0.81 |
| List 12 | 0.81 |
| Random 10% of List 12 | 0.78 |
| Random 25% of List 12 | 0.79 |

A Cancer Type (i.e. TOO) determination was attempted for all samples with a determination of cancer. Panel B in **FIGURES 15-30** shows the accuracy of these determinations. For example, the value in the top right corner of **FIGURE 15B** indicates that 151 samples classified as lung cancer based on the methylation status of the target genomic regions of List 4 had been obtained from subjects known to have lung cancer. The "3" value 3 positions to the left in the same confusion matrix indicates that three samples predicted to have lung cancer were from subjects who actually had an Upper GI cancer. Overall, the vast majority of cancer type determinations made using the target genomic regions of any of Lists 4-16 fall on the diagonals of the confusion matrices, indicating that the classifier determined the correct cancer type. Similar results were obtained using randomly selected target genomic regions from Lists 4 and 12,

These classifier results are further summarized in **TABLES 8 - 23,** which indicate the accuracy of cancer detections and cancer type determinations made with a specificity of 0.990, indicating a false positive rate of 1%. These results are delineated by cancer stage. They show improved cancer detection and cancer type determinations for samples from individuals with later stage cancers (e.g. stage IV) compared to samples from individuals with earlier stage cancers (e.g. stage I). For all cancer stages (no segregation by stage), the cancer type determination was accurate approximately 90% of the time for all target genomic region lists and for random subsets of List 4 and List 12. For Stage I cancers, an accurate cancer type determination was made approximately 75% of the time. In particular, 75.6% of the cancer type determinations were accurate for the smallest assay panel, List 8, with only 1370 target genomic regions having a total size of 395 kb.

The same accuracy results are broken down according to cancer type in **TABLE 24,** which demonstrates highly accurate cancer type determinations with the target genomic regions of all lists for common cancers such as liver and bile duct cancer, rare cancers such as sarcoma, and hard-to-detect cancers such as breast cancer.

The sensitivity for detecting 20 different cancer types using the target genomic regions of lists 4-16 or randomly selected portions from lists 4 and 12 is presented in **TABLES 25-40.** Sensitivity results are presented for a specificity of 0.990 (a 1% false positive rate). Sensitivity is presented for all cancers of the specified cancer type and for cancers at stages I through IV. The sensitivity was generally higher for later stage cancers. For stage IV cancers, the sensitivity was greater than 60% for all cancers with more than one sample and was greater than 90% for breast cancer, ovarian cancer, bladder & urothecal cancer, head & neck cancer, colorectal cancer, liver cancer, pancreas & gallbladder cancer, upper GI cancer, lymphoid neoplasm, and lung cancer. At stage II, sensitivity was best for head & neck cancer, liver cancer, pancreas & gallbladder cancer, upper GI cancer, lymphoid neoplasm, and lung cancer. List 8, the smallest group of target genomic regions, provide a sensitivity of at least 50% for these stage II cancers.

**TABLE 8.** Classification accuracy using the genomic regions of List 4. Data for Cancer Presence and Cancer type at a specificity of 0.990 show percentage accuracy, a 95% confidence interval in brackets, and the number correctly assigned over the total in parentheses.

| Stage | Cancer Presence | Cancer Type |
|---|---|---|
| I | 13% [10-16.6] (55/422) | 78.6% [63.2-89.7] (33/42) |
| II | 34.5% [29.8-39.5] (134/388) | 88.1% [81.1-93.2] (111/126) |
| III | 72.2% [66.9-77.1] (226/313) | 91% [86.1-94.6] (182/200) |
| IV | 85.1% [81-88.6] (309/363) | 91.9% [88.3-94.8] (274/298) |
| All | 49.2% [46.6-51.7] (753/1532) | 90.5% [88-92.6] (627/693) |

**TABLE 9.** Classification accuracy using the genomic regions of List 5.

| Stage | Cancer Presence | Cancer Type |
|---|---|---|
| I | 20.9% [17.1-25] (88/422) | 77.3% [66.2-86.2] (58/75) |
| II | 45.9% [40.8-51] (178/388) | 88.3% [82.4-92.8] (144/163) |
| III | 82.7% [78.1-86.8] (259/313) | 89.9% [85.5-93.4] (223/248) |
| IV | 90.6% [87.2-93.4] (329/363) | 92.1% [88.5-94.8] (291/316) |
| All | 57.4% [54.9-59.9] (880/1532) | 89.5% [87.2-91.5] (740/827) |

**TABLE 10.** Classification accuracy using the genomic regions of List 6.

| Stage | Cancer Presence | Cancer Type |
|---|---|---|
| I | 13.3% [10.2-16.9] (56/422) | 76% [61.8-86.9] (38/50) |

(continued)

| Stage | Cancer Presence | Cancer Type |
|---|---|---|
| II | 36.3% [31.5-41.3] (141/388) | 87.7% [80.8-92.8] (114/130) |
| III | 72.5% [67.2-77.4] (227/313) | 91.1% [86.3-94.7] (185/203) |
| IV | 85.1% [81-88.6] (309/363) | 91.6% [87.8-94.5] (271/296) |
| All | 49.6% [47.1-52.1] (760/1532) | 89.9% [87.4-92] (633/704) |

**TABLE 11.** Classification accuracy using the genomic regions of List 7.

| Stage | Cancer Presence | Cancer Type |
|---|---|---|
| I | 21.3% [17.5-25.5] (90/422) | 77% [65.8-86] (57/74) |
| II | 45.9% [40.8-51] (178/388) | 88.5% [82.6-92.9] (146/165) |
| III | 82.1% [77.4-86.2] (257/313) | 89.8% [85.4-93.3] (221/246) |
| IV | 90.4% [86.8-93.2] (328/363) | 92.7% [89.2-95.3] (292/315) |
| All | 57.4% [54.9-59.9] (879/1532) | 89.6% [87.3-91.6] (740/826) |

**TABLE 12.** Classification accuracy using the genomic regions of List 8.

| Stage | Cancer Presence | Cancer Type |
|---|---|---|
| I | 13% [10-16.6] (55/422) | 75.6% [59.7-87.6] (31/41) |
| II | 33% [28.3-37.9] (128/388) | 89.2% [81.9-94.3] (99/111) |
| III | 67.7% [62.2-72.9] (212/313) | 89.9% [84.7-93.8] (169/188) |
| IV | 84.6% [80.4-88.1] (307/363) | 91% [87.1-94] (262/288) |
| All | 47.5% [45-50.1] (728/1532) | 89.5% [86.9-91.8] (582/650) |

**TABLE 13.** Classification accuracy using the genomic regions of List 9.

| Stage | Cancer Presence | Cancer Type |
|---|---|---|
| I | 12.1% [9.1-15.6] (51/422) | 76.3% [59.8-88.6] (29/38) |
| II | 35.1% [30.3-40] (136/388) | 88.1% [81.1-93.2] (111/126) |
| III | 68.4% [62.9-73.5] (214/313) | 92.1% [87.2-95.5] (174/189) |
| IV | 85.1% [81-88.6] (309/363) | 90.7% [86.8-93.8] (264/291) |
| All | 48.1% [45.6-50.6] (737/1532) | 89.9% [87.3-92] (602/670) |

**TABLE 14.** Classification accuracy using the genomic regions of List 10.

| Stage | Cancer Presence | Cancer Type |
|---|---|---|
| I | 14.2% [11-17.9] (60/422) | 72.3% [57.4-84.4] (34/47) |
| II | 36.9% [32-41.9] (143/388) | 87.2% [80.3-92.4] (116/133) |
| III | 71.9% [66.6-76.8] (225/313) | 92.6% [88-95.8] (187/202) |
| IV | 85.1% [81-88.6] (309/363) | 90.9% [87-93.9] (269/296) |
| All | 49.7% [47.2-52.3] (762/1532) | 89.6% [87.1-91.7] (627/700) |

**TABLE 15.** Classification accuracy using the genomic regions of List 11.

| Stage | Cancer Presence | Cancer Type |
|---|---|---|
| I | 13% [10-16.6] (55/422) | 78.3% [63.6-89.1] (36/46) |

(continued)

| Stage | Cancer Presence | Cancer Type |
|---|---|---|
| II | 35.3% [30.6-40.3] (137/388) | 90.7% [84.3-95.1] (117/129) |
| III | 72.5% [67.2-77.4] (227/313) | 87.7% [82.5-91.8] (185/211) |
| IV | 85.1% [81-88.6] (309/363) | 91.1% [87.3-94.1] (277/304) |
| All | 49.3% [46.8-51.9] (756/1532) | 89.4% [86.9-91.6] (641/717) |

TABLE 16. Classification accuracy using the genomic regions of List 12.

| Stage | Cancer Presence | Cancer Type |
|---|---|---|
| I | 13.5% [10.4-17.1] (57/422) | 73.5% [58.9-85.1] (36/49) |
| II | 36.9% [32-41.9] (143/388) | 88.5% [81.7-93.4] (115/130) |
| III | 72.2% [66.9-77.1] (226/313) | 92.5% [87.9-95.7] (185/200) |
| IV | 84.8% [80.7-88.4] (308/363) | 91.5% [87.7-94.4] (269/294) |
| All | 49.6% [47.1-52.1] (760/1532) | 90.1% [87.6-92.2] (628/697) |

TABLE 17. Classification accuracy using the genomic regions of List 13.

| Stage | Cancer Presence | Cancer Type |
|---|---|---|
| I | 9% [6.5-12.2] (38/422) | 78.9% [54.4-93.9] (15/19) |
| II | 29.9% [25.4-34.7] (116/388) | 86% [76.9-92.6] (74/86) |
| III | 57.5% [51.8-63.1] (180/313) | 92.1% [86.3-96] (128/139) |
| IV | 80.7% [76.3-84.6] (293/363) | 90.7% [86.3-94.1] (215/237) |
| All | 42% [39.5-44.5] (643/1532) | 90.1% [87.1-92.6] (445/494) |

TABLE 18. Classification accuracy using the genomic regions of List 14.

| Stage | Cancer Presence | Cancer Type |
|---|---|---|
| I | 8.5% [6-11.6] (36/422) | 75% [50.9-91.3] (15/20) |
| II | 30.2% [25.6-35] (117/388) | 85.9% [77-92.3] (79/92) |
| III | 61.3% [55.7-66.8] (192/313) | 91.4% [85.7-95.3] (138/151) |
| IV | 81% [76.6-84.9] (294/363) | 90.2% [85.8-93.6] (222/246) |
| All | 43.4% [40.9-45.9] (665/1532) | 89.6% [86.7-92.1] (474/529) |

TABLE 19. Classification accuracy using the genomic regions of List 15.

| Stage | Cancer Presence | Cancer Type |
|---|---|---|
| I | 10.2% [7.5-13.5] (43/422) | 70.4% [49.8-86.2] (19/27) |
| II | 31.7% [27.1-36.6] (123/388) | 87.4% [79.4-93.1] (90/103) |
| III | 62% [56.4-67.4] (194/313) | 91.7% [86.3-95.5] (144/157) |
| IV | 82.1% [77.8-85.9] (298/363) | 90.5% [86.2-93.7] (237/262) |
| All | 44.7% [42.2-47.2] (685/1532) | 89.7% [86.9-92.1] (514/573) |

TABLE 20. Classification accuracy using the genomic regions of List 16.

| Stage | Cancer Presence | Cancer Type |
|---|---|---|
| I | 10.2% [7.5-13.5] (43/422) | 65.4% [44.3-82.8] (17/26) |

(continued)

| Stage | Cancer Presence | Cancer Type |
|---|---|---|
| II | 33% [28.3-37.9] (128/388) | 88.5% [81.1-93.7] (100/113) |
| III | 65.5% [59.9-70.8] (205/313) | 90.9% [85.4-94.8] (150/165) |
| IV | 83.2% [78.9-86.9] (302/363) | 91.3% [87.3-94.4] (242/265) |
| All | 46% [43.5-48.6] (705/1532) | 89.7% [87-92] (532/593) |

**TABLE 21.** Classification accuracy using a randomly selected subset of 10% of the genomic regions of List 12.

| Stage | Cancer Presence | Cancer Type |
|---|---|---|
| I | 10% [7.3-13.2] (42/422) | 78.1% [60-90.7] (25/32) |
| II | 32% [27.3-36.9] (124/388) | 87.5% [79.9-93] (98/112) |
| III | 61% [55.4-66.5] (191/313) | 89.6% [84.1-93.7] (155/173) |
| IV | 82.9% [78.6-86.6] (301/363) | 90.5% [86.4-93.7] (247/273) |
| All | 44.1% [41.6-46.7] (676/1532) | 89.3% [86.6-91.6] (542/607) |

**TABLE 22.** Classification accuracy using a randomly selected subset of 25% of the genomic regions of List 12.

| Stage | Cancer Presence | Cancer Type |
|---|---|---|
| I | 11.8% [8.9-15.3] (50/422) | 71.4% [55.4-84.3] (30/42) |
| II | 33.2% [28.6-38.2] (129/388) | 90.8% [84.2-95.3] (109/120) |
| III | 65.5% [59.9-70.8] (205/313) | 89.9% [84.7-93.8] (169/188) |
| IV | 84.6% [80.4-88.1] (307/363) | 91.5% [87.7-94.5] (260/284) |
| All | 46.5% [44-49.1] (713/1532) | 89.9% [87.4-92.1] (589/655) |

**TABLE 23.** Classification accuracy using a randomly selected subset of 50% of the genomic regions of List 4.

| Stage | Cancer Presence | Cancer Type |
|---|---|---|
| I | 11.4% [8.5-14.8] (48/422) | 73.8% [58-86.1] (31/42) |
| II | 33.2% [28.6-38.2] (129/388) | 88.5% [81.5-93.6] (108/122) |
| III | 64.9% [59.3-70.1] (203/313) | 92.9% [88.2-96.2] (171/184) |
| IV | 83.2% [78.9-86.9] (302/363) | 90.4% [86.4-93.5] (263/291) |
| All | 46.3% [43.8-48.9] (710/1532) | 89.8% [87.2-92] (598/666) |

**TABLE 24.** Cancer type classification accuracy with various genomic target regions.

| Cancer Type | List 4 | | List 5 | | List 6 | | List 7 | | List 8 | | List 9 | | List 10 | |
|---|---|---|---|---|---|---|---|---|---|---|---|---|---|---|
| | % | Fxn | % | Fxn | % | Fxn | % | Fxn | % | Fxn | % | Fxn | % | Fxn |
| All Types | 92 | 613/666 | 91 | 740/815 | 91 | 622/686 | 90 | 747/827 | 91 | 576/633 | 90 | 593/656 | 91 | 627/688 |
| Thyroid | n/a | 0/0 | 0 | 0/0 | n/a | 0/0 | n/a | 0/0 | n/a | 0/0 | n/a | 0/0 | n/a | 0/0 |
| Melanoma | n/a | 0/0 | 100 | 3/3 | n/a | 0/0 | n/a | 0/0 | n/a | 0/0 | n/a | 0/0 | n/a | 0/0 |
| Sarcoma | 100 | 3/3 | 100 | 3/3 | 100 | 3/3 | 100 | 3/3 | 100 | 3/3 | 100 | 3/3 | 100 | 3/3 |
| Myeloid Neoplasm | 100 | 3/3 | 0 | 0/0 | 100 | 3/3 | 100 | 3/3 | 100 | 3/3 | 100 | 3/3 | 100 | 3/3 |
| Renal | n/a | 0/0 | 92 | 12/13 | n/a | 0/0 | n/a | 0/0 | n/a | 0/0 | n/a | 0/0 | n/a | 0/0 |
| Prostate | 91 | 10/11 | 90 | 17/19 | 91 | 10/11 | 92 | 12/13 | 90 | 9/10 | 90 | 9/10 | 91 | 10/11 |
| Breast | 100 | 13/13 | 95 | 80/84 | 100 | 13/13 | 90 | 18/20 | 100 | 15/15 | 100 | 14/14 | 100 | 13/13 |
| Uterine | 97 | 60/62 | 86 | 19/22 | 95 | 62/65 | 93 | 82/88 | 97 | 57/59 | 97 | 60/62 | 96 | 63/66 |
| Ovary | 100 | 8/8 | 96 | 26/27 | 100 | 10/10 | 91 | 20/22 | 89 | 8/9 | 100 | 8/8 | 92 | 12/13 |
| Bladder & Urothelial | 100 | 28/28 | 100 | 2/2 | 100 | 27/27 | 96 | 26/27 | 96 | 22/23 | 92 | 23/25 | 96 | 26/27 |
| Cervical | n/a | 0/0 | 50 | 2/4 | 100 | 1/1 | 100 | 2/2 | 100 | 2/2 | 100 | 2/2 | 67 | 2/3 |
| Anorectal | 0 | 0/1 | 100 | 1/1 | n/a | 0/0 | 40 | 2/5 | 0 | 0/1 | 0 | 0/1 | 50 | 1/2 |
| Head & Neck | n/a | 0/0 | 75 | 46/61 | n/a | 0/0 | 100 | 1/1 | n/a | 0/0 | 100 | 1/1 | 100 | 1/1 |
| Colorectal | 73 | 37/51 | 99 | 93/94 | 69 | 38/55 | 73 | 46/63 | 76 | 37/49 | 71 | 37/52 | 73 | 37/51 |
| Liver & Bile duct | 100 | 74/74 | 95 | 18/19 | 99 | 73/74 | 99 | 94/95 | 100 | 63/63 | 99 | 64/65 | 100 | 71/71 |
| Pancreas & Gallbladder | 95 | 18/19 | 90 | 68/76 | 90 | 18/20 | 95 | 18/19 | 90 | 17/19 | 90 | 17/19 | 95 | 18/19 |

**TABLE 24** (continued). Cancer type classification accuracy with various genomic target regions.

| Cancer Type | List 11 | | List 12 | | List 13 | | List 14 | | List 15 | | List 16 | |
|---|---|---|---|---|---|---|---|---|---|---|---|---|
| | % | Fxn | % | Fxn | % | Fxn | % | Fxn | % | Fxn | % | Fxn |
| All Types | 92 | 627/684 | 91 | 620/681 | 91 | 416/456 | 91 | 450/497 | 92 | 488/533 | 91 | 513/565 |
| Thyroid | n/a | 0/0 | n/a | 0/0 | n/a | 0/0 | n/a | 0/0 | n/a | 0/0 | n/a | 0/0 |
| Melanoma | n/a | 0/0 | n/a | 0/0 | n/a | 0/0 | n/a | 0/0 | n/a | 0/0 | n/a | 0/0 |
| Sarcoma | 100 | 3/3 | 100 | 3/3 | 100 | 2/2 | 100 | 1/1 | 100 | 2/2 | 100 | 3/3 |
| Myeloid Neoplasm | 100 | 3/3 | 100 | 3/3 | 100 | 1/1 | 100 | 1/1 | 100 | 2/2 | 100 | 2/2 |
| Renal | n/a | 0/0 | n/a | 0/0 | n/a | 0/0 | n/a | 0/0 | n/a | 0/0 | n/a | 0/0 |
| Prostate | 91 | 10/11 | 91 | 10/11 | 100 | 3/3 | 100 | 3/3 | 100 | 6/6 | 100 | 6/6 |
| Breast | 100 | 14/14 | 100 | 13/13 | 100 | 5/5 | 100 | 6/6 | 100 | 10/10 | 100 | 9/9 |
| Uterine | 97 | 62/64 | 94 | 63/67 | 98 | 39/40 | 98 | 42/43 | 98 | 47/48 | 98 | 50/51 |
| Ovary | 100 | 11/11 | 92 | 11/12 | 100 | 1/1 | 100 | 2/2 | 67 | 2/3 | 100 | 2/2 |
| Bladder & Urothelial | 100 | 26/26 | 100 | 27/27 | 100 | 16/16 | 94 | 17/18 | 95 | 18/19 | 96 | 21/22 |
| Cervical | 100 | 2/2 | n/a | 0/0 | n/a | 0/0 | n/a | 0/0 | n/a | 0/0 | n/a | 0/0 |
| Anorectal | 0 | 0/1 | 0 | 0/1 | n/a | 0/0 | n/a | 0/0 | n/a | 0/0 | n/a | 0/0 |
| Head & Neck | 100 | 1/1 | 100 | 1/1 | n/a | 0/0 | n/a | 0/0 | n/a | 0/0 | n/a | 0/0 |
| Colorectal | 69 | 38/55 | 70 | 37/53 | 71 | 25/35 | 68 | 28/41 | 64 | 27/42 | 65 | 30/46 |
| Liver & Bile duct | 99 | 75/76 | 100 | 71/71 | 100 | 54/54 | 100 | 56/56 | 98 | 63/64 | 100 | 64/64 |
| Pancreas & Gallbladder | 95 | 19/20 | 95 | 19/20 | 93 | 13/14 | 82 | 14/17 | 94 | 15/16 | 88 | 15/17 |

**TABLE 24** (continued). Cancer type classification accuracy with various genomic target regions.

| Cancer Type | List 4 | | Random 50% of List 4 | | List 12 | | Random 10% of List 12 | | Random 25% of List 12 | |
|---|---|---|---|---|---|---|---|---|---|---|
| | % | Fxn | % | Fxn | % | Fxn | % | Fxn | % | Fxn |
| All Types | 92 | 613/666 | 91 | 578/635 | 91 | 620/681 | 91 | 531/586 | 91 | 567/622 |
| Thyroid | n/a | 0/0 | n/a | 0/0 | n/a | 0/0 | n/a | 0/0 | n/a | 0/0 |
| Melanoma | n/a | 0/0 | n/a | 0/0 | n/a | 0/0 | n/a | 0/0 | n/a | 0/0 |
| Sarcoma | 100 | 3/3 | 100 | 3/3 | 100 | 3/3 | 100 | 3/3 | 100 | 3/3 |
| Myeloid Neoplasm | 100 | 3/3 | 100 | 3/3 | 100 | 3/3 | 100 | 3/3 | 100 | 3/3 |
| Renal | n/a | 0/0 | n/a | 0/0 | n/a | 0/0 | n/a | 0/0 | n/a | 0/0 |
| Prostate | 91 | 10/11 | 89 | 8/9 | 91 | 10/11 | 100 | 7/7 | 90 | 9/10 |
| Breast | 100 | 13/13 | 100 | 13/13 | 100 | 13/13 | 93 | 13/14 | 100 | 14/14 |
| Uterine | 97 | 60/62 | 93 | 57/61 | 94 | 63/67 | 96 | 54/56 | 95 | 58/61 |
| Ovary | 100 | 8/8 | 88 | 7/8 | 92 | 11/12 | 83 | 5/6 | 100 | 7/7 |
| Bladder & Urothelial | 100 | 28/28 | 96 | 26/27 | 100 | 27/27 | 100 | 19/19 | 100 | 23/23 |
| Cervical | n/a | 0/0 | n/a | 0/0 | n/a | 0/0 | 100 | 1/1 | 100 | 1/1 |

(continued)

| Cancer Type | List 4 | | Random 50% of List 4 | | List 12 | | Random 10% of List 12 | | Random 25% of List 12 | |
|---|---|---|---|---|---|---|---|---|---|---|
| | % | Fxn | % | Fxn | % | Fxn | % | Fxn | % | Fxn |
| Anorectal | 0 | 0/1 | 0 | 0/1 | 0 | 0/1 | 0 | 0/1 | n/a | 0/0 |
| Head & Neck | n/a | 0/0 | n/a | 0/0 | 100 | 1/1 | n/a | 0/0 | n/a | 0/0 |
| Colorectal | 73 | 37/51 | 71 | 37/52 | 70 | 37/53 | 72 | 33/46 | 69 | 34/49 |
| Liver & Bile duct | 100 | 74/74 | 99 | 69/70 | 100 | 71/71 | 100 | 65/65 | 99 | 66/67 |
| Pancreas & Gallbladder | 95 | 18/19 | 94 | 17/18 | 95 | 19/20 | 95 | 18/19 | 95 | 18/19 |

TABLE 25. Classification sensitivity with 99.0% specificity using the target genomic regions of List 4.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 25 | 1/4 | 20 | 11/56 | 9 | 16/188 | 27 | 67/247 | 18 | 15/84 | 87 | 32/37 | 38 | 6/16 |
| I | 0 | 0/2 | 0 | 0/3 | 50 | 1/2 | | | 0 | 0/37 | 3 | 1/39 | 2 | 2/102 | 14 | 10/73 | 25 | 1/4 | 33 | 2/6 |
| II | 0 | 0/1 | 0 | 0/1 | 0 | 0/4 | | | 0 | 0/4 | 1 | 1/113 | 33 | 36/110 | 33 | 1/3 | 0 | 0/2 | 43 | 3/7 |
| III | | | | | 40 | 2/5 | | | 50 | 2/4 | 11 | 2/19 | 85 | 23/27 | 60 | 3/5 | 100 | 25/25 | 50 | 1/2 |
| IV | 0 | 0/1 | 100 | 3/3 | 33 | 2/6 | | | 82 | 9/11 | 71 | 12/17 | 75 | 6/8 | 33 | 1/3 | 100 | 6/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 84 | 52/62 | 65 | 79/121 | 86 | 25/29 | 68 | 65/95 | 73 | 49/67 | 77 | 26/34 | 73 | 107/147 | 64 | 168/261 |
| I | 13 | 1/8 | 25 | 1/4 | 86 | 6/7 | 8 | 1/13 | 60 | 3/5 | 33 | 5/15 | 11 | 1/9 | 60 | 6/10 | 27 | 4/15 | 12 | 7/60 |
| II | 100 | 1/1 | 60 | 3/5 | 77 | 10/13 | 36 | 8/22 | 86 | 6/7 | 60 | 9/15 | 58 | 7/12 | 69 | 9/13 | 85 | 23/27 | 65 | 15/23 |
| III | 50 | 1/2 | 100 | 5/5 | 75 | 12/16 | 68 | 28/41 | 86 | 6/7 | 58 | 11/19 | 79 | 15/19 | 100 | 11/11 | 78 | 21/27 | 75 | 54/72 |
| IV | | | | | 92 | 24/26 | 93 | 42/45 | 100 | 10/10 | 87 | 40/46 | 96 | 26/27 | | | 80 | 31/39 | 87 | 92/106 |

TABLE 26. Classification sensitivity with 99.0% specificity using the target genomic regions of List 5.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 53 | 9/17 | 0 | 0/4 | 23 | 13/56 | 13 | 25/188 | 35 | 86/247 | 30 | 25/84 | 92 | 34/37 | 63 | 10/16 |
| I | 0 | 0/2 | 0 | 0/3 | 100 | 2/2 | | | 0 | 0/37 | 5 | 2/39 | 5 | 5/102 | 25 | 18/73 | 75 | 3/4 | 67 | 4/6 |
| II | 0 | 0/1 | 0 | 0/1 | 25 | 1/4 | | | 50 | 2/4 | 5 | 6/113 | 45 | 49/110 | 67 | 2/3 | 0 | 0/2 | 57 | 4/7 |
| III | | | | | 40 | 2/5 | | | 50 | 2/4 | 11 | 2/19 | 89 | 24/27 | 60 | 3/5 | 100 | 25/25 | 50 | 1/2 |
| IV | 0 | 0/1 | 100 | 3/3 | 67 | 4/6 | | | 82 | 9/11 | 88 | 15/17 | 100 | 8/8 | 67 | 2/3 | 100 | 6/6 | 100 | 1/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 46 | 5/11 | 71 | 10/14 | 92 | 57/62 | 83 | 100/121 | 86 | 25/29 | 85 | 81/95 | 85 | 57/67 | 74 | 25/34 | 71 | 105/147 | 74 | 194/261 |
| I | 25 | 2/8 | 50 | 2/4 | 86 | 6/7 | 46 | 6/13 | 60 | 3/5 | 60 | 9/15 | 22 | 2/9 | 50 | 5/10 | 27 | 4/15 | 20 | 12/60 |
| II | 100 | 1/1 | 60 | 3/5 | 92 | 12/13 | 77 | 17/22 | 86 | 6/7 | 80 | 12/15 | 83 | 10/12 | 69 | 9/13 | 82 | 22/27 | 87 | 20/23 |
| III | 100 | 2/2 | 100 | 5/5 | 94 | 15/16 | 85 | 35/41 | 86 | 6/7 | 84 | 16/19 | 95 | 18/19 | 100 | 11/11 | 85 | 23/27 | 89 | 64/72 |
| IV | | | | | 92 | 24/26 | 93 | 42/45 | 100 | 10/10 | 96 | 44/46 | 100 | 27/27 | | | 80 | 31/39 | 93 | 98/106 |

TABLE 27. Classification sensitivity with 99.0% specificity using the target genomic regions of List 6.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 20 | 11/56 | 9 | 17/188 | 29 | 71/247 | 18 | 15/84 | 87 | 32/37 | 44 | 7/16 |
| I | 0 | 0/2 | 0 | 0/3 | 50 | 1/2 | | | 0 | 0/37 | 0 | 0/39 | 3 | 3/102 | 14 | 10/73 | 25 | 1/4 | 67 | 4/6 |
| II | 0 | 0/1 | 0 | 0/1 | 0 | 0/4 | | | 0 | 0/4 | 3 | 3/113 | 34 | 37/110 | 33 | 1/3 | 0 | 0/2 | 43 | 3/7 |
| III | | | | | 60 | 3/5 | | | 50 | 2/4 | 11 | 2/19 | 85 | 23/27 | 60 | 3/5 | 100 | 25/25 | 0 | 0/2 |
| IV | 0 | 0/1 | 100 | 3/3 | 33 | 2/6 | | | 82 | 9/11 | 71 | 12/17 | 100 | 8/8 | 33 | 1/3 | 100 | 6/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 81 | 50/62 | 68 | 82/121 | 86 | 25/29 | 70 | 66/95 | 69 | 46/67 | 79 | 27/34 | 72 | 106/147 | 65 | 169/261 |
| I | 13 | 1/8 | 25 | 1/4 | 86 | 6/7 | 15 | 2/13 | 60 | 3/5 | 27 | 4/15 | 11 | 1/9 | 60 | 6/10 | 20 | 3/15 | 12 | 7/60 |
| II | 100 | 1/1 | 60 | 3/5 | 77 | 10/13 | 41 | 9/22 | 86 | 6/7 | 67 | 10/15 | 58 | 7/12 | 77 | 10/13 | 89 | 24/27 | 65 | 15/23 |
| III | 50 | 1/2 | 100 | 5/5 | 69 | 11/16 | 71 | 29/41 | 86 | 6/7 | 63 | 12/19 | 68 | 13/19 | 100 | 11/11 | 82 | 22/27 | 76 | 55/72 |
| IV | | | | | 89 | 23/26 | 93 | 42/45 | 100 | 10/10 | 87 | 40/46 | 93 | 25/27 | | | 80 | 31/39 | 87 | 92/106 |

**TABLE 28.** Classification sensitivity with 99.0% specificity using the target genomic regions of List 7.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 53 | 9/17 | 0 | 0/4 | 23 | 13/56 | 12 | 23/188 | 35 | 86/247 | 30 | 25/84 | 89 | 33/37 | 63 | 10/16 |
| I | 0 | 0/2 | 0 | 0/3 | 100 | 2/2 | | | 0 | 0/37 | 5 | 2/39 | 5 | 5/102 | 25 | 18/73 | 75 | 3/4 | 67 | 4/6 |
| II | 0 | 0/1 | 0 | 0/1 | 25 | 1/4 | | | 50 | 2/4 | 4 | 5/113 | 45 | 49/110 | 67 | 2/3 | 0 | 0/2 | 57 | 4/7 |
| III | | | | | 40 | 2/5 | | | 50 | 2/4 | 11 | 2/19 | 89 | 24/27 | 60 | 3/5 | 96 | 24/25 | 50 | 1/2 |
| IV | 0 | 0/1 | 100 | 3/3 | 67 | 4/6 | | | 82 | 9/11 | 82 | 14/17 | 100 | 8/8 | 67 | 2/3 | 100 | 6/6 | 100 | 1/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 36 | 4/11 | 71 | 10/14 | 94 | 58/62 | 81 | 98/121 | 86 | 25/29 | 86 | 82/95 | 85 | 57/67 | 79 | 27/34 | 72 | 106/147 | 74 | 194/261 |
| I | 13 | 1/8 | 50 | 2/4 | 100 | 7/7 | 46 | 6/13 | 60 | 3/5 | 60 | 9/15 | 22 | 2/9 | 60 | 6/10 | 33 | 5/15 | 20 | 12/60 |
| II | 100 | 1/1 | 60 | 3/5 | 92 | 12/13 | 73 | 16/22 | 86 | 6/7 | 87 | 13/15 | 83 | 10/12 | 77 | 10/13 | 82 | 22/27 | 87 | 20/23 |
| III | 100 | 2/2 | 100 | 5/5 | 94 | 15/16 | 83 | 34/41 | 86 | 6/7 | 84 | 16/19 | 95 | 18/19 | 100 | 11/11 | 85 | 23/27 | 89 | 64/72 |
| IV | | | | | 92 | 24/26 | 93 | 42/45 | 100 | 10/10 | 96 | 44/46 | 100 | 27/27 | | | 80 | 31/39 | 93 | 98/106 |

**TABLE 29.** Classification sensitivity with 99.0% specificity using the target genomic regions of List 8.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 25 | 1/4 | 43 | 3/7 | 41 | 7/17 | 25 | 1/4 | 20 | 11/56 | 11 | 20/188 | 26 | 64/247 | 16 | 13/84 | 84 | 31/37 | 44 | 7/16 |
| I | 0 | 0/2 | 0 | 0/3 | 100 | 2/2 | | | 0 | 0/37 | 5 | 2/39 | 2 | 2/102 | 11 | 8/73 | 25 | 1/4 | 33 | 2/6 |
| II | 0 | 0/1 | 0 | 0/1 | 0 | 0/4 | | | 0 | 0/4 | 3 | 3/113 | 29 | 32/110 | 33 | 1/3 | 0 | 0/2 | 57 | 4/7 |
| III | | | | | 40 | 2/5 | | | 50 | 2/4 | 11 | 2/19 | 85 | 23/27 | 60 | 3/5 | 96 | 24/25 | 50 | 1/2 |
| IV | 100 | 1/1 | 100 | 3/3 | 50 | 3/6 | | | 82 | 9/11 | 77 | 13/17 | 88 | 7/8 | 33 | 1/3 | 100 | 6/6 | 0 | 0/1 |

64

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 71 | 10/14 | 81 | 50/62 | 59 | 71/121 | 83 | 24/29 | 64 | 61/95 | 69 | 46/67 | 79 | 27/34 | 69 | 102/147 | 62 | 161/261 |
| I | 13 | 1/8 | 50 | 2/4 | 100 | 7/7 | 15 | 2/13 | 40 | 2/5 | 27 | 4/15 | 11 | 1/9 | 60 | 6/10 | 27 | 4/15 | 12 | 7/60 |
| II | 100 | 1/1 | 60 | 3/5 | 77 | 10/13 | 23 | 5/22 | 86 | 6/7 | 53 | 8/15 | 50 | 6/12 | 77 | 10/13 | 85 | 23/27 | 57 | 13/23 |
| III | 50 | 1/2 | 100 | 5/5 | 63 | 10/16 | 56 | 23/41 | 86 | 6/7 | 47 | 9/19 | 68 | 13/19 | 100 | 11/11 | 74 | 20/27 | 74 | 53/72 |
| IV | | | | | 89 | 23/26 | 91 | 41/45 | 100 | 10/10 | 87 | 40/46 | 96 | 26/27 | | | 80 | 31/39 | 83 | 88/106 |

TABLE 30. Classification sensitivity with 99.0% specificity using the target genomic regions of List 9.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 21 | 12/56 | 10 | 19/188 | 27 | 66/247 | 17 | 14/84 | 81 | 30/37 | 44 | 7/16 |
| I | 0 | 0/2 | 0 | 0/3 | 50 | 1/2 | | | 3 | 1/37 | 0 | 0/39 | 3 | 3/102 | 12 | 9/73 | 25 | 1/4 | 33 | 2/6 |
| II | 0 | 0/1 | 0 | 0/1 | 0 | 0/4 | | | 0 | 0/4 | 4 | 4/113 | 31 | 34/110 | 33 | 1/3 | 0 | 0/2 | 57 | 4/7 |
| III | | | | | 40 | 2/5 | | | 50 | 2/4 | 11 | 2/19 | 82 | 22/27 | 60 | 3/5 | 92 | 23/25 | 50 | 1/2 |
| IV | 0 | 0/1 | 100 | 3/3 | 50 | 3/6 | | | 82 | 9/11 | 77 | 13/17 | 88 | 7/8 | 33 | 1/3 | 100 | 6/6 | 0 | 0/1 |

EP 4 640 843 A2

| Stage | Cervical % | Fxn | Anorectal % | Fxn | Head & Neck % | Fxn | Colorectal % | Fxn | Liver % | Fxn | Pancreas & Gallbladder % | Fxn | Upper GI % | Fxn | Multiple Myeloma % | Fxn | Lymphoid Neoplasm % | Fxn | Lung % | Fxn |
|---|---|---|---|---|---|---|---|---|---|---|---|---|---|---|---|---|---|---|---|---|
| All | 27 | 3/11 | 64 | 9/14 | 81 | 50/62 | 61 | 74/121 | 83 | 24/29 | 68 | 65/95 | 69 | 46/67 | 77 | 26/34 | 69 | 102/147 | 63 | 165/261 |
| I | 13 | 1/8 | 25 | 1/4 | 86 | 6/7 | 15 | 2/13 | 40 | 2/5 | 27 | 4/15 | 11 | 1/9 | 60 | 6/10 | 20 | 3/15 | 10 | 6/60 |
| II | 100 | 1/1 | 60 | 3/5 | 77 | 10/13 | 32 | 7/22 | 86 | 6/7 | 67 | 10/15 | 58 | 7/12 | 69 | 9/13 | 85 | 23/27 | 65 | 15/23 |
| III | 50 | 1/2 | 100 | 5/5 | 69 | 11/16 | 61 | 25/41 | 86 | 6/7 | 58 | 11/19 | 68 | 13/19 | 100 | 11/11 | 70 | 19/27 | 74 | 53/72 |
| IV | | | | | 89 | 23/26 | 89 | 40/45 | 100 | 10/10 | 87 | 40/46 | 93 | 25/27 | | | 82 | 32/39 | 86 | 91/106 |

**TABLE 31.** Classification sensitivity with 99.0% specificity using the target genomic regions of List 10.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 25 | 1/4 | 20 | 11/56 | 10 | 18/188 | 28 | 69/247 | 19 | 16/84 | 84 | 31/37 | 38 | 6/16 |
| I | 0 | 0/2 | 0 | 0/3 | 50 | 1/2 | | | 0 | 0/37 | 3 | 1/39 | 3 | 3/102 | 15 | 11/73 | 25 | 1/4 | 33 | 2/6 |
| II | 0 | 0/1 | 0 | 0/1 | 0 | 0/4 | | | 0 | 0/4 | 3 | 3/113 | 34 | 37/110 | 33 | 1/3 | 0 | 0/2 | 43 | 3/7 |
| III | | | | | 40 | 2/5 | | | 50 | 2/4 | 11 | 2/19 | 85 | 23/27 | 60 | 3/5 | 96 | 24/25 | 50 | 1/2 |
| IV | 0 | 0/1 | 100 | 3/3 | 33 | 2/6 | | | 82 | 9/11 | 71 | 12/17 | 75 | 6/8 | 33 | 1/3 | 100 | 6/6 | 0 | 0/1 |

EP 4 640 843 A2

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 82 | 51/62 | 68 | 82/121 | 86 | 25/29 | 72 | 68/95 | 70 | 47/67 | 79 | 27/34 | 69 | 102/147 | 67 | 174/261 |
| I | 13 | 1/8 | 25 | 1/4 | 86 | 6/7 | 15 | 2/13 | 60 | 3/5 | 33 | 5/15 | 11 | 1/9 | 60 | 6/10 | 27 | 4/15 | 17 | 10/60 |
| II | 100 | 1/1 | 60 | 3/5 | 77 | 10/13 | 41 | 9/22 | 86 | 6/7 | 67 | 10/15 | 67 | 8/12 | 77 | 10/13 | 89 | 24/27 | 70 | 16/23 |
| III | 50 | 1/2 | 100 | 5/5 | 75 | 12/16 | 71 | 29/41 | 86 | 6/7 | 68 | 13/19 | 63 | 44184 | 100 | 11/11 | 74 | 20/27 | 76 | 55/72 |
| IV | | | | | 89 | 23/26 | 93 | 42/45 | 100 | 10/10 | 87 | 40/46 | 96 | 26/27 | | | 80 | 31/39 | 88 | 93/106 |

TABLE 32. Classification sensitivity with 99.0% specificity using the target genomic regions of List 11.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 20 | 11/56 | 10 | 18/188 | 28 | 69/247 | 18 | 15/84 | 84 | 31/37 | 38 | 6/16 |
| I | 0 | 0/2 | 0 | 0/3 | 50 | 1/2 | | | 0 | 0/37 | 0 | 0/39 | 4 | 4/102 | 14 | 10/73 | 25 | 1/4 | 33 | 2/6 |
| II | 0 | 0/1 | 0 | 0/1 | 0 | 0/4 | | | 0 | 0/4 | 3 | 3/113 | 33 | 36/110 | 33 | 1/3 | 0 | 0/2 | 43 | 3/7 |
| III | | | | | 60 | 3/5 | | | 50 | 2/4 | 11 | 2/19 | 85 | 23/27 | 60 | 3/5 | 96 | 24/25 | 50 | 1/2 |
| IV | 0 | 0/1 | 100 | 3/3 | 33 | 2/6 | | | 82 | 9/11 | 77 | 13/17 | 75 | 6/8 | 33 | 1/3 | 100 | 6/6 | 0 | 0/1 |

| Stage | Cervical % | Cervical Fxn | Anorectal % | Anorectal Fxn | Head & Neck % | Head & Neck Fxn | Colorectal % | Colorectal Fxn | Liver % | Liver Fxn | Pancreas & Gallbladder % | Pancreas & Gallbladder Fxn | Upper GI % | Upper GI Fxn | Multiple Myeloma % | Multiple Myeloma Fxn | Lymphoid Neoplasm % | Lymphoid Neoplasm Fxn | Lung % | Lung Fxn |
|---|---|---|---|---|---|---|---|---|---|---|---|---|---|---|---|---|---|---|---|---|
| All | 27 | 3/11 | 64 | 9/14 | 84 | 52/62 | 65 | 78/121 | 86 | 25/29 | 71 | 67/95 | 70 | 47/67 | 79 | 27/34 | 70 | 103/147 | 66 | 171/261 |
| I | 13 | 1/8 | 25 | 1/4 | 86 | 6/7 | 8 | 1/13 | 60 | 3/5 | 33 | 5/15 | 11 | 1/9 | 60 | 6/10 | 20 | 3/15 | 13 | 8/60 |
| II | 100 | 1/1 | 60 | 3/5 | 77 | 10/13 | 27 | 6/22 | 86 | 6/7 | 67 | 10/15 | 67 | 8/12 | 77 | 10/13 | 85 | 23/27 | 65 | 15/23 |
| III | 50 | 1/2 | 100 | 5/5 | 75 | 12/16 | 71 | 29/41 | 86 | 6/7 | 63 | 12/19 | 68 | 13/19 | 100 | 11/11 | 74 | 20/27 | 78 | 56/72 |
| IV | | | | | 92 | 24/26 | 93 | 42/45 | 100 | 10/10 | 87 | 40/46 | 93 | 25/27 | | | 80 | 31/39 | 87 | 92/106 |

**TABLE 33.** Classification sensitivity with 99.0% specificity using the target genomic regions of List 12.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 20 | 11/56 | 10 | 18/188 | 28 | 69/247 | 18 | 15/84 | 87 | 32/37 | 44 | 7/16 |
| I | 0 | 0/2 | 0 | 0/3 | 50 | 1/2 | | | 0 | 0/37 | 0 | 0/39 | 2 | 2/102 | 14 | 10/73 | 25 | 1/4 | 50 | 3/6 |
| II | 0 | 0/1 | 0 | 0/1 | 0 | 0/4 | | | 0 | 0/4 | 4 | 4/113 | 35 | 38/110 | 33 | 1/3 | 0 | 0/2 | 43 | 3/7 |
| III | | | | | 60 | 3/5 | | | 50 | 2/4 | 11 | 2/19 | 85 | 23/27 | 60 | 3/5 | 100 | 25/25 | 50 | 1/2 |
| IV | 0 | 0/1 | 100 | 3/3 | 33 | 2/6 | | | 82 | 9/11 | 71 | 12/17 | 75 | 6/8 | 33 | 1/3 | 100 | 6/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 81 | 50/62 | 66 | 80/121 | 86 | 25/29 | 76 | 72/95 | 70 | 47/67 | 79 | 27/34 | 69 | 101/147 | 65 | 169/261 |
| I | 13 | 1/8 | 25 | 1/4 | 86 | 6/7 | 15 | 2/13 | 60 | 3/5 | 40 | 6/15 | 11 | 1/9 | 60 | 6/10 | 20 | 3/15 | 13 | 8/60 |
| II | 100 | 1/1 | 60 | 3/5 | 77 | 10/13 | 36 | 8/22 | 86 | 6/7 | 73 | 11/15 | 67 | 8/12 | 77 | 10/13 | 85 | 23/27 | 65 | 15/23 |
| III | 50 | 1/2 | 100 | 5/5 | 69 | 11/16 | 68 | 28/41 | 86 | 6/7 | 74 | 14/19 | 68 | 13/19 | 100 | 11/11 | 74 | 20/27 | 75 | 54/72 |
| IV | | | | | 89 | 23/26 | 93 | 42/45 | 100 | 10/10 | 89 | 41/46 | 93 | 25/27 | | | 80 | 31/39 | 87 | 92/106 |

TABLE 34. Classification sensitivity with 99.0% specificity using the target genomic regions of List 13.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 14 | 8/56 | 9 | 16/188 | 21 | 52/247 | 11 | 9/84 | 62 | 23/37 | 38 | 6/16 |
| I | 0 | 0/2 | 0 | 0/3 | 50 | 1/2 | | | 0 | 0/37 | 0 | 0/39 | 1 | 1/102 | 8 | 6/73 | 25 | 1/4 | 50 | 3/6 |
| II | 0 | 0/1 | 0 | 0/1 | 0 | 0/4 | | | 0 | 0/4 | 2 | 2/113 | 25 | 27/110 | 33 | 1/3 | 0 | 0/2 | 43 | 3/7 |
| III | | | | | 40 | 2/5 | | | 25 | 1/4 | 11 | 2/19 | 67 | 18/27 | 20 | 1/5 | 68 | 17/25 | 0 | 0/2 |
| IV | 0 | 0/1 | 100 | 3/3 | 50 | 3/6 | | | 64 | 7/11 | 71 | 12/17 | 75 | 6/8 | 33 | 1/3 | 83 | 5/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 71 | 44/62 | 55 | 66/121 | 83 | 24/29 | 60 | 57/95 | 54 | 36/67 | 65 | 22/34 | 62 | 91/147 | 59 | 154/261 |
| I | 13 | 1/8 | 25 | 1/4 | 86 | 6/7 | 8 | 1/13 | 40 | 2/5 | 7 | 1/15 | 0 | 0/9 | 40 | 4/10 | 20 | 3/15 | 8 | 5/60 |
| II | 100 | 1/1 | 60 | 3/5 | 77 | 10/13 | 18 | 4/22 | 86 | 6/7 | 60 | 9/15 | 33 | 4/12 | 54 | 7/13 | 89 | 24/27 | 57 | 13/23 |
| III | 50 | 1/2 | 100 | 5/5 | 44 | 7/16 | 56 | 23/41 | 86 | 6/7 | 37 | 7/19 | 37 | 7/19 | 100 | 11/11 | 70 | 19/27 | 68 | 49/72 |
| IV | | | | | 81 | 21/26 | 84 | 38/45 | 100 | 10/10 | 87 | 40/46 | 93 | 25/27 | | | 77 | 30/39 | 82 | 87/106 |

TABLE 35. Classification sensitivity with 99.0% specificity using the target genomic regions of List 14.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 50 | 2/4 | 16 | 9/56 | 9 | 16/188 | 22 | 55/247 | 12 | 10/84 | 65 | 24/37 | 38 | 6/16 |
| I | 0 | 0/2 | 0 | 0/3 | 50 | 1/2 | | | 0 | 0/37 | 0 | 0/39 | 1 | 1/102 | 8 | 6/73 | 25 | 1/4 | 50 | 3/6 |
| II | 0 | 0/1 | 0 | 0/1 | 0 | 0/4 | | | 0 | 0/4 | 2 | 2/113 | 26 | 28/110 | 33 | 1/3 | 0 | 0/2 | 43 | 3/7 |
| III | | | | | 40 | 2/5 | | | 25 | 1/4 | 11 | 2/19 | 74 | 20/27 | 40 | 2/5 | 72 | 18/25 | 0 | 0/2 |
| IV | 0 | 0/1 | 100 | 3/3 | 33 | 2/6 | | | 73 | 8/11 | 71 | 12/17 | 75 | 6/8 | 33 | 1/3 | 83 | 5/6 | 0 | 0/1 |

EP 4 640 843 A2

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 79 | 49/62 | 58 | 70/121 | 83 | 24/29 | 57 | 54/95 | 60 | 40/67 | 65 | 22/34 | 67 | 99/147 | 58 | 151/261 |
| I | 13 | 1/8 | 25 | 1/4 | 86 | 6/7 | 8 | 1/13 | 40 | 2/5 | 7 | 1/15 | 0 | 0/9 | 40 | 4/10 | 20 | 3/15 | 5 | 3/60 |
| II | 100 | 1/1 | 60 | 3/5 | 77 | 10/13 | 18 | 4/22 | 86 | 6/7 | 53 | 8/15 | 42 | 5/12 | 54 | 7/13 | 85 | 23/27 | 61 | 14/23 |
| III | 50 | 1/2 | 100 | 5/5 | 63 | 10/16 | 61 | 25/41 | 86 | 6/7 | 42 | 8/19 | 53 | 10/19 | 100 | 11/11 | 70 | 19/27 | 67 | 48/72 |
| IV | | | | | 89 | 23/26 | 89 | 40/45 | 100 | 10/10 | 80 | 37/46 | 93 | 25/27 | | | 80 | 31/39 | 81 | 86/106 |

TABLE 36. Classification sensitivity with 99.0% specificity using the target genomic regions of List 15.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 35 | 6/17 | 25 | 1/4 | 16 | 9/56 | 9 | 17/188 | 23 | 56/247 | 13 | 11/84 | 65 | 24/37 | 38 | 6/16 |
| I | 0 | 0/2 | 0 | 0/3 | 50 | 1/2 | | | 0 | 0/37 | 0 | 0/39 | 1 | 1/102 | 8 | 6/73 | 25 | 1/4 | 50 | 3/6 |
| II | 0 | 0/1 | 0 | 0/1 | 0 | 0/4 | | | 0 | 0/4 | 3 | 3/113 | 27 | 30/110 | 33 | 1/3 | 0 | 0/2 | 43 | 3/7 |
| III | | | | | 40 | 2/5 | | | 25 | 1/4 | 11 | 2/19 | 70 | 19/27 | 60 | 3/5 | 72 | 18/25 | 0 | 0/2 |
| IV | 0 | 0/1 | 100 | 3/3 | 50 | 3/6 | | | 73 | 8/11 | 71 | 12/17 | 75 | 6/8 | 33 | 1/3 | 83 | 5/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 81 | 50/62 | 60 | 73/121 | 86 | 25/29 | 60 | 57/95 | 63 | 42/67 | 68 | 23/34 | 70 | 103/147 | 59 | 154/261 |
| I | 13 | 1/8 | 25 | 1/4 | 86 | 6/7 | 15 | 2/13 | 60 | 3/5 | 20 | 3/15 | 11 | 1/9 | 50 | 5/10 | 27 | 4/15 | 5 | 3/60 |
| II | 100 | 1/1 | 60 | 3/5 | 77 | 10/13 | 27 | 6/22 | 86 | 6/7 | 53 | 8/15 | 50 | 6/12 | 54 | 7/13 | 85 | 23/27 | 61 | 14/23 |
| III | 50 | 1/2 | 100 | 5/5 | 69 | 11/16 | 61 | 25/41 | 86 | 6/7 | 37 | 7/19 | 53 | 10/19 | 100 | 11/11 | 70 | 19/27 | 69 | 50/72 |
| IV | | | | | 89 | 23/26 | 89 | 40/45 | 100 | 10/10 | 85 | 39/46 | 93 | 25/27 | | | 80 | 31/39 | 82 | 87/106 |

TABLE 37. Classification sensitivity with 99.0% specificity using the target genomic regions of List 16.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 41 | 7/17 | 25 | 1/4 | 16 | 9/56 | 9 | 16/188 | 25 | 62/247 | 13 | 11/84 | 73 | 27/37 | 44 | 7/16 |
| I | 0 | 0/2 | 0 | 0/3 | 50 | 1/2 | | | 0 | 0/37 | 0 | 0/39 | 1 | 1/102 | 8 | 6/73 | 25 | 1/4 | 67 | 4/6 |
| II | 0 | 0/1 | 0 | 0/1 | 0 | 0/4 | | | 0 | 0/4 | 2 | 2/113 | 30 | 33/110 | 33 | 1/3 | 0 | 0/2 | 43 | 3/7 |
| III | | | | | 60 | 3/5 | | | 25 | 1/4 | 11 | 2/19 | 74 | 20/27 | 60 | 3/5 | 80 | 20/25 | 0 | 0/2 |
| IV | 0 | 0/1 | 100 | 3/3 | 50 | 3/6 | | | 73 | 8/11 | 71 | 12/17 | 100 | 8/8 | 33 | 1/3 | 100 | 6/6 | 0 | 0/1 |

| Stage | Cervical % | Fxn | Anorectal % | Fxn | Head & Neck % | Fxn | Colorectal % | Fxn | Liver % | Fxn | Pancreas & Gallbladder % | Fxn | Upper GI % | Fxn | Multiple Myeloma % | Fxn | Lymphoid Neoplasm % | Fxn | Lung % | Fxn |
|---|---|---|---|---|---|---|---|---|---|---|---|---|---|---|---|---|---|---|---|---|
| All | 27 | 3/11 | 64 | 9/14 | 81 | 50/62 | 62 | 75/121 | 86 | 25/29 | 64 | 61/95 | 64 | 43/67 | 68 | 23/34 | 71 | 104/147 | 60 | 156/261 |
| I | 13 | 1/8 | 25 | 1/4 | 86 | 6/7 | 15 | 2/13 | 60 | 3/5 | 20 | 3/15 | 0 | 0/9 | 40 | 4/10 | 27 | 4/15 | 7 | 4/60 |
| II | 100 | 1/1 | 60 | 3/5 | 77 | 10/13 | 27 | 6/22 | 86 | 6/7 | 60 | 9/15 | 58 | 7/12 | 62 | 8/13 | 85 | 23/27 | 61 | 14/23 |
| III | 50 | 1/2 | 100 | 5/5 | 69 | 11/16 | 63 | 26/41 | 86 | 6/7 | 53 | 10/19 | 58 | 11/19 | 100 | 11/11 | 74 | 20/27 | 71 | 51/72 |
| IV |  |  |  |  | 89 | 23/26 | 91 | 41/45 | 100 | 10/10 | 85 | 39/46 | 93 | 25/27 |  |  | 80 | 31/39 | 82 | 87/106 |

**TABLE 38.** Classification sensitivity with 99.0% specificity using a randomly selected subset of 10% of the target genomic regions of List 12.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 0 | 0/4 | 16 | 9/56 | 9 | 16/188 | 25 | 62/247 | 11 | 9/84 | 65 | 24/37 | 38 | 6/16 |
| I | 0 | 0/2 | 0 | 0/3 | 50 | 1/2 | | | 0 | 0/37 | 0 | 0/39 | 2 | 2/102 | 7 | 5/73 | 25 | 1/4 | 33 | 2/6 |
| II | 0 | 0/1 | 0 | 0/1 | 0 | 0/4 | | | 0 | 0/4 | 2 | 2/113 | 29 | 32/110 | 33 | 1/3 | 0 | 0/2 | 43 | 3/7 |
| III | | | | | 40 | 2/5 | | | 25 | 1/4 | 11 | 2/19 | 78 | 21/27 | 40 | 2/5 | 68 | 17/25 | 0 | 0/2 |
| IV | 0 | 0/1 | 100 | 3/3 | 33 | 2/6 | | | 73 | 8/11 | 71 | 12/17 | 88 | 7/8 | 33 | 1/3 | 100 | 6/6 | 100 | 1/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 77 | 48/62 | 59 | 71/121 | 86 | 25/29 | 62 | 59/95 | 60 | 40/67 | 74 | 25/34 | 65 | 95/147 | 59 | 154/261 |
| I | 13 | 1/8 | 25 | 1/4 | 86 | 6/7 | 8 | 1/13 | 60 | 3/5 | 33 | 5/15 | 0 | 0/9 | 50 | 5/10 | 20 | 3/15 | 7 | 4/60 |
| II | 100 | 1/1 | 60 | 3/5 | 77 | 10/13 | 32 | 7/22 | 86 | 6/7 | 53 | 8/15 | 42 | 5/12 | 69 | 9/13 | 89 | 24/27 | 48 | 11/23 |
| III | 50 | 1/2 | 100 | 5/5 | 56 | 9/16 | 56 | 23/41 | 86 | 6/7 | 42 | 8/19 | 47 | 9/19 | 100 | 11/11 | 70 | 19/27 | 71 | 51/72 |
| IV | | | | | 89 | 23/26 | 89 | 40/45 | 100 | 10/10 | 83 | 38/46 | 96 | 26/27 | | | 80 | 31/39 | 83 | 88/106 |

**TABLE 39.** Classification sensitivity with 99.0% specificity using a randomly selected subset of 25% of the target genomic regions of List 12.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 25 | 1/4 | 20 | 11/56 | 9 | 17/188 | 27 | 66/247 | 14 | 12/84 | 78 | 29/37 | 44 | 7/16 |
| I | 0 | 0/2 | 0 | 0/3 | 50 | 1/2 | | | 0 | 0/37 | 0 | 0/39 | 2 | 2/102 | 10 | 7/73 | 25 | 1/4 | 50 | 3/6 |
| II | 0 | 0/1 | 0 | 0/1 | 0 | 0/4 | | | 0 | 0/4 | 3 | 3/113 | 32 | 35/110 | 33 | 1/3 | 0 | 0/2 | 43 | 3/7 |
| III | | | | | 40 | 2/5 | | | 50 | 2/4 | 11 | 2/19 | 82 | 22/27 | 60 | 3/5 | 88 | 22/25 | 0 | 0/2 |
| IV | 0 | 0/1 | 100 | 3/3 | 33 | 2/6 | | | 82 | 9/11 | 71 | 12/17 | 88 | 7/8 | 33 | 1/3 | 100 | 6/6 | 100 | 1/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 71 | 10/14 | 79 | 49/62 | 61 | 74/121 | 86 | 25/29 | 65 | 62/95 | 64 | 43/67 | 68 | 23/34 | 67 | 98/147 | 62 | 161/261 |
| I | 13 | 1/8 | 50 | 2/4 | 86 | 6/7 | 15 | 2/13 | 60 | 3/5 | 33 | 5/15 | 11 | 1/9 | 50 | 5/10 | 27 | 4/15 | 7 | 4/60 |
| II | 100 | 1/1 | 60 | 3/5 | 77 | 10/13 | 27 | 6/22 | 86 | 6/7 | 73 | 11/15 | 33 | 4/12 | 54 | 7/13 | 85 | 23/27 | 61 | 14/23 |
| III | 50 | 1/2 | 100 | 5/5 | 63 | 10/16 | 59 | 24/41 | 86 | 6/7 | 42 | 8/19 | 63 | 12/19 | 100 | 11/11 | 70 | 19/27 | 72 | 52/72 |
| IV | | | | | 89 | 23/26 | 93 | 42/45 | 100 | 10/10 | 83 | 38/46 | 96 | 26/27 | | | 80 | 31/39 | 86 | 91/106 |

**TABLE 40.** Classification sensitivity with 99.0% specificity using a randomly selected subset of 50% of the target genomic regions of List 4.

| Stage | Thyroid | | Melanoma | | Sarcoma | | Myeloid Neoplasm | | Renal | | Prostate | | Breast | | Uterine | | Ovary | | Bladder & Urothecal | |
|---|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 0 | 0/4 | 43 | 3/7 | 29 | 5/17 | 0 | 0/4 | 18 | 10/56 | 8 | 15/188 | 25 | 62/247 | 17 | 14/84 | 81 | 30/37 | 31 | 5/16 |
| I | 0 | 0/2 | 0 | 0/3 | 50 | 1/2 | | | 0 | 0/37 | 0 | 0/39 | 1 | 1/102 | 12 | 9/73 | 25 | 1/4 | 33 | 2/6 |
| II | 0 | 0/1 | 0 | 0/1 | 0 | 0/4 | | | 0 | 0/4 | 1 | 1/113 | 29 | 32/110 | 33 | 1/3 | 0 | 0/2 | 43 | 3/7 |
| III | | | | | 40 | 2/5 | | | 50 | 2/4 | 11 | 2/19 | 82 | 22/27 | 60 | 3/5 | 92 | 23/25 | 0 | 0/2 |
| IV | 0 | 0/1 | 100 | 3/3 | 33 | 2/6 | | | 73 | 8/11 | 71 | 12/17 | 88 | 7/8 | 33 | 1/3 | 100 | 6/6 | 0 | 0/1 |

| Stage | Cervical | | Anorectal | | Head & Neck | | Colorectal | | Liver | | Pancreas & Gallbladder | | Upper GI | | Multiple Myeloma | | Lymphoid Neoplasm | | Lung | |
|---|---|---|---|---|---|---|---|---|---|---|---|---|---|---|---|---|---|---|---|---|
| | % | Fxn | % | Fxn | % | Fxn | % | Fxn | % | Fxn | % | Fxn | % | Fxn | % | Fxn | % | Fxn | % | Fxn |
| All | 27 | 3/11 | 64 | 9/14 | 76 | 47/62 | 63 | 76/121 | 86 | 25/29 | 60 | 57/95 | 66 | 44/67 | 74 | 25/34 | 69 | 101/147 | 63 | 165/261 |
| I | 13 | 1/8 | 25 | 1/4 | 86 | 6/7 | 8 | 1/13 | 60 | 3/5 | 20 | 3/15 | 11 | 1/9 | 50 | 5/10 | 20 | 3/15 | 13 | 8/60 |
| II | 100 | 1/1 | 60 | 3/5 | 77 | 10/13 | 41 | 9/22 | 86 | 6/7 | 60 | 9/15 | 50 | 6/12 | 69 | 9/13 | 85 | 23/27 | 61 | 14/23 |
| III | 50 | 1/2 | 100 | 5/5 | 56 | 9/16 | 63 | 26/41 | 86 | 6/7 | 37 | 7/19 | 58 | 11/19 | 100 | 11/11 | 67 | 18/27 | 71 | 51/72 |
| IV | | | | | 85 | 22/26 | 89 | 40/45 | 100 | 10/10 | 83 | 38/46 | 96 | 26/27 | | | 77 | 30/39 | 87 | 92/106 |

**EXAMPLE 6 - Detection of cancer using cancer assay panel**

Blood samples are collected from a group of individuals previously diagnosed with cancer of a TOO ("test group"), and other groups of individuals without cancer or diagnosed with a different type of cancer ("other group"). cfDNA fragments are extracted from the blood samples and treated with bisulfite to convert unmethylated cytosines to uracils. The cancer assay panel described herein was applied to the bisulfite treated samples. Unbound cfDNA fragments are washed and cfDNA fragments bound to the probes are collected. The collected cfDNA fragments are amplified and sequenced. The sequence reads confirm that the probes specifically enrich cfDNA fragments having methylation patterns indicative of cancer of a TOO and samples from the test group include significantly more of the differentially methylated cfDNA fragments compared to other groups.

While preferred embodiments of the present disclosure have been shown and described herein, it will be obvious to those skilled in the art that such embodiments are provided by way of example only. Numerous variations, changes, and substitutions will now occur to those skilled in the art without departing from the disclosure. It should be understood that various alternatives to the embodiments of the disclosure described herein may be employed in practicing the disclosure. It is intended that the following claims define the scope of the disclosure and that methods and structures within the scope of these claims and their equivalents be covered thereby.

Exemplary embodiments of the invention are disclosed herein as embodiment E1 to embodiment E263:

E1. A composition comprising a plurality of different bait oligonucleotides,

wherein the plurality of different bait oligonucleotides is configured to collectively hybridize to DNA molecules derived from at least 200 target genomic regions,
wherein each genomic region of the at least 200 target genomic regions is differentially methylated in at least one cancer type relative to another cancer type or relative to a non-cancer type, and
wherein the at least 200 target genomic regions comprise, for at least 80% of all possible pairs of cancer types selected from a set comprising at least 10 cancer types, at least one target genomic region that is differentially methylated between the pair of cancer types.

E2. The composition of E1, wherein the at least 10 cancer types comprise at least 2, 3, 4, 5, 10, 12, 14, 16, 18, or 20 cancer types.

E3. The composition of any one of E1-E2, wherein the cancer types are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer.

E4. The composition of any one of E1-E2, wherein the cancer types are selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer.

E5. The composition of any one of E1-E2, wherein the cancer types are selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper GI cancer, multiple myeloma, lymphoid neoplasm, and lung cancer.

E6. The composition of any one of E1-E5, wherein the at least 200 target genomic regions are selected from any one of lists 1-16.

E7. The composition of any one of E1-E6, wherein the at least 200 target genomic regions comprise at least 20%, 30%, 40%, 50%, 60%, 70%, 80%, 90% or 95% of the target genomic regions in any one of lists 1-16.

E8. The composition of any one of E1-E7, wherein the at least 200 target genomic regions comprise at least 500, 1,000, 5,000, 10,000, 15,000, 20,000, 30,000, 40,000, or 50,000 target genomic regions in any one of lists 1-16.

E9. The composition of any one of E1-E5, wherein the at least 200 target genomic regions are selected from any one of lists 1-3.

E10. The composition of any one of E1-E5 and E9, wherein the at least 200 target genomic regions comprise at least 20%, 30%, 40%, 50%, 60%, 70%, 80%, 90% or 95% of the target genomic regions in any one of lists 1-3.

E11. The composition of any one of E1-E5 and E9-E10, wherein the at least 200 target genomic regions comprise at least 500, 1,000, 5,000, 10,000, 15,000, 20,000, 30,000, 40,000, or 50,000 target genomic regions in any one of lists 1-3.

E12. The composition of any one of E1-E5, wherein the at least 200 target genomic regions are selected from any one of lists 13-16.

E13. The composition of any one of E1-E5 and E12, wherein the at least 200 target genomic regions comprise at least 10%, 20%, 25%, 30%, 40%, 50%, 60%, 70%, 80%, 90% or 95% of the target genomic regions in any one of lists 13-16.

E14. The composition of any one of E1-E5 and E12-E13, wherein the at least 200 target genomic regions comprise at least 500, 1,000, 5,000, 10,000, 15,000, 20,000, 30,000, 40,000, or 50,000 target genomic regions in any one of lists 13-16.

E15. The composition of any one of E1-E5, wherein the at least 200 target genomic regions are selected from list 12.

E16. The composition of any one of E1-E5 and E15, wherein the at least 200 target genomic regions comprise at least 10%, 20%, 25%, 30%, 40%, 50%, 60%, 70%, 80%, 90% or 95% of the target genomic regions in list 12.

E17. The composition of any one of claims E1-E5 and E15-E17, wherein the at least 200 target genomic regions comprise at least 500, 1,000, 5,000, 10,000, 15,000, 20,000, 30,000, 40,000, or 50,000 target genomic regions in list 12.

E18. The composition of any one of E1-E5, wherein the at least 200 target genomic regions are selected from any one of lists 8-11.

E19. The composition of any one of E1-E5 and E18, wherein the at least 200 target genomic regions comprise at least 20%, 30%, 40%, 50%, 60%, 70%, 80%, 90% or 95% of the target genomic regions in any one of lists 8-11.

E20. The composition of any one of E1-E5 and E18-E19, wherein the at least 200 target genomic regions comprise at least 500, 1,000, 5,000, 10,000, 15,000, 20,000, 30,000, 40,000, or 50,000 target genomic regions in any one of lists 8-11.

E21. The composition of any one of E1-E5, wherein the at least 200 target genomic regions comprise at least 40%, 50%, 60%, or 70% of the target genomic regions listed in List 4.

E22. The composition of any one of E1-E21, wherein the at least 200 target genomic regions comprise, for at least 90% or for 100% of all possible pairs of cancer types selected from a set comprising at least 10 cancer types, at least one target genomic region that is differentially methylated between the pair of cancer types.

E23. The composition of any one of E1-E22, wherein the plurality of bait oligonucleotides hybridize to at least 15 nucleotides or to at least 30 nucleotides of the DNA molecules derived from the at least 200 target genomic regions.

E24. The composition of any one of E1-E23, wherein the DNA molecules derived from the at least 200 target genomic regions are converted cfDNA fragments.

E25. The composition of E24, wherein the cfDNA fragments are converted by a process comprising treatment with bisulfite.

E26. The composition of E24, wherein the cfDNA fragments are converted by an enzymatic conversion reaction.

E27. The composition of E24, wherein the cfDNA fragments are converted by a cytosine deaminase.

E28. The composition of any one of E1-E27, wherein each bait oligonucleotide is conjugated to an affinity moiety.

E29. The composition of E28, wherein the affinity moiety is biotin.

E30. The composition of any one of E1-E29, wherein each bait oligonucleotide is between 50 and 300 bases in length, between 60 and 200 bases in length, between 100 and 150 bases in length, between 110 and 130 bases in length, and/or 120 bases in length.

E31. A composition comprising a plurality of different bait oligonucleotides configured to hybridize to DNA molecules derived from at least 100 target genomic regions selected from any one of Lists 1-16.

E32. The composition of E31, wherein the at least 100 target genomic regions comprises at least 200 target genomic regions.

E33. The composition of E31 or E32, wherein the at least 100 target genomic regions are selected from any one of lists 1-16.

E34. The composition of any one of E31-E33, wherein the at least 100 target genomic regions comprise at least 20%, 30%, 40%, 50%, 60%, 70%, 80%, 90% or 95% of the target genomic regions in any one of lists 1-16.

E35. The composition of any one of E31-E34, wherein the at least 100 target genomic regions comprise at least 500, 1,000, 5,000, 10,000, 15,000, 20,000, 30,000, 40,000, or 50,000 target genomic regions in any one of lists 1-16.

E36. The composition of any one of E31-E32, wherein the at least 100 target genomic regions are selected from any one of lists 1-3.

E37. The composition of any one of E31-E32 and E36, wherein the at least 100 target genomic regions comprise at least 20%, 30%, 40%, 50%, 60%, 70%, 80%, 90% or 95% of the target genomic regions in any one of lists 1-3.

E38. The composition of any one of E31-E32 and E36-E37, wherein the at least 100 target genomic regions comprise at least 500, 1,000, 5,000, 10,000, 15,000, 20,000, 30,000, 40,000, or 50,000 target genomic regions in any one of lists 1-3.

E39. The composition of E31 or E32, wherein the at least 100 target genomic regions are selected from list 12.

E40. The composition of any one of E31-E32 and E39, wherein the at least 100 target genomic regions comprise at least 10%, 20%, 25%, 30%, 40%, 50%, 60%, 70%, 80%, 90% or 95% of the target genomic regions in list 12.

E41. The composition of any one of E31-E32 and E39-E40, wherein the at least 100 target genomic regions comprise at least 500, 1,000, 5,000, 10,000, 15,000, 20,000, 30,000, 40,000, or 50,000 target genomic regions in list 12.

E42. The composition of any one of E31-E32, wherein the at least 100 target genomic regions are selected from list 8.

E43. The composition of any one of E31-E32 and E42, wherein the at least 100 target genomic regions comprise at least 20%, 30%, 40%, 50%, 60%, 70%, 80%, 90% or 95% of the target genomic regions in list 8.

E44. The composition of any one of E31-E32 and E42-E43, wherein the at least 100 target genomic regions comprise at least 500, 1,000, 5,000, 10,000, 15,000, 20,000, 30,000, 40,000, or 50,000 target genomic regions in list 8.

E45. The composition of any one of E31-E32, wherein the at least 100 target genomic regions comprise at least 40%, 50%, 60%, or 70% of the target genomic regions listed in List 4.

E46. The composition of any one of E31-E45, wherein the DNA molecules derived from the at least 100 target genomic regions are converted cfDNA fragments.

E47. The composition of E46, wherein the cfDNA fragments are converted by a process comprising treatment with bisulfite.

E48. The composition of any one of E1-E47, further comprising cfDNA fragments from a test subject.

E49. The composition of E48, wherein the cfDNA fragments from the test subject are converted cfDNA molecules.

E50. The composition of E49, wherein the cfDNA fragments from the test subject are converted by a process comprising treatment with bisulfite.

E51. The composition of any one of E1-E50, wherein each target genomic region comprises at least 5 CpG dinucleotides.

E52. The composition of any one of E1-E51, wherein each bait oligonucleotide is between 60 and 200 bases in length, between 100 and 150 bases in length, between 110 and 130 bases in length, and/or 120 bases in length.

E53. The composition of any one of E1-E52, wherein the different bait oligonucleotides comprise a plurality of sets of two or more bait oligonucleotides, wherein each bait oligonucleotide within a set of bait oligonucleotides is configured to bind to the converted DNA molecules from the same target genomic region.

E54. The composition of any one of E1-E53, wherein the ratio of bait oligonucleotides configured to hybridize to hypermethylated target regions to bait oligonucleotides configured to hybridize to hypomethylated target regions is between 0.5 and 1.0.

E55. The composition of E54, wherein:

each set of bait oligonucleotides comprises one or more pairs of a first bait oligonucleotide and a second bait oligonucleotide,
each bait oligonucleotide comprises a 5' end and a 3' end,
a sequence of at least X nucleotide bases at the 3' end of the first bait oligonucleotide is identical to a sequence of X nucleotide bases at the 5' end the second bait oligonucleotide, and
X is at least 20, at least 25, or at least 30.

E56. The composition of E55, wherein X is 30.

E57. A method for enriching a cfDNA sample, the method comprising:

contacting a converted or unconverted cfDNA sample with the bait set of any one of E1-E56; and
enriching the sample for cfDNA corresponding to a first set of genomic regions by hybridization capture.

E58. The method of E57, wherein the cfDNA sample is a converted cfDNA sample

E59. A method for obtaining sequence information informative of a presence or absence of cancer or a type of cancer, the method comprising sequencing enriched converted cfDNA prepared by the method of E57 or E58.

E60. A method of determining a presence or absence of cancer in a subject, the method comprising,

a) capturing cfDNA fragments from the subject with the composition of any one of E1-E56,
b) sequencing the captured cfDNA fragments, and
c) applying a trained classifier to the cfDNA sequences to determine the presence or absence of cancer.

E61. The method of E60, wherein the likelihood of a false positive determination of a presence or absence of cancer is less than 1% and the likelihood of an accurate determination of a presence or absence of cancer is at least 40%.

E62. The method of E60, wherein the cancer is a stage I cancer, the likelihood of a false positive determination of a presence or absence of cancer is less than 1%, and the likelihood of an accurate determination of a presence or absence of cancer is at least 10%.

E63. The method of any one of E60-E62, wherein the cfDNA fragments are converted cfDNA fragments.

E64. A method of detecting a cancer type comprising,

d) capturing cfDNA fragments from a subject with a composition comprising a plurality of different oligonucleotide baits,

e) sequencing the captured cfDNA fragments, and

f) applying a trained classifier to the cfDNA sequences to determine a cancer type;

wherein the oligonucleotide baits are configured to hybridize to cfDNA fragments derived from a plurality of target genomic regions,

wherein the plurality of target genomic regions is differentially methylated in one or more cancer types relative to a different cancer type or a non-cancer type,

wherein the likelihood of a false-positive determination of cancer is less than 1%, and

wherein the likelihood of an accurate assignment of a cancer type is at least 75%, at least 80%, at least 85% or at least 89%, or at least 90%.

E65. The method of E64, further comprises applying the trained classifier to the cfDNA sequences to determine a presence of cancer before determining the cancer type.

E66. The method of any one of E64 or E65, wherein the cancer type is a stage I cancer type, and the likelihood of an accurate assignment is at least 75%.

E67. The method of any one of E64 or E65, wherein the cancer type is a stage II cancer type, and the likelihood of an accurate assignment is at least 85%.

E68. The method of any one of E64-E67, wherein the cancer type is prostate cancer and the likelihood of an accurate assignment of prostate cancer is at least 85% or at least 90%.

E69. The method of any one of E64-E67, wherein the cancer type is breast cancer and the likelihood of an accurate assignment of breast cancer is at least 90% or at least 95%.

E70. The method of any one of E64-E67, wherein the cancer type is uterine cancer and the likelihood of an accurate assignment of uterine cancer is at least 90% or at least 95%.

E71. The method of any one of E64-E67, wherein the cancer type is ovarian cancer and the likelihood of an accurate assignment of ovarian cancer is at least 85% or at least 90%.

E72. The method of any one of E64-E67, wherein the cancer type is bladder & urothelial cancer and the likelihood of an accurate assignment of bladder & urothelial is at least 90% or at least 95%.

E73. The method of any one of E64-E67, wherein the cancer type is colorectal cancer and the likelihood of an accurate assignment of colorectal cancer is at least 65% or at least 70%.

E74. The method of any one of E64-E67, wherein the cancer type is liver & bile duct cancer and the likelihood of an accurate assignment of liver & bile duct cancer is at least 90% or at least 95%.

E75. The method of any one of E64-E67, wherein the cancer type is pancreas & gallbladder cancer and the likelihood of an accurate assignment of pancreas & gallbladder cancer is at least 85% or at least 90%.

E76. The method of any one of E63-E67, wherein the cfDNA fragments are converted cfDNA fragments.

E77. The method of any one of E61-E76, wherein the cancer type is selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer.

E78. The method of any one of E63-E76, wherein the cancer type is selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer.

E79. The method of any one of E63-E76, wherein the cancer type is selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer, gallbladder cancer, upper GI cancer, multiple myeloma, lymphoid neoplasm, and lung cancer.

E80. The method of any one of E63-E79, wherein the likelihood of detecting sarcoma is at least 35% or at least 40%.

E81. The method of any one of E63-E76, wherein the likelihood of detecting stage III or stage IV renal cancer is at least 50% or at least 70%

E82. The method of any one of E63-E76, wherein the likelihood of detecting stage III or stage IV breast cancer is at least 70% or at least 85%.

E83. The method of any one of E63-E76, wherein the likelihood of detecting stage III or stage IV uterine cancer is at least 50%.

E84. The method of any one of E63-E76, wherein the likelihood of detecting ovarian cancer is at least 60% or at least 80%.

E85. The method of any one of E63-E76, wherein the likelihood of detecting bladder cancer is at least 35% or at least 40%

E86. The method of any one of E63-E76, wherein the likelihood of detecting anorectal cancer is at least 60% or 70%

E87. The method of any one of E63-E76, wherein the likelihood of detecting head and neck cancer is at least 75% or at least 80%.

E88. The method of any one of E63-E76, wherein the likelihood of detecting stage I head and neck cancer is at least 80%.

E89. The method of any one of E63-E76, wherein the likelihood of detecting colorectal cancer is at least 50% or at least 59%.

E90. The method of any one of E63-E76, wherein the likelihood of detecting liver cancer is at least 75% or 80%.

E91. The method of any one of E63-E76, wherein the likelihood of detecting pancreas and gallbladder cancer is at least 64% or at least 70%.

E92. The method of any one of E63-E76, wherein the likelihood of detecting upper GI cancer is at least at least 60% or at least 68%.

E93. The method of any one of E63-E76, wherein the likelihood of detecting multiple myeloma is at least 65% or at least 75%.

E94. The method of any one of E63-E76, wherein likelihood of detecting type I multiple myeloma is at least 60%.

E95. The method of any one of E63-E76, wherein the likelihood of detecting lymphoid neoplasm is at least 65% or at least 69%.

E96. The method of any one of E63-E76, wherein the likelihood of detecting lung cancer is at least 50% or at least 58%.

E97. The method of any one of E63-E96, wherein the composition comprising oligonucleotide baits is the composition of any one of E1-E56.

E98. The method of any one of E63-E97, wherein the plurality of genomic regions comprises no more than 95,000 genomic regions, no more than 60,000 genomic regions, no more than 40,000 genomic regions, no more than 35,000 genomic regions, no more than 20,000 genomic regions, no more than 15,000 genomic regions, no more than 8,000 genomic regions, no more than 4,000 genomic regions, no more than 2,000 genomic regions, or no more than 1,400 genomic regions.

E99. The method of any one of E61-E98, wherein the total size of the plurality of genomic regions is less than 4 MB, less than 2 MB, less than 1 MB, less than 0.7 MB, or less than 0.4 MB.

E100. The method of any one of E61-E99, wherein the subject has an elevated risk of one or more cancer types.

E101. The method of any one of E61-E100, wherein the subject manifests symptoms associated with one or more cancer types.

E102. The method of any one of E61-E101, wherein the subject has not been diagnosed with a cancer.

E103. The method of any one of E61-E102, wherein the classifier was trained on converted DNA sequences derived from a least 100 subjects with a first cancer type, at least 100 subjects with a second cancer type, and at least 100 subjects with no cancer.

E104. The method of E103, wherein the first cancer type is ovarian cancer.

E105. The method of E103, wherein the first cancer type is liver cancer.

E106. The method of E103, wherein the first cancer type is selected from thyroid cancer, melanoma, sarcoma, myeloid neoplasm, renal cancer, prostate cancer, breast cancer, uterine cancer, ovarian cancer, bladder cancer, urothecal cancer, cervical cancer, anorectal cancer head & neck cancer, colorectal cancer, liver cancer, pancreatic cancer, gallbladder cancer, esophageal cancer, stomach cancer, multiple myeloma, lymphoid neoplasm, lung cancer, or leukemia.

E107. The method of any one of E61-E106, wherein the classifier was trained on converted DNA sequences derived from at least 1000, at least 2000, or at least 4000 target genomic regions selected from any one of Lists 1-16.

E108. The method of E107, wherein the trained classifier determines the presence or absence of cancer or a cancer type by:

g) generating a set of features for the sample, wherein each feature in the set of features comprises a numerical value;

h) inputting the set of features into the classifier, wherein the classifier comprises a multinomial classifier;

i) based on the set of features, determining, at the classifier, a set of probability scores, wherein the set of probability scores comprises one probability score per cancer type class and per non-cancer type class; and

j) thresholding the set of probability scores based on one or more values determined during training of the classifier to determine a final cancer classification of the sample.

E109. The method of E108, wherein the set of features comprises a set of binarized features.

E110. The method of any of one of E108-E109, wherein the numerical value comprises a single binary value.

E111. The method of any of one E108-E110, wherein the multinomial classifier comprises a multinomial logistic regression ensemble trained to predict a source tissue for the cancer.

E112. The method of any of one E108-E111, further comprising determining the final cancer classification based on a top-two probability score differential relative to a minimum value, wherein the minimum value corresponds to a predefined percentage of training cancer samples that had been assigned the correct cancer type as their highest score during training of the classifier.

E113. The method of E112, wherein

k) in accordance with a determination that the top-two probability score differential exceeds the minimum value, assign a cancer label corresponding to the highest probability score determined by the classifier as the final cancer classification; and

1) in accordance with a determination that the top-two probability score differential does not exceed the minimum value, assigning an indeterminate cancer label as the final cancer classification.

E114. A method of treating a type of cancer in a subject in need thereof, the method comprising,

m) detecting the type of cancer by the method of any one of E61-E113, and

n) administering an anti-cancer therapeutic agent to the subject.

E115. The method of E114, wherein the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

E116. A cancer assay panel, comprising:

at least 500 pairs of probes, wherein each pair of the at least 500 pairs comprise two probes configured to overlap each other by an overlapping sequence,

wherein the overlapping sequence comprises a 30-nucleotide sequence, and

wherein the 30-nucleotide sequence is configured to hybridize to a converted cfDNA molecule corresponding to, or derived from one or more of genomic regions, wherein each of the genomic regions comprises at least five methylation sites, and wherein the at least five methylation sites have an abnormal methylation pattern in cancerous samples.

E117. The cancer assay panel of E116, wherein each of the at least 500 pairs of probes is conjugated to a non-nucleotide affinity moiety.

E118. The cancer assay panel of E117, wherein the non-nucleotide affinity moiety is a biotin moiety.

E119. The cancer assay panel of any one of E116-E118, wherein the cancerous samples are from subjects having cancer selected from the group consisting of breast cancer, uterine cancer, cervical cancer, ovarian cancer, bladder cancer, urothelial cancer of renal pelvis, renal cancer other than urothelial, prostate cancer, anorectal cancer, colorectal cancer, hepatobiliary cancer arising from hepatocytes, hepatobiliary cancer arising from cells other than hepatocytes, pancreatic cancer, squamous cell cancer of the upper gastrointestinal tract, upper gastrointestinal cancer other than squamous, head and neck cancer, lung adenocarcinoma, small cell lung cancer, squamous cell lung cancer and cancer other than adenocarcinoma or small cell lung cancer, neuroendocrine cancer, melanoma, thyroid cancer, sarcoma, multiple myeloma, lymphoma, and leukemia.

E120. The cancer assay panel of any one of E116-E119, wherein the abnormal methylation pattern has at least a threshold p-value rarity in the cancerous samples.

E121. The cancer assay panel of any one of E116-E120, wherein each of the probes is designed to have less than 20 off-target genomic regions.

E122. The cancer assay panel of E121, wherein the less than 20 off-target genomic regions are identified using a k-mer seeding strategy.

E123. The cancer assay panel of E122, wherein the less than 20 off-target genomic regions are identified using k-mer seeding strategy combined to local alignment at seed locations.

E124. The cancer assay panel of any of E116-E123, comprising at least 10,000, 50,000, 100,000, 200,000, 300,000, 400,000, 500,000, 600,000, 700,000 or 800,000 probes.

E125. The cancer assay panel of any of E116-E124, wherein the at least 500 pairs of probes together comprise at least 2 million, 3 million, 4 million, 5 million, 6 million, 8 million, 10 million, 12 million, 14 million, or 15 million nucleotides.

E126. The cancer assay panel of any of E116-E125, wherein each of the probes comprises at least 50, 75, 100, or 120 nucleotides.

E127. The cancer assay panel of any of E116-E126, wherein each of the probes comprises less than 300, 250, 200, or 150 nucleotides.

E128. The cancer assay panel of any of E116-E127, wherein each of the probes comprises 100-150 nucleotides.

E129. The cancer assay panel of any of E116-E128, wherein each of the probes comprises less than 20, 15, 10, 8, or 6 methylation sites.

E130. The cancer assay panel of any of E116-E129, wherein at least 80, 85, 90, 92, 95, or 98% of the at least five methylation sites are either methylated or unmethylated in the cancerous samples.

E131. The cancer assay panel of any of E116-E130, wherein at least 3%, 5%, 10%, 15%, or 20% of the probes comprise no G (Guanine).

E132. The cancer assay panel of any of E116-E131, wherein each of the probes comprise multiple binding sites to the methylation sites of the converted cfDNA molecule, wherein at least 80, 85, 90, 92, 95, or 98% of the multiple binding sites comprise exclusively either CpG or CpA.

E133. The cancer assay panel of any of E116-E132, wherein each of the probes is configured to have less than 15, 10 or 8 off-target genomic regions.

E134. The cancer assay panel of any of E116-E133, wherein at least 30% of the genomic regions are in exons or introns.

E135. The cancer assay panel of any of E116-E134, wherein at least 15% of the genomic regions are in exons.

E136. The cancer assay panel of any of E116-E135, wherein at least 20% of the genomic regions are in exons.

E137. The cancer assay panel of any of E116-E136, wherein less than 10% of the genomic regions are in intergenic regions.

E138. The cancer assay panel of any of E116-E137, wherein the genomic regions are selected from any one of Lists 1-3 or Lists 4-16.

E139. The cancer assay panel of any of E116-E138, wherein the genomic regions comprise at least 20%, 30%, 40%, 50%, 60%, 70%, 80%, 90% or 95% of the genomic regions in any one of Lists 1-3 or Lists 4-16.

E140. The cancer assay panel of any of E116-E139, wherein the genomic regions comprise at least 500, 1,000, 5000, 10,000, or 15,000, 20,000, 30,000, 40,000, 50,000, 60,000, or 70,000 genomic regions in any one of Lists 1-3 or Lists 4-16.

E141. A cancer assay panel comprising a plurality of probes, wherein each of the plurality of probes is configured to hybridize to a converted cfDNA molecule corresponding to one or more of the genomic regions in any one of Lists 1-3 or Lists 4-16.

E142. The cancer assay panel of E141, wherein the plurality of probes together is configured to hybridize to a plurality of converted cfDNA molecules corresponding to at least 20%, 30%, 40%, 50%, 60%, 70%, 80%, or 90%, 95% or 100% of the genomic regions of any one of Lists 1-3 or Lists 4-16.

E143. The cancer assay panel of E0, wherein the plurality of probes together is configured to hybridize to a plurality of converted cfDNA molecules corresponding to at least 500, 1,000, 5000, 10,000, 15,000, 20,000, 30,000, 40,000, or 50,000 genomic regions of any one of Lists 1-3 or Lists 4-16.

E144. The cancer assay panel of any one of E141-E143, wherein at least 3%, 5%, 10%, 15%, or 20% of the probes comprise no G (Guanine).

E145. The cancer assay panel of any one of E141-E144, wherein each of the probes comprise multiple binding sites to methylation sites of the converted cfDNA molecule, wherein at least 80, 85, 90, 92, 95, or 98% of the multiple binding sites comprise exclusively either CpG or CpA.

E146. The cancer assay panel of any one of E141-E145, wherein each of the probes is conjugated to a non-nucleotide affinity moiety.

E147. The cancer assay panel of E146, wherein the non-nucleotide affinity moiety is a biotin moiety.

E148. A method of determining a tissue of origin (TOO) of a cancer, comprising:

o) receiving a sample comprising a plurality of cfDNA molecules;
p) treating the plurality of cfDNA molecules to convert unmethylated C (cytosine) to U (uracil), thereby obtaining a plurality of converted cfDNA molecules;
q) applying the cancer assay panel of any one of E0-E0 to the plurality of converted cfDNA molecules, thereby enriching a subset of the converted cfDNA molecules; and
r) sequencing the enriched subset of the converted cfDNA molecule, thereby providing a set of sequence reads.

E149. The method of E148, further comprising the step of:
determining a health condition by evaluating the set of sequence reads, wherein the health condition is

s) a presence or absence of cancer;
t) a presence or absence of cancer of a tissue of origin (TOO);

u) a presence or absence of a cancer cell type; or

v) a presence or absence of at least 5, 10, 15, or 20 different types of cancer.

E150. The method of any of E148-E149, wherein the sample comprising a plurality of cfDNA molecules was obtained from a human subject.

E151. A method for detecting a cancer, comprising the steps of:

w) obtaining a set of sequence reads by sequencing a set of nucleic acid fragments from a subject, wherein the nucleic acid fragments are corresponding to, or derived from a plurality of genomic regions selected from any one of Lists 1-3 or Lists 4-16;

x) for each of the nucleic acid fragments, determining methylation status at a plurality of CpG sites; and

y) detecting a health condition of the subject by evaluating the methylation status for the sequence reads, wherein the health condition is (i) a presence or absence of cancer; (ii) a presence or absence of cancer of a tissue of origin (TOO); (iii) a presence or absence of a cancer cell type; or (iv) a presence or absence of at least 5, 10, 15, or 20 different types of cancer.

E152. The method of E151, wherein the plurality of genomic regions comprises at least 20%, 30%, 40%, 50%, 60%, 70%, 80%, 90%, 95%, or 100% of the genomic regions of any one of Lists 1-3 or lists 4-16.

E153. The method of E151, wherein the plurality of genomic regions comprises 500, 1,000, 5000, 10,000, 15,000, 20,000, 30,000, 40,000, 50,000, 60,000, 70,000, or 80,000 of the genomic regions of any one of Lists 1-3 or Lists 4-16.

E154. A method of designing a cancer assay panel for diagnosing cancer of a tissue of origin (TOO) comprising the steps of:

z) identifying a plurality of genomic regions, wherein each of the plurality of genomic regions (i) comprises at least 30 nucleotides, and (ii) comprises at least five methylation sites,

aa) selecting a subset of the genomic regions, wherein the selection is made when cfDNA molecules corresponding to, or derived from each of the genomic regions in cancerous samples have an abnormal methylation pattern, wherein the abnormal methylation pattern comprises at least five methylation sites either hypomethylated or hypermethylated, and

bb) designing a cancer assay panel comprising a plurality of probes, wherein each of the probes is configured to hybridize to a converted cfDNA molecule corresponding to or derived from one or more of the subset of the genomic regions.

E155. A bait set for hybridization capture, the bait set comprising a plurality of different oligonucleotide-containing probes, wherein each of the oligonucleotide-containing probes comprises a sequence of at least 30 bases in length that is complementary to either:

(1) a sequence of a genomic region; or

(2) a sequence that varies from the sequence of (1) only by one or more transitions, wherein each respective transition of the one or more transitions occurs at a cytosine in the genomic region, and wherein each probe of the different oligonucleotide-containing probes is complementary to a sequence corresponding to a CpG site that is differentially methylated in samples from subjects with a

first cancer type relative to samples from subjects with a second cancer type or a non-cancer type. E156. The bait set of E155, wherein the first cancer type and the second cancer type are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer.

E157. The bait set of any of E155-E156, wherein the bait set comprises at least 500, 1,000, 2,000, 2,500, 5,000, 6,000, 7,500, 10,000, 15,000, 20,000, 25,000, 50,000, 100,000, 200,000, 300,000, 500,000, or 800,000 different oligonucleotide-containing probes.

E158. The bait set of any one of E155-E157, wherein, for each of the different oligonucleotide-containing probes, the sequence of at least 30 bases in length is complementary to either (1) a sequence within a genomic region selected from the genomic regions set forth in any one of Lists 1-16; or (2) a sequence that varies from the sequence of (1) only by one or more transitions, wherein each respective transition of the one or more transitions occurs at a cytosine in the genomic region.

E159. The bait set of E158, wherein the sequence of at least 30 bases in length is complementary to either (1) a sequence within a genomic region selected from the genomic regions set forth in any one of Lists 1-3; or (2) a sequence that varies from the sequence of (1) only by one or more transitions, wherein each respective transition of the one or more transitions occurs at a cytosine in the genomic region.

E160. The bait set of E158, wherein the sequence of at least 30 bases in length is complementary to either (1) a sequence within a genomic region selected from the genomic regions set forth in in any one of Lists 5 or 7; or (2) a sequence that varies from the sequence of (1) only by one or more transitions, wherein each respective transition of the one or more transitions occurs at a cytosine in the genomic region.

E161. The bait set of E158, wherein the sequence of at least 30 bases in length is complementary to either (1) a sequence within a genomic region selected from the genomic regions set forth in any one of Lists 4, 8, or 8-12; or (2) a sequence that varies from the sequence of (1) only by one or more transitions, wherein each respective transition of the one or more transitions occurs at a cytosine in the genomic region.

E162. The bait set of E158, wherein the sequence of at least 30 bases in length is complementary to either (1) a sequence within a genomic region selected from the genomic regions set forth in any one of Lists 13-16; or (2) a sequence that varies from the sequence of (1) only by one or more transitions, wherein each respective transition of the one or more transitions occurs at a cytosine in the genomic region.

E163. The bait set of E158, wherein the sequence of at least 30 bases in length is complementary to either (1) a sequence within a genomic region selected from the genomic regions set forth in any one of Lists 13-16; or (2) a sequence that varies from the sequence of (1) only by one or more transitions, wherein each respective transition of the one or more transitions occurs at a cytosine in the genomic region.

E164. The bait set of E158, wherein the sequence of at least 30 bases in length is complementary to either (1) a sequence within a genomic region selected from the genomic regions set forth in any one of Lists 4 or 6; or (2) a sequence that varies from the sequence of (1) only by one or more transitions, wherein each respective transition of the one or more transitions occurs at a cytosine in the genomic region.

E165. The bait set of E158, wherein the sequence of at least 30 bases in length is complementary to either (1) a sequence within a genomic region selected from the genomic regions set forth in List 4; or (2) a sequence that varies from the sequence of (1) only by one or more transitions, wherein each respective transition of the one or more transitions occurs at a cytosine in the genomic region.

E166. The bait set of E158, wherein the sequence of at least 30 bases in length is complementary to either (1) a sequence within a genomic region selected from the genomic regions set forth in List 8; or (2) a sequence that varies from the sequence of (1) only by one or more transitions, wherein each respective transition of the one or more transitions occurs at a cytosine in the genomic region.

E167. The bait set of E158, wherein the sequence of at least 30 bases in length is complementary to either (1) a sequence within a genomic region selected from the genomic regions set forth in List 9; or (2) a sequence that varies from the sequence of (1) only by one or more transitions, wherein each respective transition of the one or more transitions occurs at a cytosine in the genomic region.

E168. The bait set of E158, wherein the sequence of at least 30 bases in length is complementary to either (1) a sequence within a genomic region selected from the genomic regions set forth in List 10; or (2) a sequence that varies from the sequence of (1) only by one or more transitions, wherein each respective transition of the one or more transitions occurs at a cytosine in the genomic region.

E169. The bait set of E158, wherein the sequence of at least 30 bases in length is complementary to either (1) a sequence within a genomic region selected from the genomic regions set forth in List 11; or (2) a sequence that varies from the sequence of (1) only by one or more transitions, wherein each respective transition of the one or more transitions occurs at a cytosine in the genomic region.

E170. The bait set of E158, wherein the sequence of at least 30 bases in length is complementary to either (1) a sequence within a genomic region selected from the genomic regions set forth in List 12; or (2) a sequence that varies from the sequence of (1) only by one or more transitions, wherein each respective transition of the one or more transitions occurs at a cytosine in the genomic region.

E171. The bait set of any one of E155-E170, wherein the plurality of different oligonucleotide-containing probes are each conjugated to an affinity moiety.

E172. The bait set of E171, wherein the affinity moiety is biotin.

E173. The bait set of any one of E155-E172, wherein at least 80%, 90%, or 95% of the oligonucleotide-containing probes in the bait set do not include an at least 30, at least 40, or at least 45 base sequence that has 20 or more off-target regions in the genome.

E174. The bait set of any one of E155-E173, wherein the oligonucleotide-containing probes in the bait set do not include an at least 30, at least 40, or at least 45 base sequence that has 20 or more off-targets regions in the genome.

E175. The bait set of any one of E155-E174, wherein the sequence of at least 30 bases of each of the probes is at least 40 bases, at least 45 bases, at least 50 bases, at least 60 bases, at least 75, or at least 100 bases in length.

E176. The bait set of any one of E155-E175, wherein each of the oligonucleotide-containing probes has a nucleic acid sequence of at least 45, 40, 75, 100, or 120 bases in length.

E177. The bait set of any one of E155-E176, wherein each of the oligonucleotide-containing probes have a nucleic acid sequence of no more than 300, 250, 200, or 150 bases in length.

E178. The bait set of any one of E155-E177, wherein each of the plurality of different oligonucleotide-containing probes is between 60 and 200 bases in length, between 100 and 150 bases in length, between 110 and 130 bases in length, and/or 120 bases in length.

E179. The bait set of any one of E155-E178, wherein the different oligonucleotide-containing probes comprise at least 500, at least 1000, at least 2,000, at least 2,500, at least 5,000, at least 6,000, at least 7,500, and least 10,000, at least 15,000, at least 20,000, or at least 25,000 different pairs of probes, wherein each pair of probes comprises a first probe and second probe, wherein the second probe differs from the first probe and overlaps with the first probe by an overlapping sequence that is at least 30, at least 40, at least 50, or at least 60 nucleotides in length.

E180. The bait set of any one of E155-E179, wherein the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 1-16.

E181. The bait set of E180, wherein the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 1-3.

E182. The bait set of E180, wherein the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 4-12.

E183. The bait set of E180, wherein the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in any one of Lists 4, 6, or 8-12.

E184. The bait set of E180, wherein the bait set comprises oligonucleotide-containing probes that are configured to target at least 20%, at least 25%, at least 30%, at least 40%, at least 50%, at least 60%, at least 70%, at least 80%, at least 90%, at least 95%, or 100% of the genomic regions identified in List 8.

E185. The bait set of any one of E155-E184, wherein an entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 1-16.

E186. The bait set of E185, wherein the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 1-3.

E187. The bait set of E185, wherein the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 4-12.

E188. The bait set of E185, wherein the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from any one of Lists 4, 6, or 8-12.

E189. The bait set of E185, wherein the entirety of oligonucleotide probes in the bait set are configured to hybridize to fragments obtained from cfDNA molecules corresponding to at least 30%, 40%, 50%, 60%, 70%, 80%, 90% or 95% of the genomic regions in a list selected from List 8.

E190. The bait set of any one of E155-E189, wherein an entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 1-16.

E191. The bait set of E190, wherein the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 1-3.

E192. The bait set of E190, wherein the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 4-12.

E193. The bait set of E190, wherein the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in any one of Lists 4, 6, or 8-12.

E194. The bait set of E190, wherein the entirety of oligonucleotide-containing probes in the bait set are configured to hybridize to fragments obtained from cfDNA molecules corresponding to at least 500, 1,000, 5000, 10,000, 15,000, 20,000, at least 25,000, at least 30,000, at least 50,000 or at least 80,000 genomic regions in List 8.

E195. The bait set of any one of E155-E194, wherein the plurality of oligonucleotide-containing probes comprise at least 500, 1,000, 5,000, or 10,000 different subsets of probes, wherein each subset of probes comprises a plurality of probes that collectively extend across a genomic region selected from the genomic regions of any one of Lists 1-16 in a 2 × tiled fashion.

E196. The bait set of any one of E155-E195, wherein the plurality of oligonucleotide-containing probes comprise at least 500, 1,000, 5,000, or 10,000 different subsets of probes, wherein each subset of probes comprises a plurality of probes that collectively extend across a genomic region selected from the genomic regions of any one of Lists 1-4, 6, or 8-12 in a 2× tiled fashion.

E197. The bait set of any one of E195-E196, wherein the plurality of probes that collectively extend across the genomic region in a 2× tiled fashion comprises at least one pair of probes that overlap by a sequence of at least 30 bases, at least 40 bases, at least 50 bases, or at least 60 bases in length.

E198. The bait set of any one of E155-E196, wherein the plurality of probes collectively extend across portions of the genome that collectively are a combined size of less than 4 MB, less than 2 MB, less than 1 MB, less than 0.7 MB, or less than 0.4 MB.

E199. The bait set of any one of E155-E196, wherein the plurality of probes collectively extend across portions of the genome that collectively are a combined size of between 0.2 and 30 MB, between 0.5 MB and 30 MB, between 1 MB and 30 MB, between 3 MB and 25 MB, between 3 MB and 15, MB, between 5 MB and 20 MB, or between 7 MB and 12 MB.

E200. The bait set of any one of E155-E199, wherein each of the different oligonucleotide-containing probes comprises less than 20, 15, 10, 8, or 6 CpG detection sites.

E201. The bait set of any of E155-E200, wherein at least 80%, 85%, 90%, 92%, 95%, or 98% of the plurality of oligonucleotide-containing probes have exclusively either CpG or CpA on all CpG detection sites.

E202. A mixture comprising:

   converted cfDNA; and
   the bait set of any one of E155-E201.

E203. The mixture of E202, wherein the converted cfDNA comprises bisulfite-converted cfDNA.

E204. The mixture of E202, wherein the converted cfDNA comprises cfDNA that has been converted via a cytosine deaminase.

E205. A method for enriching a converted cfDNA sample, the method comprising:

   contacting the converted cell-free DNA sample with the bait set of any one of E155-E201; and
   enriching the sample for a first set of genomic regions by hybridization capture.

E206. A method for providing sequence information informative of a presence or absence of a cancer or a type of cancer, the method comprising:

   cc) processing cfDNA from a biological sample with a deaminating agent to generate a cell-free DNA sample comprising deaminated nucleotides;
   dd) enriching the cfDNA sample for informative cell-free DNA molecules; and
   ee) sequencing the enriched cfDNA molecules, thereby obtaining a set of sequence reads informative of a presence or absence of a cancer or a type of cancer.

E207. The method of E206, wherein enriching the cfDNA comprises amplifying, via PCR, portions of the cell-free DNA fragments using primers configured to hybridize to a plurality of genomic regions selected from any one of Lists 1-16.

E208. The method of E206, wherein enriching the cfDNA sample comprises contacting the cell-free DNA with a plurality of probes configured to hybridize to converted fragments obtained from the cfDNA molecules corresponding to or derived from the genomic regions in any one of Lists 1-16.

E209. The method of E206, wherein enriching the cfDNA sample comprises contacting the cell-free DNA with a plurality of probes configured to hybridize to converted fragments obtained from the cfDNA molecules corresponding to or derived from at least 30%, 40%, 50%, 60%, 70%, 80%, 90%, 95% of the genomic regions in any one of Lists 1-16.

E210. The method of any one of E206-E209, wherein the genomic regions are selected from any one of Lists 1-3.

E211. The method of any one of E206-E209, wherein the genomic regions are selected from any one of Lists 4-12.

E212. The method of any one of E206-E209, wherein the genomic regions are selected from any one of Lists 4, 6, or 8-12.

E213. The method of any one of E206-E209, wherein the genomic regions are selected from List 8.

E214. The method of any one of E206-E213, wherein the cfDNA sample is enriched by the method of E0.

E215. The method of any one of E206-E214, further comprising determining a cancer classification by evaluating the set of sequence reads, wherein the cancer classification is

ff) a presence or absence of cancer; or
gg) a presence or absence of a type of cancer.

E216. The method of E215, wherein the step of determining a cancer classification comprises:

hh) generating a test feature vector based on the set of sequence reads; and
ii) applying the test feature vector to a classifier.

E217. The method of E216, wherein the classifier comprises a model that is trained by a training process with a first cancer set of fragments from one or more training subjects with a first cancer type and a second cancer set of fragments from one or more training subjects with a second cancer type, wherein both the first cancer set of fragments and the second cancer set of fragments comprise a plurality of training fragments.

E218. The method of any one of E206-E217, wherein the cancer classification is a presence or absence of cancer.

E219. The method of E218, wherein the classifier has an area under a receiver operating characteristic curve of at least 0.8.

E220. The method of any one of E206-E217, wherein the cancer classification is a type of cancer.

E221. The method of E220, wherein the type of cancer is selected from among at least 12, 14, 16, 18, or 20 cancer types.

E222. The method of E220, wherein the cancer types are selected from uterine cancer, upper GI squamous cancer, all other upper GI cancers, thyroid cancer, sarcoma, urothelial renal cancer, all other renal cancers, prostate cancer, pancreatic cancer, ovarian cancer, neuroendocrine cancer, multiple myeloma, melanoma, lymphoma, small cell lung cancer, lung adenocarcinoma, all other lung cancers, leukemia, hepatobiliary carcinoma, hepatobiliary biliary, head and neck cancer, colorectal cancer, cervical cancer, breast cancer, bladder cancer, and anorectal cancer.

E223. The method of E220, wherein the cancer types are selected from anal cancer, bladder cancer, colorectal cancer, esophageal cancer, head and neck cancer, liver/bile-duct cancer, lung cancer, lymphoma, ovarian cancer, pancreatic cancer, plasma cell neoplasm, and stomach cancer.

E224. The method of E220, wherein the cancer types are selected from thyroid cancer, melanoma, sarcoma, myeloid neoplasm, renal cancer, prostate cancer, breast cancer, uterine cancer, ovarian cancer, bladder cancer, urothelial cancer, cervical cancer, anorectal cancer, head & neck cancer, colorectal cancer, liver cancer, bile duct cancer, pancreatic cancer gallbladder cancer, upper GI cancer, multiple myeloma, lymphoid neoplasm, and lung cancer.

E225. The method of any one of E220-E224,

wherein at 99% specificity the sensitivity of the method for head and neck cancer is at least 79% or at least 84%;
wherein at 99% specificity the sensitivity of the method for liver cancer is at least 82% or at least 85%;
wherein at 99% specificity the sensitivity of the method for upper GI tract cancer is at least 62% or at least 68%;
wherein at 99% specificity the sensitivity of the method for pancreatic or gallbladder cancer is at least 62% or at least 68%%;
wherein at 99% specificity the sensitivity of the method for colorectal cancer is at least 60% or at least 65%;
wherein at 99% specificity the sensitivity of the method for ovarian cancer is at least 75% or at least 80%;
wherein at 99% specificity the sensitivity of the method for lung cancer is at least 60% or at least 65%;
wherein at 99% specificity the sensitivity of the method for multiple myeloma is at least 68% or at least 75%;
wherein at 99% specificity the sensitivity of the method for lymphoid neoplasm is at least 65% or at least 70%;
wherein at 99% specificity the sensitivity of the method for anorectal cancer is at least 60% or at least 65%; and
wherein at 99% specificity the sensitivity of the method for bladder cancer is at least 40% or at least 44%.

E226. The method of E215, wherein the cancer classification is a presence or absence of a type of cancer.

E227. The method of E226, wherein the step of determining a cancer classification comprises:

jj) generating a test feature vector based on the set of sequence reads; and
kk) applying the test feature vector to a classifier.

E228. The method of E227, wherein the classifier comprises a model that is trained by a training process with a first cancer type set of converted DNA sequences from one or more training subjects with a first cancer type and a second cancer type set of converted DNA sequences from one or more training subjects with a second cancer type, wherein both the first cancer type set of converted DNA sequences and the second cancer type set of converted DNA sequences comprise a plurality of training converted DNA sequences.

E229. The method of any one of E226-E228, wherein the type of cancer is selected from the group consisting of head and neck cancer, liver/bile duct cancer, upper GI cancer, pancreatic/gallbladder cancer; colorectal cancer, ovarian cancer, lung cancer, multiple myeloma, lymphoid neoplasms, melanoma, sarcoma, breast cancer, and uterine cancer.

E230. The method of any one of E227-E229, wherein the type of cancer is head and neck cancer, and the method, at 99.0% specificity, has a sensitivity of at least 79% or at least 84$.

E231. The method of any one of E227-E229, wherein the type of cancer is liver cancer, and the method, at 99.0% specificity, has a sensitivity of at least 82% or at least 85%.

E232. The method of any one of E227-E229, wherein the type of cancer is an upper GI tract cancer, and the method, at 99.0% specificity, has a sensitivity of at least 62% or at least 68%.

E233. The method of any one of E227-E229, wherein the type of cancer is a pancreatic or gallbladder cancer, and the method, at 99.0% specificity, has a sensitivity of at least 62% or at least 68%.

E234. The method of any one of E227-E229, wherein the type of cancer is colorectal cancer, and the method, at 99.0% specificity, has a sensitivity of at least 60% or at least 65%.

E235. The method of any one of E227-E229, wherein the type of cancer is ovarian cancer, and the method, at 99.0% specificity, has a sensitivity of at least 75% or at least 80%.

E236. The method of any one of E227-E229, wherein the type of cancer is lung cancer, and the method, at 99.0% specificity, has a sensitivity of at least60% or at least 65%.

E237. The method of any one of E227-E229, wherein the type of cancer is multiple myeloma, and the method, at 99.0% specificity, has a sensitivity of at least 68% or at least 75%.

E238. The method of any one of E227-E229, wherein the type of cancer is a lymphoid neoplasm, and the method, at 99.0% specificity, has a sensitivity of at least 65% or at least 70%.

E239. The method of any one of E227-E229, wherein the type of cancer is anorectal cancer, and the method, at 99.0% specificity, has a sensitivity of at least 60% or at least 65%.

E240. The method of any one of E227-E229, wherein the type of cancer is bladder cancer, and the method, at 99.0% specificity, has a sensitivity of at least 40% or at least 44%.

E241. The method of any one of E206-E240, wherein the total size of the target genomic regions is less than 4 Mb, less than 2 Mb, less than 1 Mb, less than 0.7 Mb or less than 0.4 Mb.

E242. The method of any one of E206-E241, wherein the step of determining a cancer classification comprises:

ll) generating a test feature vector based on the set of sequence reads; and

mm) applying the test feature vector to a model obtained by a training process with a cancer set of fragments from one or more training subjects with a cancer and a non-cancer set of fragments from one or more training subjects without cancer, wherein both the cancer set of fragments and the non-cancer set of fragments comprise a plurality of training fragments.

E243. The method of E242, wherein the training process comprises:

a) obtaining sequence information of training fragments from a plurality of training subjects;

b) for each training fragment, determining whether that training fragment is hypomethylated or hypermethylated, wherein each of the hypomethylated and hypermethylated training fragments comprises at least a threshold number of CpG sites with at least a threshold percentage of the CpG sites being unmethylated or methylated, respectively,

c) for each training subject, generating a training feature vector based on the hypomethylated training fragments and hypermethylated training fragments, and

d) training the model with the training feature vectors from the one or more training subjects without cancer and the training feature vectors from the one or more training subjects with cancer.

E244. The method of E242, wherein the training process comprises:

nn) obtaining sequence information of training fragments from a plurality of training subjects;

oo) for each training fragment, determining whether that training fragment is hypomethylated or hypermethylated, wherein each of the hypomethylated and hypermethylated training fragments comprises at least a threshold number of CpG sites with at least a threshold percentage of the CpG sites being unmethylated or methylated, respectively,

**pp)** for each of a plurality of CpG sites in a reference genome:

quantifying a count of hypomethylated training fragments which overlap the CpG site and a count of hypermethylated training fragments which overlap the CpG site; and

generating a hypomethylation score and a hypermethylation score based on the count of hypomethylated training fragments and hypermethylated training fragments;

qq) for each training fragment, generating an aggregate hypomethylation score based on the hypomethylation score of the CpG sites in the training fragment and an aggregate hypermethylation score based on the hypermethylation score of the CpG sites in the training fragment;

rr) for each training subject:

ranking the plurality of training fragments based on aggregate hypomethylation score and ranking the plurality of training fragments based on aggregate hypermethylation score; and

generating a feature vector based on the ranking of the training fragments;

ss) obtaining training feature vectors for one or more training subjects without cancer and training feature vectors for the one or more training subjects with cancer; and

tt) training the model with the feature vectors for the one or more training subjects without cancer and the feature vectors for the one or more training subjects with cancer.

E245. The method of any one of E242-E244, wherein the model comprises one of a kernel logistic regression classifier, a random forest classifier, a mixture model, a convolutional neural network, and an autoencoder model.

E246. The method of any one of E242-E245, further comprising the steps of:

a) obtaining a cancer probability for the test sample based on the model; and

b) comparing the cancer probability to a threshold probability to determine whether the test sample is from a subject with cancer or without cancer.

E247. The method of E246, further comprising the steps of:

a) obtaining a cancer type probability for the test sample based on the model; and

b) comparing the cancer type probability to a threshold probability to determine whether the test sample is from a subject with the cancer type or another cancer type or without cancer.

E248. The method of any one of E246-E247, further comprising administering an anti-cancer agent to the subject.

E249. A method of treating a cancer patient, the method comprising:

administering an anti-cancer agent to a subject who has been identified as a cancer subject by the method of E246.

E250. A method of treating a cancer patient, the method comprising:

administering an anti-cancer agent to a subject who has been identified as a cancer type subject by the method of E247.

E251. The method of any one of E249-E250, wherein the anti-cancer agent is a chemotherapeutic agent selected from the group consisting of alkylating agents, antimetabolites, anthracyclines, anti-tumor antibiotics, cytoskeletal disruptors (taxans), topoisomerase inhibitors, mitotic inhibitors, corticosteroids, kinase inhibitors, nucleotide analogs, and platinum-based agents.

E252. A method for assessing whether a subject has a cancer, the method comprising:

obtaining cfDNA from the subject;

isolating a portion of the cfDNA from the subject by hybridization capture;

obtaining sequence reads derived from the captured cfDNA to determine methylation states cfDNA fragments;

applying a classifier to the sequence reads; and

determining whether the subject has cancer based on application of the classifier;

wherein the classifier has an area under the receiver operator characteristic curve of at least 0.80.

E253. The method of E252, further comprising determining a cancer type,

wherein the sensitivity of the method for head and neck cancer is at least 79% or at least 84%;

wherein the sensitivity of the method for liver cancer is at least 82% or at least 85%;

wherein the sensitivity of the method for upper GI tract cancer is at least 62% or at least 68%;

wherein the sensitivity of the method for pancreatic or gallbladder cancer is at least 62% or at least 68%%;

wherein the sensitivity of the method for colorectal cancer is at least 60% or at least 65%;
wherein the sensitivity of the method for ovarian cancer is at least 75% or at least 80%;
wherein the sensitivity of the method for lung cancer is at least 60% or at least 65%;
wherein the sensitivity of the method for multiple myeloma is at least 68% or at least 75%;
wherein the sensitivity of the method for lymphoid neoplasm is at least 65% or at least 70%;
wherein the sensitivity of the method for anorectal cancer is at least 60% or at least 65%; and
wherein the sensitivity of the method for bladder cancer is at least 40% or at least 44%.

E254. The method of any one of E252-E253, wherein the total size of the target genomic regions is less than 4 Mb, less than 2 Mb, less than 1 Mb, less than 0.7 Mb or less than 0.4 Mb

E255. The method of any one of E252-E254, further comprising converting unmethylated cytosines in the cfDNA to uracil prior to isolating the portion of the cfDNA from the subject by hybridization capture.

E256. The method of any one of E252-E255, further comprising converting unmethylated cytosines in the cfDNA to uracil after isolating the portion of the cfDNA from the subject by hybridization capture.

E257. The method of any one of E252-E256, wherein the classifier is a binary classifier.

E258. The method of any one of E252-E256, wherein the classifier is a mixture model classifier.

E259. The method of any one of E252-E258, wherein isolating a portion of the cfDNA from the subject by hybridization capture comprises contacting the cell-free DNA with a bait set comprising a plurality of different oligonucleotide-containing probes.

E260. The method of any one of E252-E259, wherein the bait set is the bait set of any one of E155-E201.

E261. A method comprising the steps of:

uu) obtaining a set of sequence reads of modified test fragments, wherein the modified test fragments are or have been obtained by processing a set of nucleic acid fragments from a test subject, wherein each of the nucleic acid fragments corresponds to or is derived from a plurality of genomic regions selected from any one of Lists 1-16; and

vv) applying the set of sequence reads or a test feature vector obtained based on the set of sequence reads to a model obtained by a training process with a first set of fragments from a plurality of training subjects with a first cancer type and a second set of fragments from a plurality of training subjects with a second cancer type, wherein both the first set of fragments and the second set of fragments comprise a plurality of training fragments.

E262. The method of E261, wherein the model comprises one of a kernel logistic regression classifier, a random forest classifier, a mixture model, a convolutional neural network, and an autoencoder model.

E263. The method of any one of E261-E262, wherein the set of sequence reads is obtained by using the assay panel of any one of E155-E201.

**Claims**

1.  A cancer assay panel, comprising:

    at least 500 pairs of probes, wherein each pair of the at least 500 pairs comprise two probes configured to overlap each other by an overlapping sequence,
    wherein the overlapping sequence comprises an at least 30-nucleotide sequence, and
    wherein the at least 30-nucleotide sequence is hybridized to a cfDNA molecule corresponding to, or derived from one or more of genomic regions, wherein each of the genomic regions comprises at least five methylation sites, and wherein the at least five methylation sites have an abnormal methylation pattern in cancerous samples;
    wherein the cancerous samples are from subjects having cancer selected from the group consisting of breast cancer, uterine cancer, cervical cancer, ovarian cancer, bladder cancer, urothelial cancer of renal pelvis, renal cancer other than urothelial, prostate cancer, anorectal cancer, colorectal cancer, hepatobiliary cancer arising from hepatocytes, hepatobiliary cancer arising from cells other than hepatocytes, pancreatic cancer, squamous cell cancer of the upper gastrointestinal tract, upper gastrointestinal cancer other than squamous, head and neck cancer, lung adenocarcinoma, small cell lung cancer, squamous cell lung cancer and cancer other than adenocarcinoma or small cell lung cancer, neuroendocrine cancer, melanoma, thyroid cancer, sarcoma, multiple myeloma, lymphoma, and leukemia.

2.  The cancer assay panel of claim 1, wherein the cfDNA molecule is a converted cfDNA molecule.

3.  The cancer assay panel of claim 1 or claim 2, wherein each of the at least 500 pairs of probes is conjugated to a non-nucleotide affinity moiety, optionally wherein the non-nucleotide affinity moiety is a biotin moiety.

4. The cancer assay panel of any one of claims 1-3, wherein the abnormal methylation pattern has at least a threshold p-value rarity in the cancerous samples.

5. The cancer assay panel of any one of claims 1-4, wherein each of the probes is designed to have less than 20 off-target genomic regions, optionally wherein the less than 20 off-target genomic regions are identified using a k-mer seeding strategy, and optionally wherein the less than 20 off-target genomic regions are identified using k-mer seeding strategy combined to local alignment at seed locations.

6. The cancer assay panel of any of claims 1-5, comprising at least 10,000, 50,000, 100,000, 200,000, 300,000, 400,000, 500,000, 600,000, 700,000 or 800,000 probes.

7. The cancer assay panel of any of claims 1-6, wherein the at least 500 pairs of probes together comprise at least 2 million, 3 million, 4 million, 5 million, 6 million, 8 million, 10 million, 12 million, 14 million, or 15 million nucleotides.

8. The cancer assay panel of any of claims 1-7, wherein each of the probes comprises:

(i) at least 50, 75, 100, or 120 nucleotides; and/or
(ii) less than 300, 250, 200, or 150 nucleotides; and/or
(iii) 100-150 nucleotides; and/or
(iv) less than 20, 15, 10, 8, or 6 methylation sites; and/or
(v) multiple binding sites to the methylation sites of the converted cfDNA molecule, wherein at least 80, 85, 90, 92, 95, or 98% of the multiple binding sites comprise exclusively either CpG or CpA; and/or
(vi) less than 15, 10 or 8 off-target genomic regions.

9. The cancer assay panel of any of claims 1-8, wherein at least 80, 85, 90, 92, 95, or 98% of the at least five methylation sites are either methylated or unmethylated in the cancerous samples.

10. The cancer assay panel of any of claims 1-9, wherein at least 3%, 5%, 10%, 15%, or 20% of the probes comprise no G (Guanine).

11. The cancer assay panel of any of claims 1-10, wherein:

(i) at least 30% of the genomic regions are in exons or introns; and/or
(ii) at least 15% of the genomic regions are in exons; and/or
(iii) at least 20% of the genomic regions are in exons, and/or
(iv) less than 10% of the genomic regions are in intergenic regions.

12. The cancer assay panel of any of claims 1-11, wherein the genomic regions:

(i) are selected from any one of Lists 1-3 or Lists 4-16; and/or
(ii) comprise at least 20%, 30%, 40%, 50%, 60%, 70%, 80%, 90% or 95% of the genomic regions in any one of Lists 1-3 or Lists 4-16; and/or
(iii) comprise at least 500, 1,000, 5000, 10,000, or 15,000, 20,000, 30,000, 40,000, 50,000, 60,000, or 70,000 genomic regions in any one of Lists 1-3 or Lists 4-16.

FIG. 1A

FIG. 1B

Binary targets (pull down both types)

Methylated    Un-methylated

OR

semi-binary targets (pull down one type of fragment)

**FIG. 1C**

FIG. 2

Generate data structure for a control group
300

```
┌─────────────────────────────────────────────┐
│    Generate set of methylation state vectors │
│              for a control group             │
│                     100                      │
└─────────────────────────────────────────────┘
                      │
                      ▼
┌─────────────────────────────────────────────┐
│ For each methylation state vector, subdivide into │
│          strings of methylation sites        │
│                     310                      │
└─────────────────────────────────────────────┘
                      │
                      ▼
┌─────────────────────────────────────────────┐
│    Tally strings for each position and methylation │
│              state combination               │
│                     320                      │
└─────────────────────────────────────────────┘
                      │
                      ▼
┌─────────────────────────────────────────────┐
│    Create data structure storing counts of all │
│      possible strings from the control group │
│                     330                      │
└─────────────────────────────────────────────┘
                      ┊
                      ▼
┌ ─ ─ ─ ─ ─ ─ ─ ─ ─ ─ ─ ─ ─ ─ ─ ─ ─ ─ ─ ─ ─ ─ ┐
      Validate data structure consistency
│                    340                       │
└ ─ ─ ─ ─ ─ ─ ─ ─ ─ ─ ─ ─ ─ ─ ─ ─ ─ ─ ─ ─ ─ ─ ┘
```

# FIG. 3A

Validate data structure consistency
340

Generate set of methylation state vectors
for a validation group
100

Calculate a p-value score for each methylation
state vector with control group data structure
350

Build cumulative density function (CDF) of all p-
value's from the validation group
360

Validate consistency of CDF
370

## FIG. 3B

Identifying target genomic regions
400

Generate set of methylation state vectors from a sample
100

For each methylation state vector, enumerate all possible methylation state vectors at that position
410

Calculate probabilities for all possible methylation state vectors from control group data structure
420

Use sliding window for methylation state vectors with greater than a threshold number of methylation sites
435

Calculate a p-value score for each methylation state vector based on calculated probabilities
430

Filter set resulting in a subset of anomalously methylated vectors based on p-value scores being below a threshold value
440

Identify fragments indicative of cancer from filtered set
450

Identify genomic regions indicative of cancer from the fragments
460

Identify genomic regions indicative of smoking
465

Remove probes having off-target regions more than a threshold number
475

Design probes targeting the genomic regions
470

**FIG. 4**

Calculate P-Value with
Markov Chain Model
500

Test Methylation State Vector
505

< M23, M24, M25, U26 >

410
420

| P | < M23, M24, M25, M26 > |
|---|---|
| P | < M23, M24, M25, U26 > |

$= P(M_{26} \mid M_{23}, M_{24}, M_{25}) * P(M_{25} \mid M_{23}, M_{24}) * P(M_{24} \mid M_{23}) * P(M_{23})$

$\approx P(M_{26} \mid M_{24}, M_{25}) * P(M_{25} \mid M_{23}, M_{24}) * P(M_{24} \mid M_{23}) * P(M_{23})$

| P | < U23, U24, U25, U26 > |
|---|---|

$= P(U_{26} \mid U_{23}, U_{24}, U_{25}) * P(U_{25} \mid U_{23}, U_{24}) * P(U_{24} \mid U_{23}) * P(U_{23})$

$\approx P(U_{26} \mid U_{24}, U_{25}) * P(U_{25} \mid U_{23}, U_{24}) * P(U_{24} \mid U_{23}) * P(U_{23})$

Probabilities of Possible
Methylation State Vectors
515

430

| p-value | < M23, M24, M25, U26 > |
|---|---|

$= \sum [\, All\ probabilities \leq P(\ <M23, M24, M25, U26>\ )\,]$

P-Value of Test Methylation
State Vector
525

# FIG. 5

Training a classifier
600

Obtain anomalous fragments from a non-cancer training group
and a cancer training group
605

For each methylation state vector from the two training groups,
determine if hypomethylated or hypermethylated
610

For each methylation site in the genome, generate a
hypomethylation score and a hypermethylation score
620

For each methylation state vector, generate an aggregate
hypomethylation score and an aggregate hypermethylation score
630

For each subject, rank methylation state vectors by aggregate
hypomethylation score and by aggregate hypermethylation score
640

For each subject, generate a feature vector based on rankings
650

Train to distinguish feature vectors from non-cancer training
group and from cancer training group
660

Compute information gain of each CpG site
670

# FIG. 6A

Computing pairwise information gain
680

For each sample, define a feature vector for each
cancer type at each region based on count of
fragments above a log-likelihood ratio above
various thresholds
690

Calculate an informative score for each CpG site
describing ability to distinguish between pairs of
cancer type
695

# FIG. 6B

Start

105 — Extract nucleic acids from a test sample

110 — Treat extracted nucleic acid to convert unmethylated cytosine to uracil

115 — Prepare library using adapter ligation

120 — Hybridize nucleic acids using hybridization probes

125 — Perform enrichment on nucleic acid fragments

130 — Generate sequence reads of the nucleic acid

140 — Generate methylation state vector for nucleic acids

End

## FIG. 7A

Generate a methylation state vector from a
cell-free (cf) DNA fragment in sample
100

Methyl Groups
114

Fragment cfDNA
112

-C-G-        -C-G-        -C-G-

120

Converted cfDNA
122

-C-G-        -U-G-        -C-G-

130
140

Sequence Read
142

Methylated        Unmethylated        Methylated

-C-G-        -T-G-        -C-G-

Reference Genome
144

-C-G-        -C-G-        -C-G-

23            24            25

160

Methylation State
Vector
152

< M23, U24, M25 >

**FIG. 7B**

**FIG. 8**

cfDNA Concentration Per Sample
190

FIG. 9

**FIG. 10**

**FIG. 11A**

**FIG. 11B**

FIG. 11C

**FIG. 12A**

**FIG. 12B**

FIG. 13

**FIG. 14**

**List 4**

**FIG. 15B**

**FIG. 15A**

**List 5**

FIG. 16B

FIG. 16A

**List 6**

FIG. 17A

FIG. 17B

**List 7**

**FIG. 18B**

**FIG. 18A**

List 8

FIG. 19A

FIG. 19B

# List 9

**FIG. 20B**

**FIG. 20A**

# List 10

FIG. 21A

FIG. 21B

# List 11

**FIG. 22A**

**FIG. 22B**

**List 12**

**FIG. 23B**

**FIG. 23A**

# List 13

FIG. 24A

FIG. 24B

**List 14**

FIG. 25B

FIG. 25A

# List 15

**FIG. 26A**

**FIG. 26B**

List 16

FIG. 27A

FIG. 27B

**Random 10% of List 12**

FIG. 28B

FIG. 28A

**Random 25% of List 12**

**FIG. 29A**

**FIG. 29B**

# Random 50% of List 4

FIG. 30A

FIG. 30B

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

**Patent documents cited in the description**

- US 62797176 A
- US 62797174 A
- US 62797170 A
- WO 2010037001 A
- WO 2011127136 A

- US 20160340740 A, Zhang
- US 352602 A
- US 20190287652 A
- US 35260219 A
- US 2019022122 W

**Non-patent literature cited in the description**

- AJCC Cancer Staging Manual. Springer, 2017

- **RIEDMILLER M** ; **BRAUN H.** RPROP - A Fast Adaptive Learning Algorithm. *Proceedings of the International Symposium on Computer and Information Science VII*, 1992